# EUROPEAN PATENT APPLICATION

(11) **EP 4 759 330 A1**
(43) Date of publication of application: **17.06.2026**
(21) Application number: 24853716.9
(22) Date of filing: 09.08.2024
(51) Int. Cl.: A61K 47/68, C07K 16/28, C12N 15/13, C12N 15/85, C12N 5/10, A61K 31/4745, A61P 35/00

(54) **APPLICATION OF ANTI-FR-ALPHA ANTIBODY-DRUG CONJUGATE IN TREATMENT OF DISEASES**

(30) Priority: 11.08.2023 CN 202311018230; 25.03.2024 CN 202410347310; 29.05.2024 CN 202410686915
(71) Applicant: Bio-Thera Solutions, Ltd., Guangzhou, Guangdong 510005 (CN)
(72) Inventor: FU, Ziyi, Guangzhou, Guangdong 510005 (CN); SONG, Shuqiang, Guangzhou, Guangdong 510005 (CN); TANG, Weijia, Guangzhou, Guangdong 510005 (CN); LI, Shengfeng, Guangzhou, Guangdong 510005 (CN); YU, Jin-Chen, Guangzhou, Guangdong 510005 (CN)
(74) Representative: Engelhard, Markus
(86) International application number: PCT/CN2024/111203
(87) International publication number: WO 2025/036307

(57) **Abstract**

The present invention discloses an application of an anti-FRα antibody-drug conjugate in the treatment of diseases. A treatment method comprises administering an effective amount of the anti-FRα antibody-drug conjugate to a patient in need thereof.

## Description

### TECHNICAL FIELD

The present invention relates to the field of pharmaceutical therapy, specifically to the application of anti-FRα antibody-drug conjugate in treatment of diseases.

### BACKGROUND

An antibody-drug conjugate (ADC) is formed by linking a monoclonal antibody targeting a specific antigen to a small-molecule cytotoxic drug via a linker. It has both the powerful killing effect of conventional small-molecule chemotherapy and the tumor-targeting property of antibody drugs, and can efficiently deliver small-molecule cytotoxic drugs to target tumor cells in a targeted manner, thereby overcoming the killing effect of cytotoxicity on normal cells. At present, ADCs have become one of the hot research directions in the field of precise tumor treatment.

Folate, as an essential vitamin required for DNA synthesis and repair as well as the process of cell division, is transported through endocytosis by binding to folate receptors on the cell surface, which are internalized and then recycled back to the cell membrane. The folate receptor is a transmembrane single-chain glycoprotein linked to glycosylated phosphatidylinositol and has a high affinity for folate. The folate receptor includes three subtypes, i.e., folate receptor α (FOLR1, FRα), folate receptor β (FOLR2) and folate receptor γ (FOLR3). The expression of the folate receptor is highly restricted in normal cells and is significantly highly expressed on tumor cells, wherein the folate receptor α is found to be overexpressed in various malignant tumors, and thus becomes one of hot targets of anti-cancer drugs.

### SUMMARY

The present invention provides use of an anti-folate receptor α (FRα) antibody-drug conjugate in the treatment of diseases (e.g., cancer).

In one or more embodiments, the present invention provides a method for treating and/or preventing diseases (e.g., cancer), which comprises administering to a patient in need (e.g., a human patient) an effective amount of an antibody-drug conjugate. In one or more embodiments, the administration is by injection.

In one or more embodiments, the present invention provides a method for treating and/or preventing cancer, which comprises administering to a human patient in need an effective amount of an antibody-drug conjugate by injection.

In one or more embodiments, the present invention provides use of an effective amount of the antibody-drug conjugate or the pharmaceutical composition thereof in the manufacture of a drug for treating and/or preventing diseases (e.g., cancer).

In one or more embodiments, the present invention provides use of an effective amount of the antibody-drug conjugate or the pharmaceutical composition thereof in the treatment and/or prevention of diseases (e.g., cancer).

In one or more embodiments, the antibody-drug conjugate is administered at a dose of 0.5 mg/kg to 5 mg/kg at a frequency of once per day to once every 7 weeks. In one or more embodiments, the antibody-drug conjugate is administered at a dose of 1.7 mg/kg to 2.5 mg/kg once every 2-4 weeks.

In one or more embodiments, the antibody-drug conjugate is administered at a dose of 10 mg to 300 mg at a frequency of once per day to once every 7 weeks. In one or more embodiments, the antibody-drug conjugate is administered at a dose of 100 mg to 250 mg once every 2-4 weeks.

In one or more embodiments, the antibody-drug conjugate is administered at a dose of 5 mg/m² to 150 mg/m² at a frequency of once per day to once every 7 weeks. In one or more embodiments, the antibody-drug conjugate is administered at a dose of 70 mg/m² to 100 mg/m² once every 2-4 weeks. In one or more embodiments, the antibody-drug conjugate is administered at a dose of 75 mg/m² to 100 mg/m² once every 2-4 weeks.

In one or more embodiments, the antibody-drug conjugate comprises an anti-FRα antibody or an antigen-binding unit thereof conjugated to a drug via a linker.

In one or more embodiments, the linker is a cleavable linker.

In one or more embodiments, the antibody-drug conjugate has a structure of Formula I, or a stereoisomer thereof, or a pharmaceutically acceptable salt or solvate thereof: 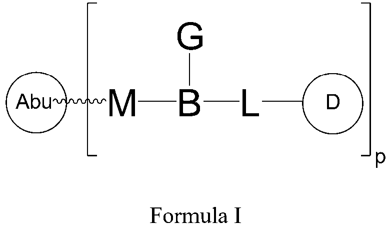 wherein,
Abu is an anti-FRα antibody or an antigen-binding unit thereof;
D is a drug;
M is 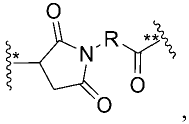 wherein * links to Abu, ** links to B, and R is selected from: -(CH₂)ᵣ-, - (CHR^{m})ᵣ, C3-C8 carbocyclyl, -O-(CH₂)ᵣ-, arylene, -(CH₂)ᵣ-arylene-, -arylene-(CH₂)r-, -(CH₂)ᵣ-(C3-C8 carbocyclyl)-, -(C3-C8 carbocyclyl)-(CH₂)ᵣ-, C3-C8 heterocyclyl, -(CH₂)ᵣ-(C3-C8 heterocyclyl)-, -(C3-C8 heterocyclyl)-(CH₂)ᵣ-, -(CH₂)ᵣC(O)NR^{m}(CH₂)ᵣ-, -(CH₂CH₂O)ᵣ-, -(CH₂CH₂O)ᵣ-CH₂-, - (CH₂)ᵣC(O)NR^{m}(CH₂CH₂O)ᵣ-, -(CH₂)ᵣC(O)NR^{m}(CH₂CH₂O)ᵣ-CH₂-, - (CH₂CH₂O)ᵣC(O)NR^{m}(CH₂CH₂O)ᵣ-, -(CH₂CH₂O)ᵣC(O)NR^{m}(CH₂CH₂O)ᵣ-CH₂- and - (CH₂CH₂O)ᵣC(O)NR^{m}(CH₂)ᵣ; wherein each R^{m} is independently H, C1-C6 alkyl, C3-C8 carbocyclyl, phenyl or benzyl, and each r is independently 1, 2, 3, 4, 5, 6, 7, 8, 9 or 10;
B is 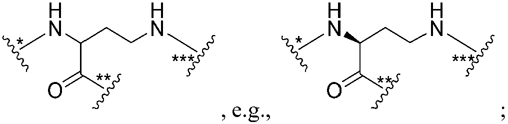 wherein * links to M, ** links to L, and *** links to G;
L is-(AA)ᵢ-(FF)_{f}-, wherein AA is an amino acid or polypeptide, and i is 1, 2, 3, 4, 5, 6, 7, 8, 9, 10, 11, 12, 13, 14, 15, 16, 17, 18, 19 or 20;
each FF is independently 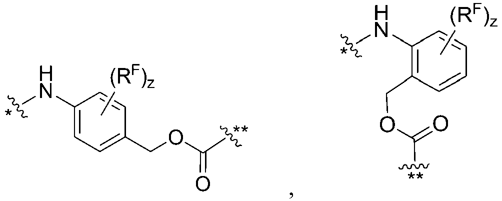 or 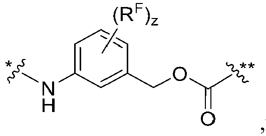 wherein each R^{F} is independently C1-C6 alkyl, C1-C6 alkoxy, -NO₂ or halogen; z is 0, 1, 2, 3 or 4; wherein * links to AA, and ** links to D; f is 1, 2, 3, 4, 5, 6, 7, 8, 9 or 10;
G is 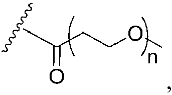 wherein n is an integer from 1-24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 8, 9 or 10.

In one or more embodiments, R is -(CH₂)ᵣ-.

In one or more embodiments, R is -(CH₂)ᵣ-, wherein r is 1 or 5.

In one or more embodiments, each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu, and Gly-Phe-Leu-Gly.

In one or more embodiments, AA is Val-Cit.

In one or more embodiments, i is 1.

In one or more embodiments, AA is Val-Cit, i is 1.

In one or more embodiments, the halogen is F.

In one or more embodiments, R^{F} is -CH₃, F, -NO₂ or -OCH₃.

In one or more embodiments, z is 0.

In one or more embodiments, z is 1 or 2.

In one or more embodiments, each FF is independently 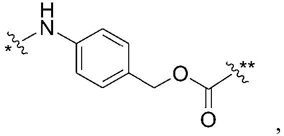 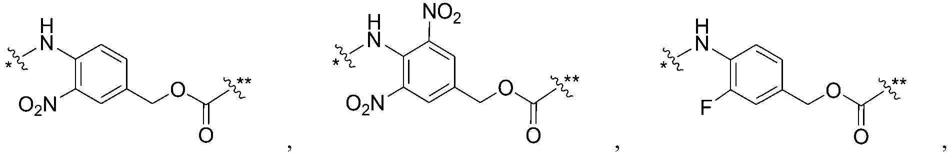 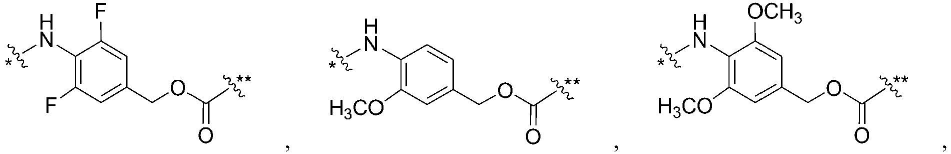 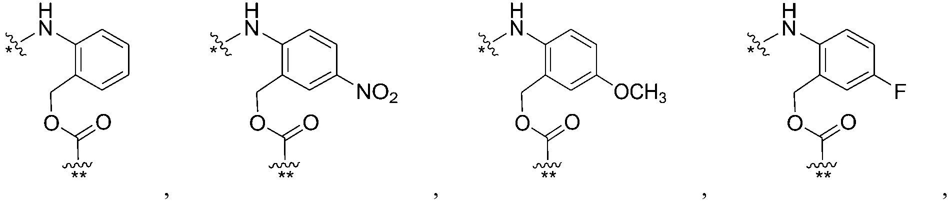 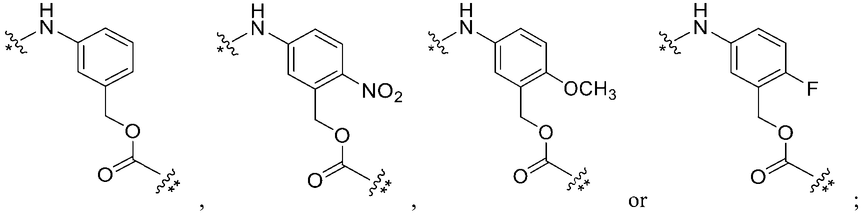 wherein * links to AA, and ** links to D.

In one or more embodiments, f is 1.

In one or more embodiments, FF is 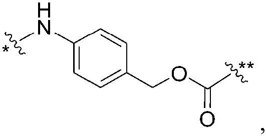 wherein * links to AA, and ** links to D.

In one or more embodiments, FF is 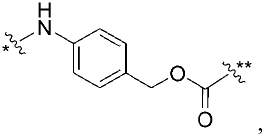 and f is 1; wherein * links to AA, and ** links to D.

In one or more embodiments, L is 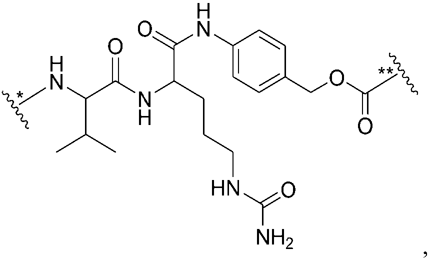 wherein * links to B, and ** links to D.

In one or more embodiments, L is 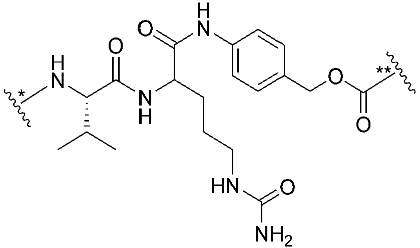 or 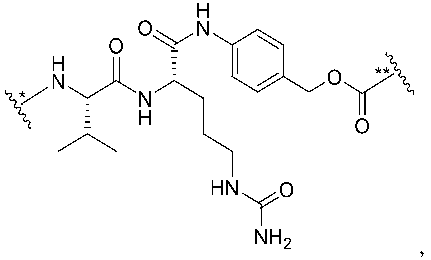 wherein * links to B, and ** links to D.

In one or more embodiments, n is an integer from 4-12.

In one or more embodiments, n is an integer from 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the antibody-drug conjugate has a structure of Formula I-1, or a stereoisomer thereof, or a pharmaceutically acceptable salt or solvate thereof: 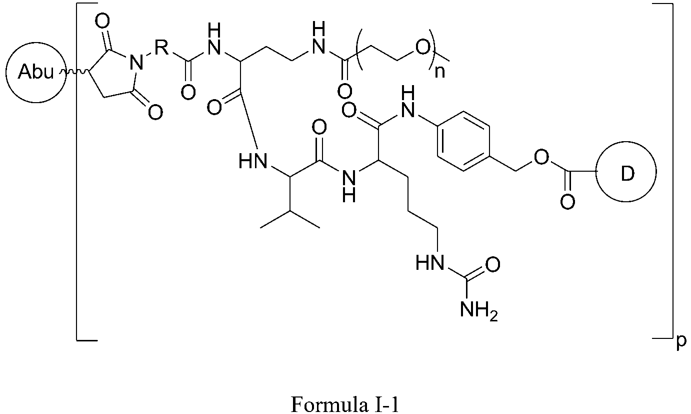 wherein,
Abu is an anti-FRα antibody or an antigen-binding unit thereof;
R is selected from: -(CH₂)ᵣ-, -(CHR^{m})ᵣ-, C3-C8 carbocyclyl, -O-(CH₂)ᵣ-, arylene, -(CH₂)ᵣ-arylene-, - arylene-(CH₂)r-, -(CH₂)ᵣ-(C3-C8 carbocyclyl)-, -(C3-C8 carbocyclyl)-(CH₂)ᵣ-, C3-C8 heterocyclyl, - (CH₂)ᵣ-(C3-C8 heterocyclyl)-, -(C3-C8 heterocyclyl)-(CH₂)ᵣ, -(CH₂)ᵣC(O)NR^{m}(CH₂)ᵣ-, -(CH₂CH₂O)ᵣ-, -(CH₂CH₂O)ᵣ-CH₂-, -(CH₂)ᵣC(O)NR^{m}(CH₂CH₂O)ᵣ-, -(CH₂)ᵣC(O)NR^{m}(CH₂CH₂O)ᵣ-CH₂-, - (CH₂CH₂O)ᵣC(O)NR^{m}(CH₂CH₂O)ᵣ, -(CH₂CH₂O)ᵣC(O)NR^{m}(CH₂CH₂O)ᵣ-CH₂- and - (CH₂CH₂O)ᵣC(O)NR^{m}(CH₂)ᵣ-; wherein each R^{m} is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, R is -(CH₂)ᵣ-.

In one or more embodiments, R is -(CH₂)ᵣ-, wherein r is 1 or 5.

In one or more embodiments, n is an integer from 4-12.

In one or more embodiments, n is an integer from 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the antibody-drug conjugate has a structure of Formula I-2 or I-2-1, or a stereoisomer thereof, or a pharmaceutically acceptable salt or solvate thereof: 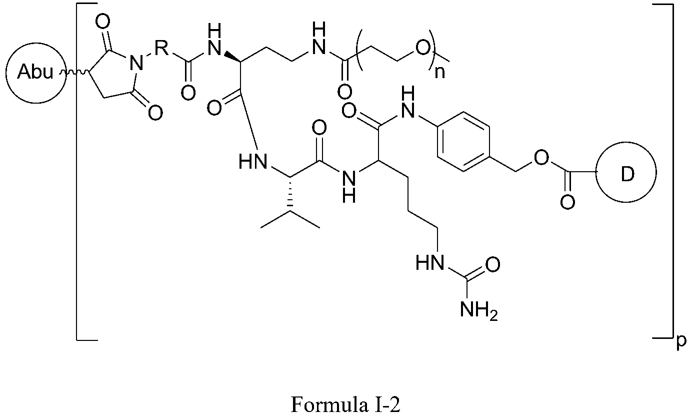 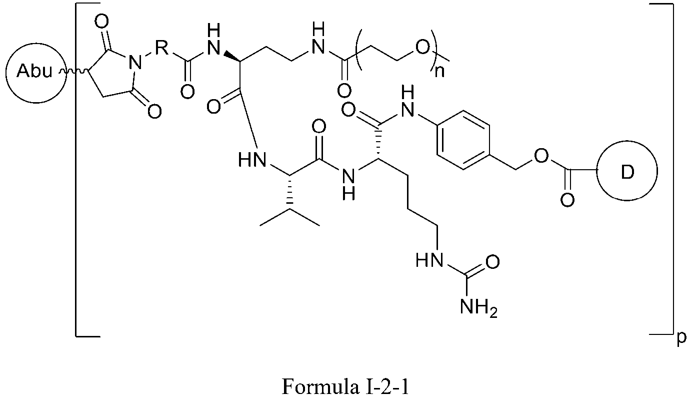 wherein
Abu is an anti-FRα antibody or an antigen-binding unit thereof;
R is selected from: -(CH₂)ᵣ-, -(CHR^{m})ᵣ-, C3-C8 carbocyclyl, -O-(CH₂)ᵣ-, arylene, -(CH₂)ᵣ-arylene-, - arylene-(CH₂)r-, -(CH₂)ᵣ-(C3-C8 carbocyclyl)-, -(C3-C8 carbocyclyl)-(CH₂)ᵣ-, C3-C8 heterocyclyl, - (CH₂)ᵣ-(C3-C8 heterocyclyl)-, -(C3-C8 heterocyclyl)-(CH₂)ᵣ, -(CH₂)ᵣC(O)NR^{m}(CH₂)ᵣ-, -(CH₂CH₂O)ᵣ-, -(CH₂CH₂O)ᵣ-CH₂-, -(CH₂)ᵣC(O)NR^{m}(CH₂CH₂O)ᵣ-, -(CH₂)ᵣC(O)NR^{m}(CH₂CH₂O)ᵣ-CH₂-, - (CH₂CH₂O)ᵣC(O)NR^{m}(CH₂CH₂O)ᵣ, -(CH₂CH₂O)ᵣC(O)NR^{-m}(CH₂CH₂O)ᵣ-CH₂- and - (CH₂CH₂O)ᵣC(O)NR^{m}(CH₂)ᵣ-; wherein each R^{m} is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, R is -(CH₂)ᵣ-.

In one or more embodiments, R is -(CH₂)ᵣ-, wherein r is 1 or 5.

In one or more embodiments, n is an integer from 4-12.

In one or more embodiments, n is an integer from 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the antibody-drug conjugate has a structure of Formula I-3, or a stereoisomer thereof, or a pharmaceutically acceptable salt or solvate thereof: 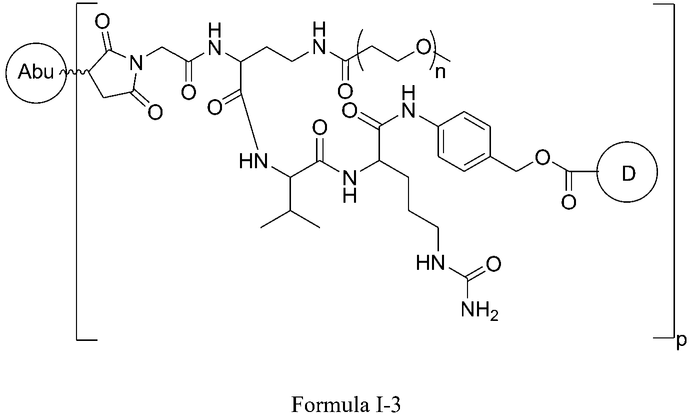 wherein
Abu is an anti-FRα antibody or an antigen-binding unit thereof;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, n is an integer from 4-12.

In one or more embodiments, n is an integer from 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the antibody-drug conjugate has a structure of Formula I-4 or I-4-1, or a stereoisomer thereof, or a pharmaceutically acceptable salt or solvate thereof: 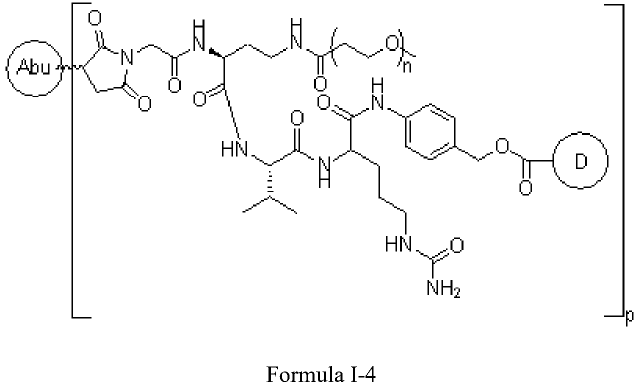 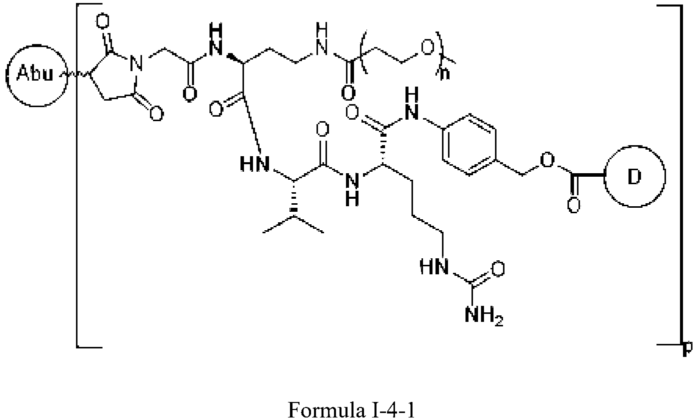 wherein
Abu is an anti-FRα antibody or an antigen-binding unit thereof;
D is a drug;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, n is an integer from 4-12.

In one or more embodiments, n is an integer from 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the antibody-drug conjugate has a structure of Formula I-5, I-5-1, I-6, I-6-1, I-7, I-7-1, I-8, I-8-1, I-9, I-9-1, I-10, I-10-1, I-11 or I-11-1, or a stereoisomer thereof, or a pharmaceutically acceptable salt or solvate thereof: 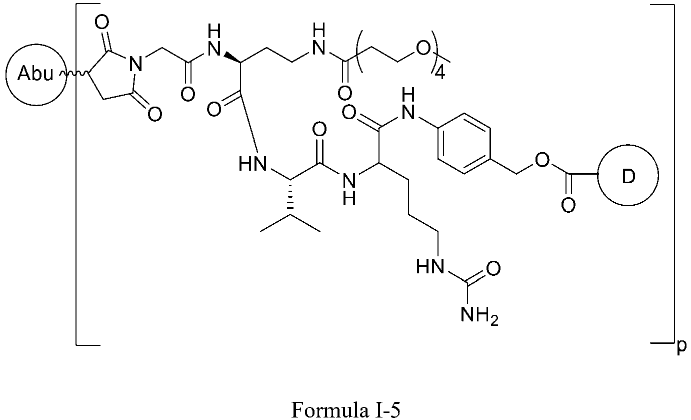 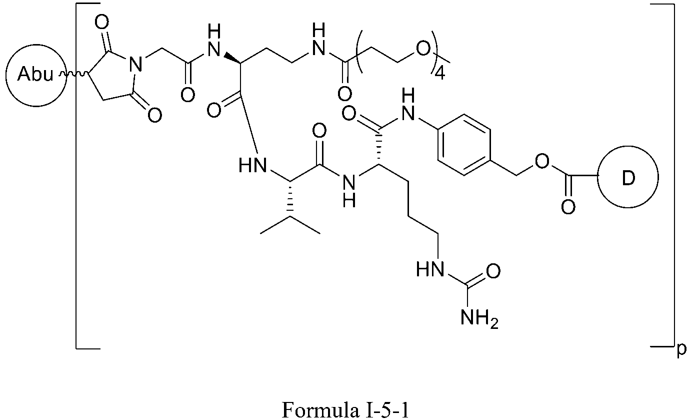 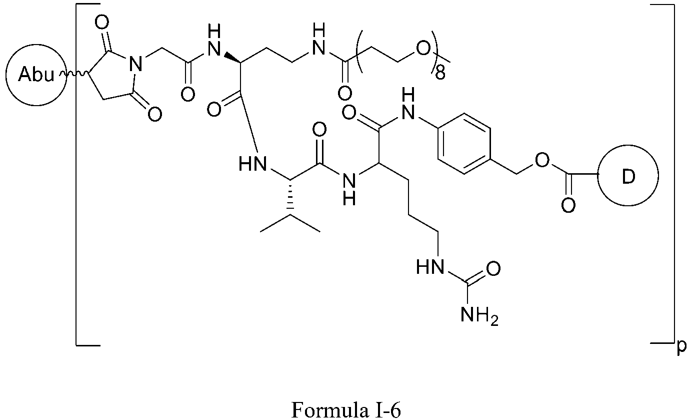 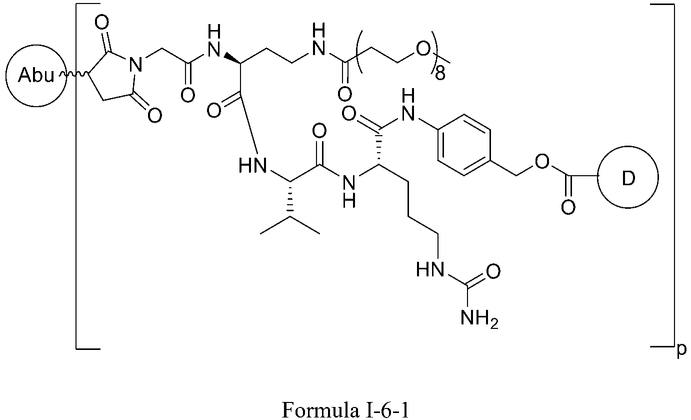 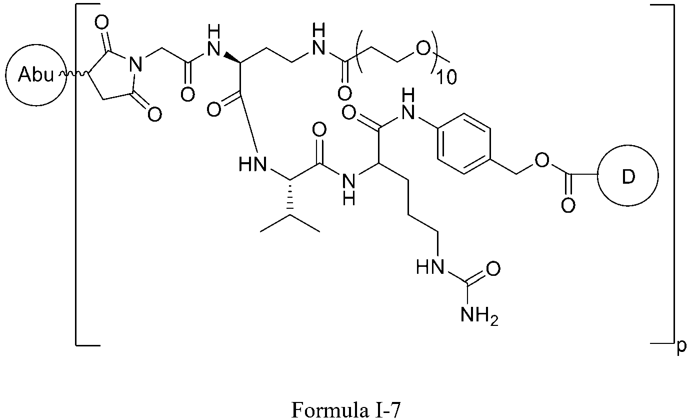 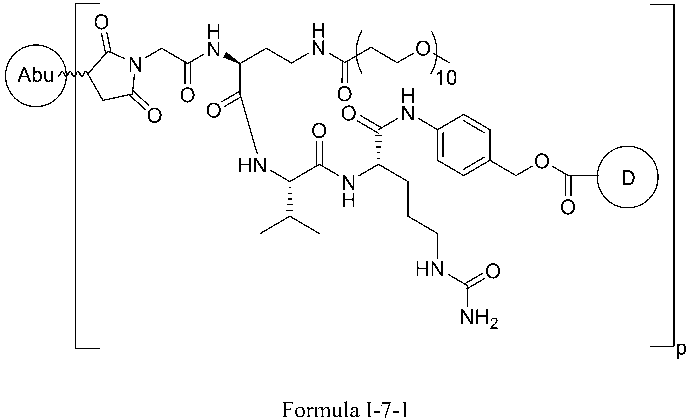 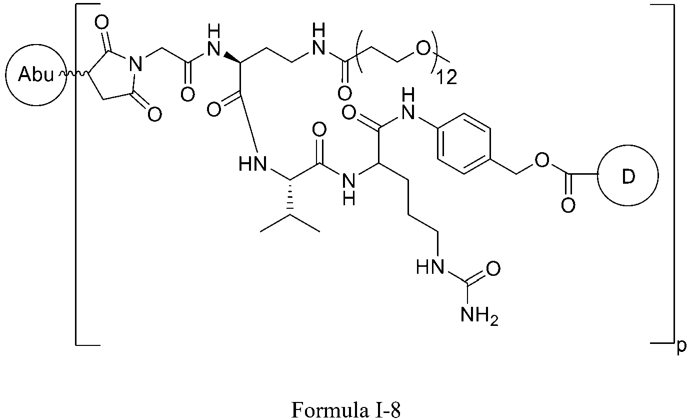 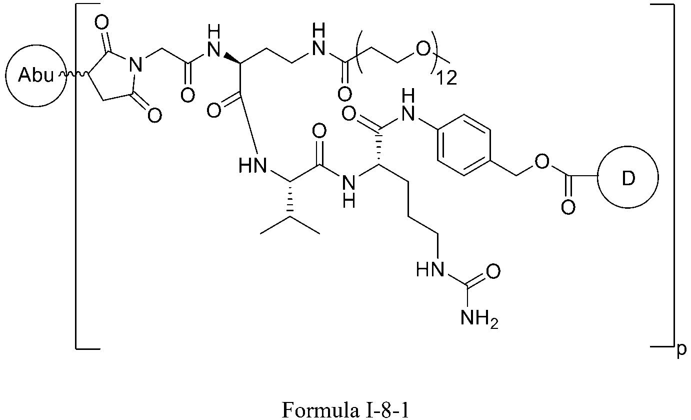 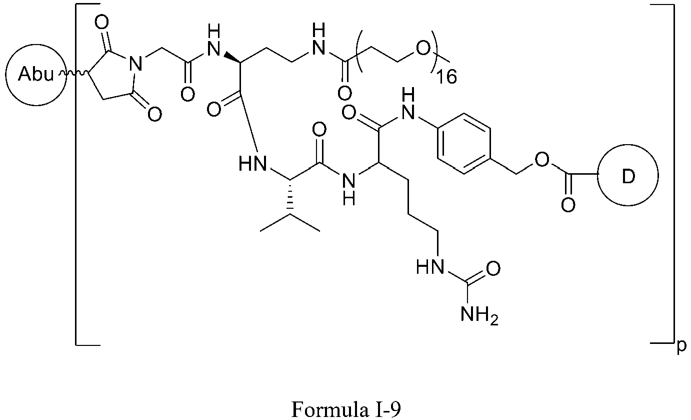 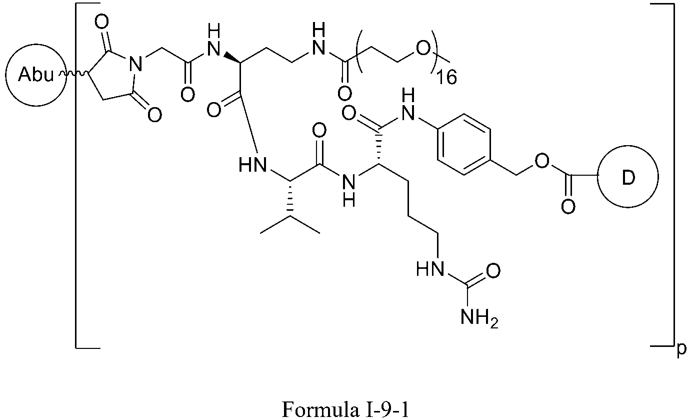 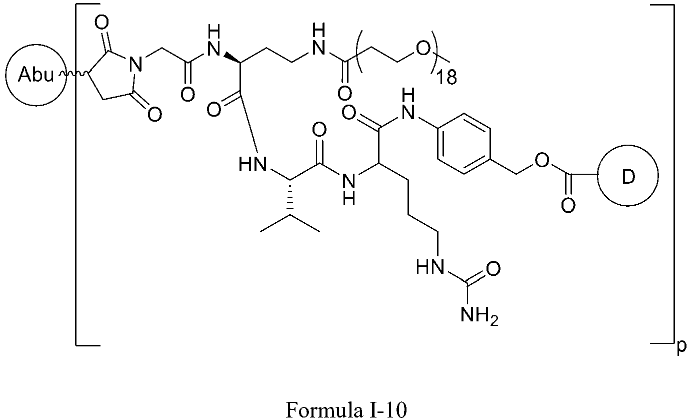 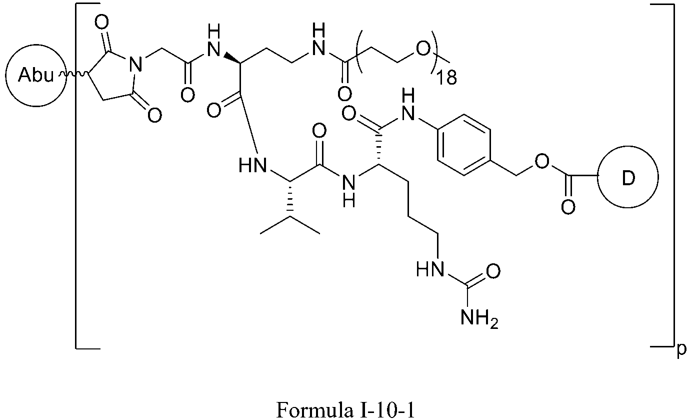 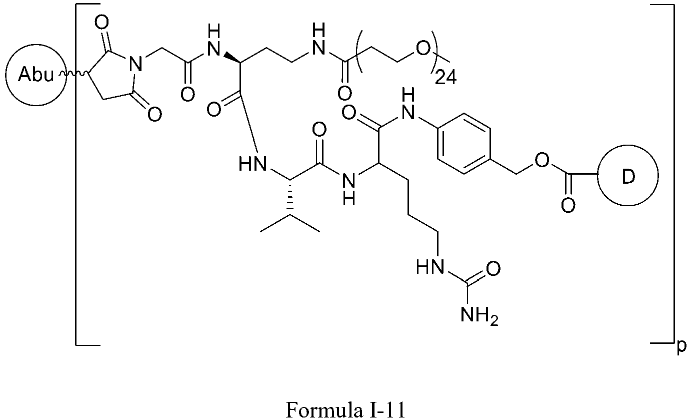 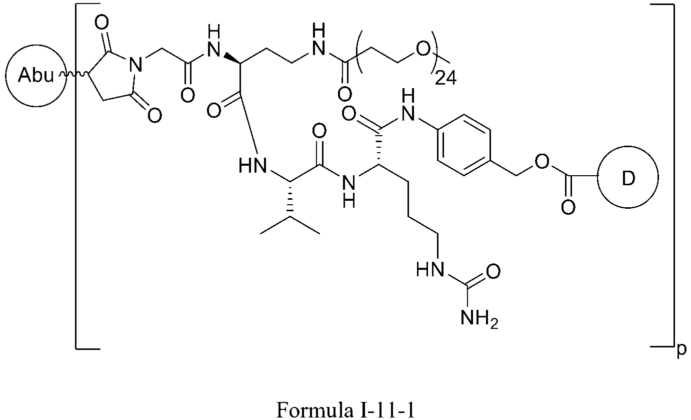 wherein
Abu is an anti-FRα antibody or an antigen-binding unit thereof;
D is a drug;
p is 1-10, e.g., 1, 2, 3, 4, 5, 6, 7, 7.4, 8, 9 or 10.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the drug is an anti-cancer drug, a cytotoxic drug, a cell differentiation factor, a stem cell trophic factor, a steroid drug, a drug for treating autoimmune diseases, an antiinflammatory drug or a drug for treating infectious diseases.

In one or more embodiments, the drug is a tubulin inhibitor, a DNA damaging agent, or a DNA topoisomerase inhibitor.

In one or more embodiments, the tubulin inhibitor is selected from dolastatin, auristatins and maytansinoids (maytansine).

In one or more embodiments, the drug is an auristatin, e.g., monomethyl auristatin E (MMAE), monomethyl auristatin F (MMAF), or auristatin F (AF).

In one or more embodiments, the drug is a DNA damaging agent, e.g., a calicheamicin, a duocarmycin, or an anthramycin derivative PBD (pyrrolobenzodiazepine).

In one or more embodiments, the drug is a DNA topoisomerase inhibitor or a salt thereof, e.g., irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-*N*-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, or *N*-[2-(dimethylamino)ethyl]-4-acridinecarboxamide.

In one or more embodiments, the DNA topoisomerase inhibitor is camptothecin, 10-hydroxycamptothecin, topotecan, belotecan, irinotecan, 22-hydroxyacuminatine, or exatecan, or a salt thereof.

In one or more embodiments, the drug is a tubulysin, a taxane drug derivative, a leptomycine derivative, CC-1065 or an analog thereof, an amatoxin, a spliceosome inhibitor, a benzodiazepine (PBD) dimer, adriamycin, methotrexate, vincristine, vinblastine, daunorubicin, mitomycin C, melphalan or a chlorambucil derivative.

In one or more embodiments, the drug is 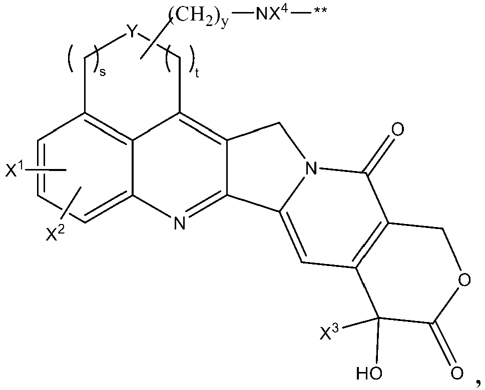 wherein
X¹ and X² are each independently:
H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,
amino-substituted heterocyclyl, which is optionally substituted at a nitrogen atom of the heterocyclyl moiety or at the amino moiety with a protecting group or one or more C1-C6 alkyl groups,
heterocyclylamino, which is optionally substituted at a nitrogen atom of the heterocyclic moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
X³ is C1-C6 alkyl;
X⁴ is H, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-aryle ne-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, -(CH₂)_{q} C(O)NRⁿ(CH₂)_{q}-CH₃, -(CH₂CH₂O)_{q}-CH₃, -(CH₂CH₂O)_{q}-CH₂-CH₃, -(CH₂)_{q}C(O)NRⁿ(CH₂CH₂O)_{q}-CH₃, -(CH₂)_{q}C(O)NRⁿ(CH₂CH₂O)_{q}-CH₂-CH₃, -(CH₂CH₂O)_{q}C(O)NRⁿ(CH₂CH₂O)_{q}-CH₃, -(CH₂CH₂O)_{q}C(O) NRⁿ(CH₂CH₂O)_{q}-CH₂-CH₃ or -(CH₂CH₂O)_{q}C(O)NRⁿ(CH₂)_{q}-CH₃; wherein each Rⁿ is independently H, C1-C6 alkyl, C3-C8 carbocyclyl, phenyl or benzyl, and each q is independently 1, 2, 3, 4, 5, 6, 7, 8, 9 or 10;
** is an attachment point;
y is 0, 1 or 2;
Y is O, S or CR¹R², wherein R¹ and R² are each independently H or C1-C6 alkyl;
s and t are each independently 0, 1 or 2, but not both 0.

In one or more embodiments, X⁴ is H or C1-C6 alkyl.

In one or more embodiments, the heterocyclyl is azetidine, niverazine, morpholine, pyrrolidine, piperidine, imidazole, thiazole, oxazole or pyridine.

In one or more embodiments, the amino-protecting group is formyl, acetyl, trityl, *t*-butoxycarbonyl, benzyl, or*p*-methoxybenzyloxycarbonyl.

In one or more embodiments, the drug is 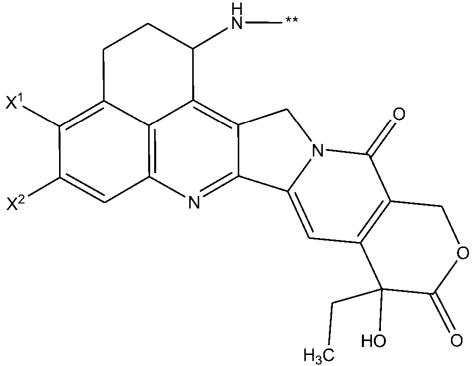 or 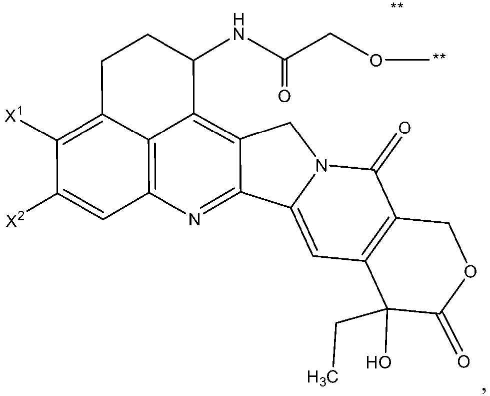 wherein X¹ and X² are each independently C1-C6 alkyl, halogen, or -OH; ** is an attachment point.

In one or more embodiments, the drug is 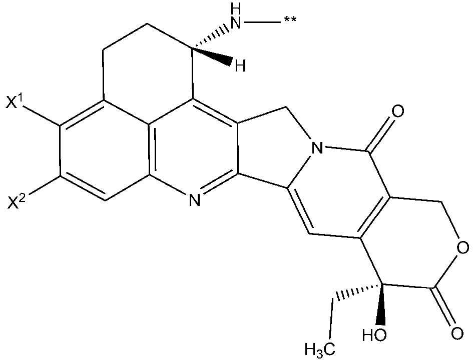 or 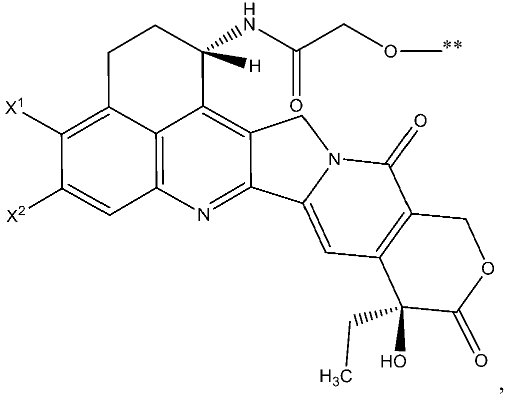 wherein X¹ and X² are each independently C1-C6 alkyl, halogen, or -OH; ** is an attachment point.

In one or more embodiments, the C1-C6 alkyl is -CH₃.

In one or more embodiments, the halogen is F.

In one or more embodiments, X¹ and X² are each independently -CH₃, F, or -OH.

In one or more embodiments, X¹ and X² are each -CH₃.

In one or more embodiments, X¹ and X² are each independently F, Cl, Br or I.

In one or more embodiments, X¹ and X² are each F.

In one or more embodiments, X¹ and X² are each independently F or -CH₃.

In one or more embodiments, X¹ is -CH₃, and X² is F.

In one or more embodiments, the antibody-drug conjugate has a structure of Formula I-26, I-27, I-12, I-12-1, I-13, I-13-1, I-14, I-14-1, I-15, I-15-1, I-16, I-16-1, I-17, I-17-1, I-18, I-18-1, I-19, I-19-1, I-20, I-20-1, I-21, I-21-1, I-22, 1-22-1, I-23, I-23-1, I-24, I-24-1, I-25 or I-25-1, or a stereoisomer thereof, or a pharmaceutically acceptable salt or solvate thereof: 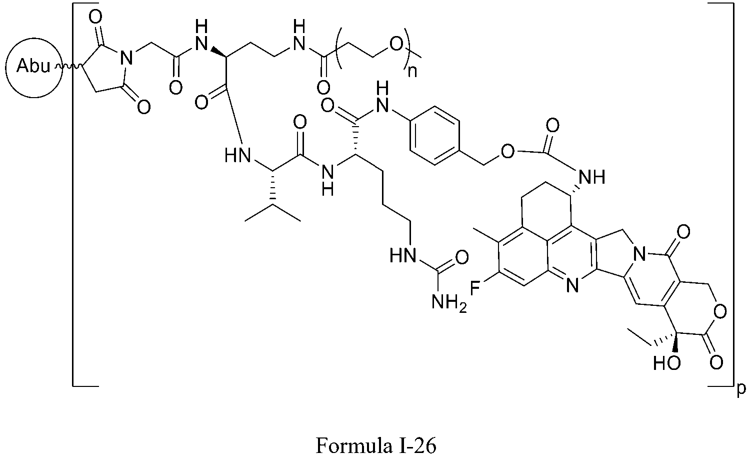 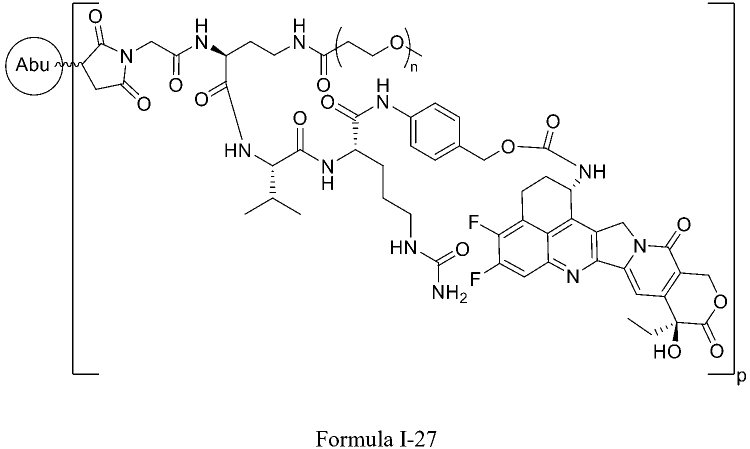 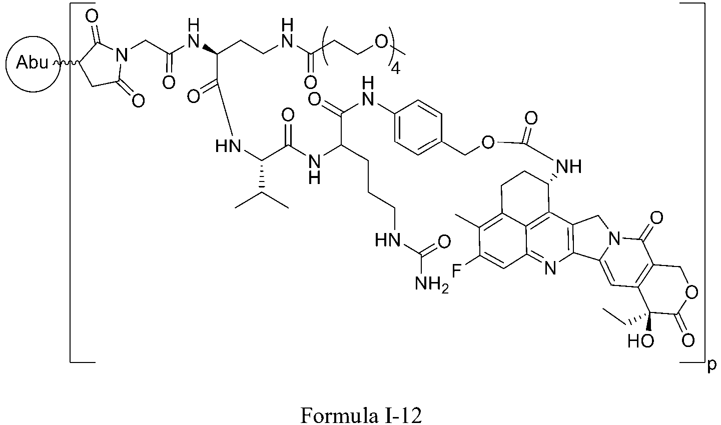 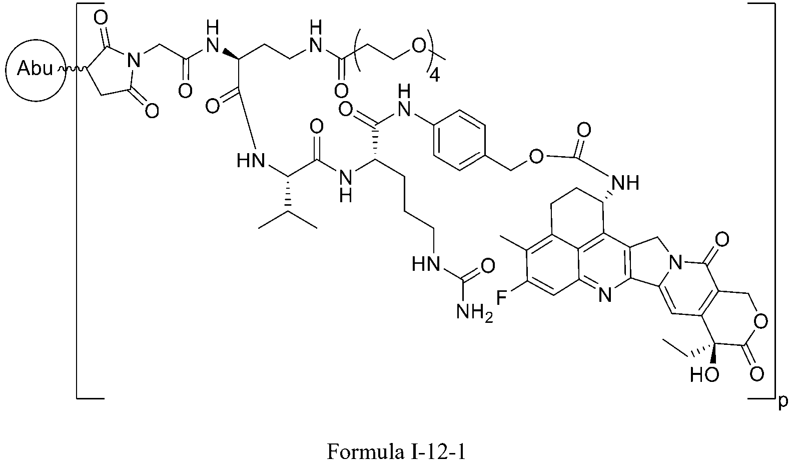 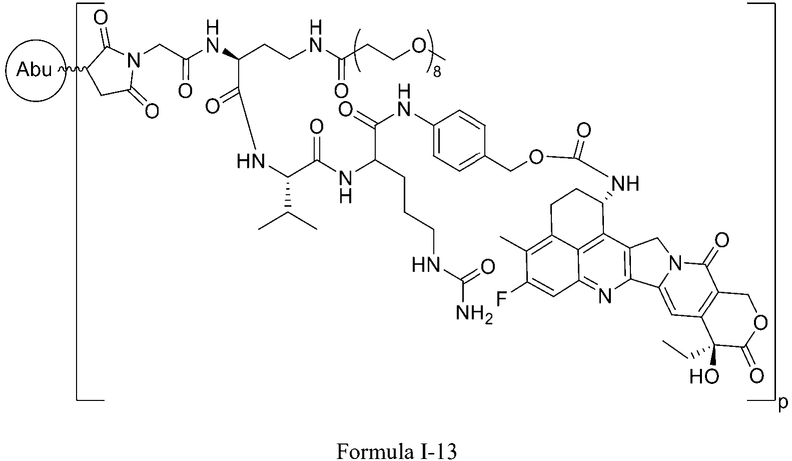 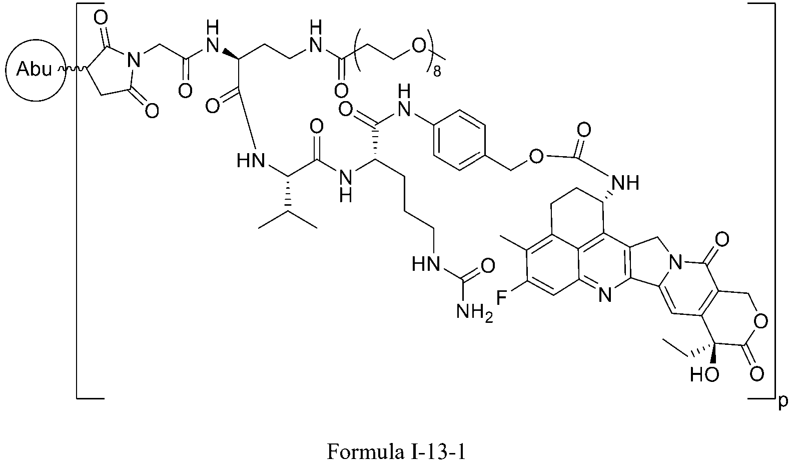 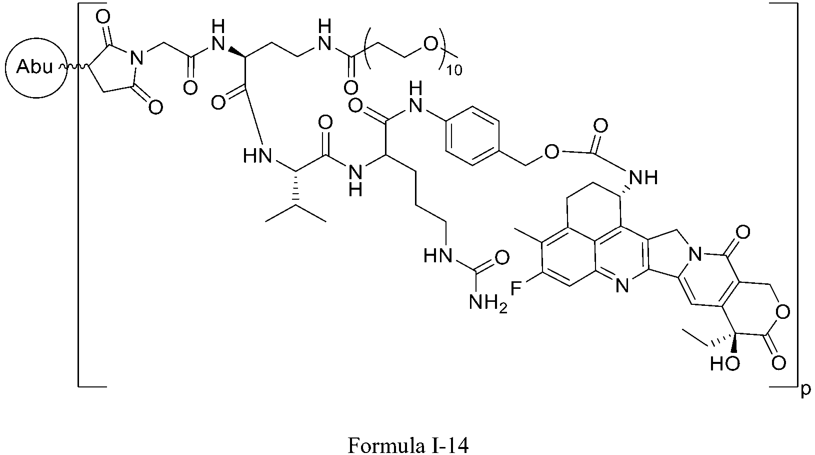 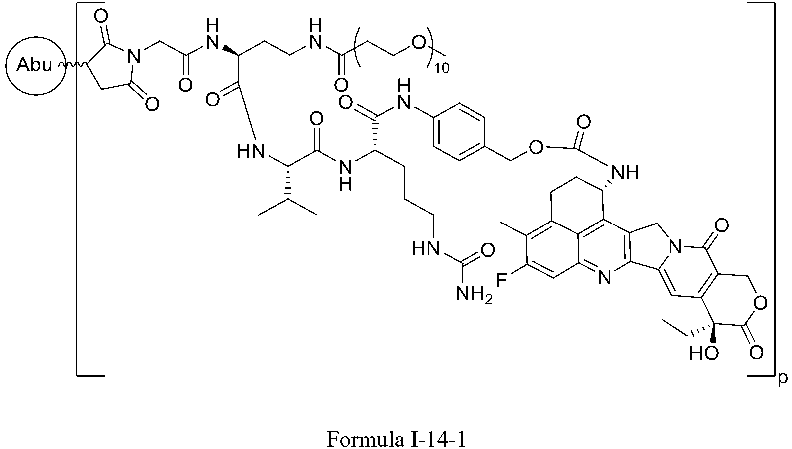 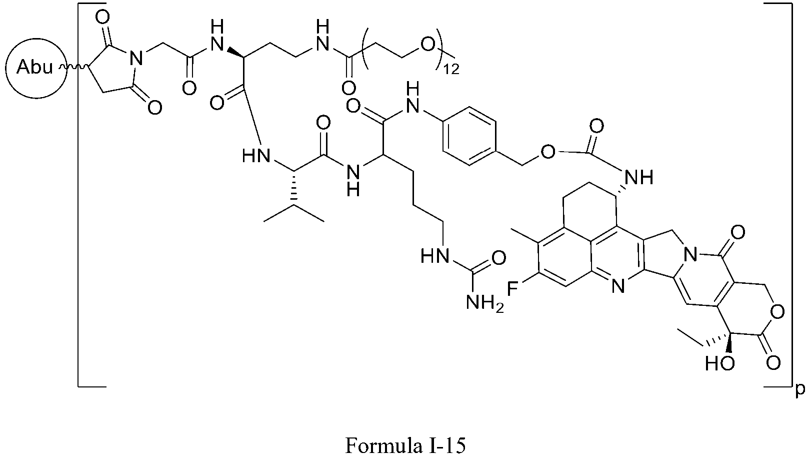 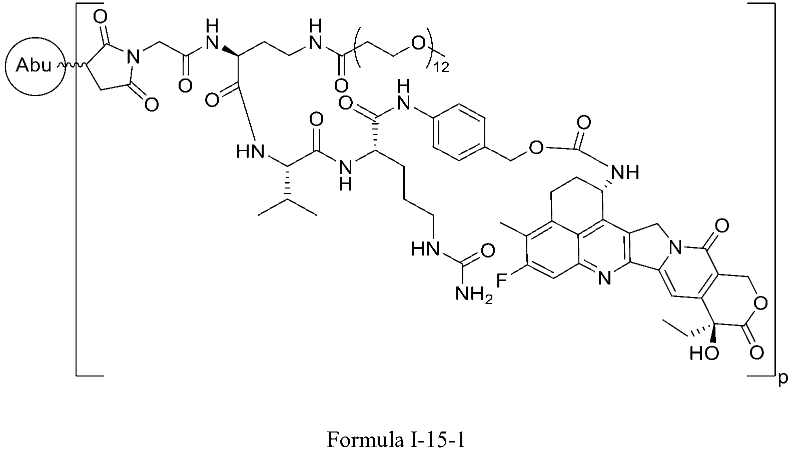 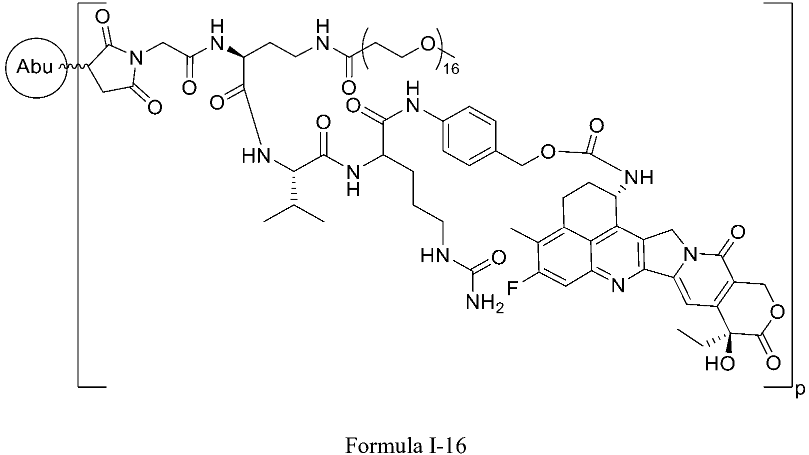 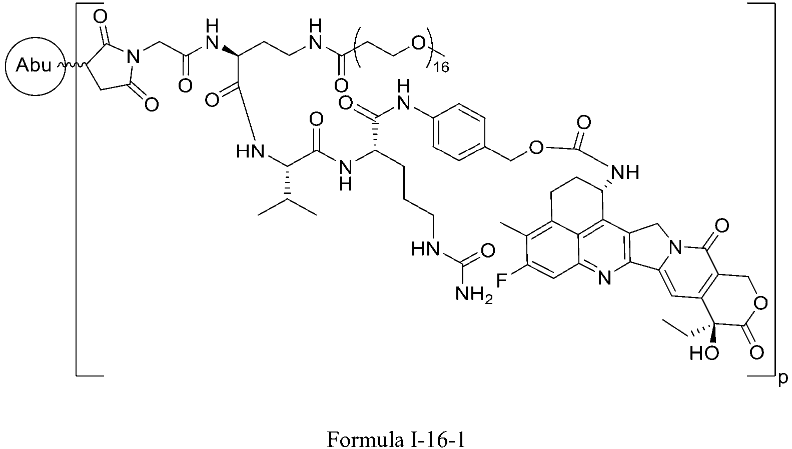 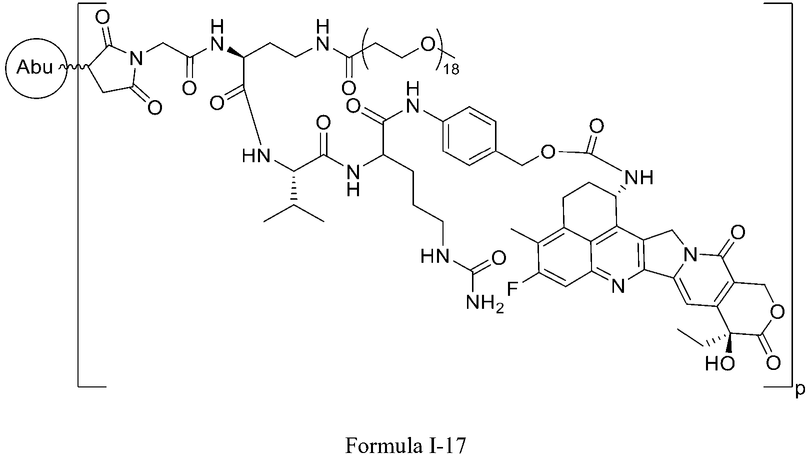 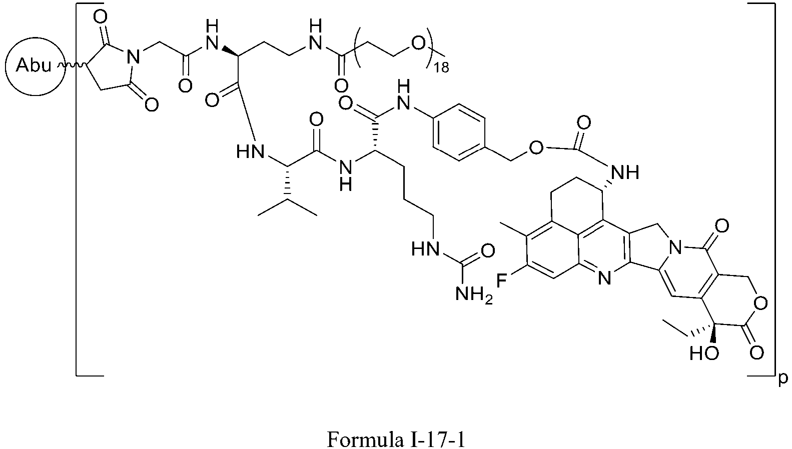 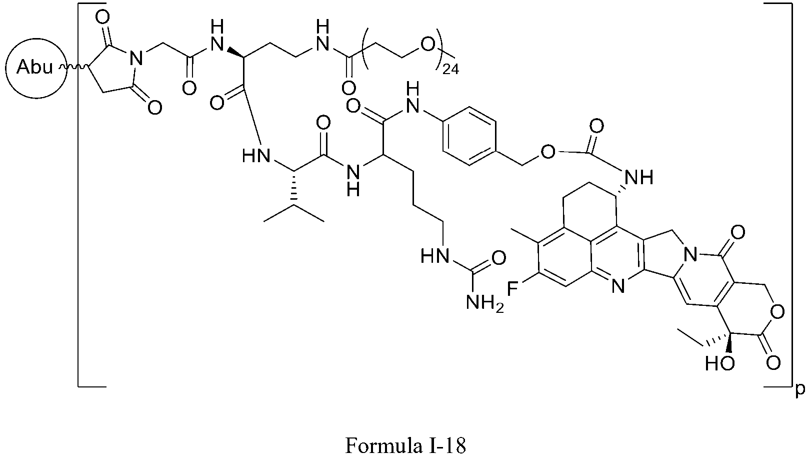 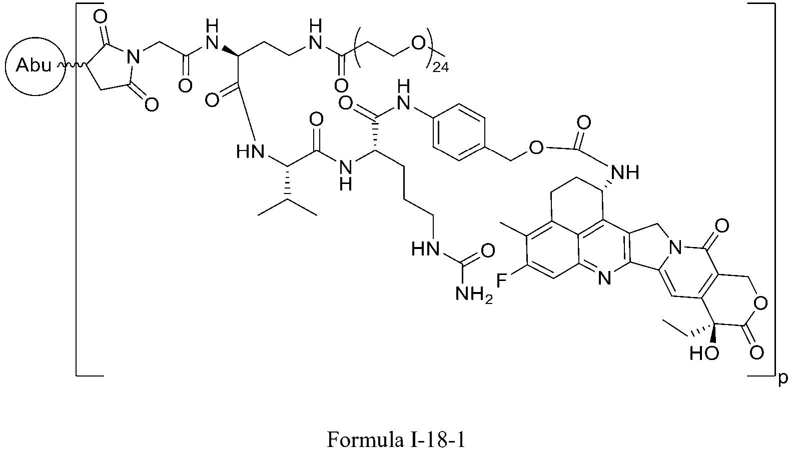 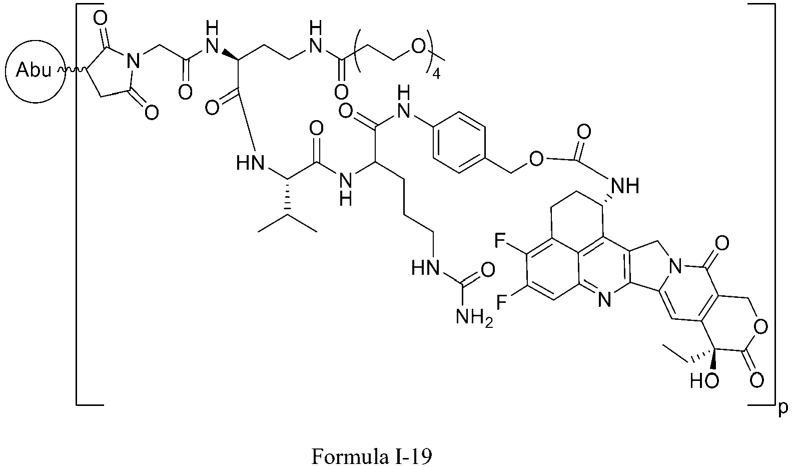 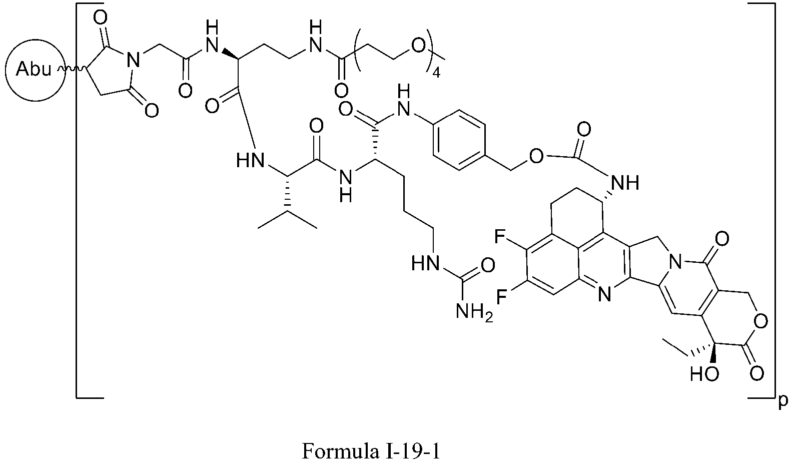 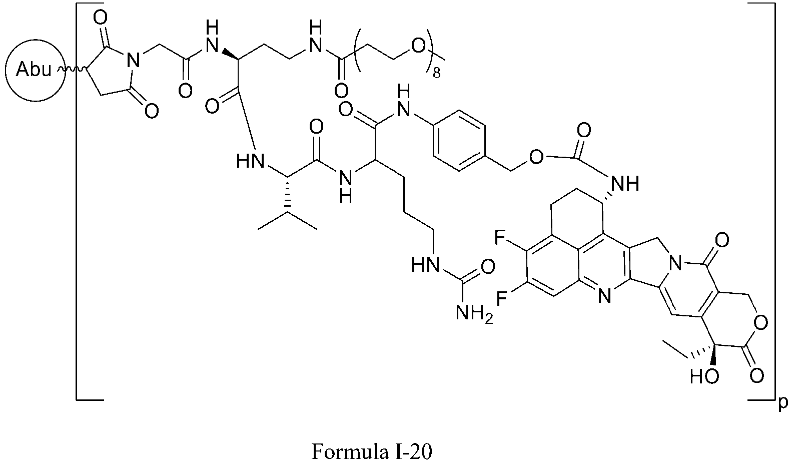 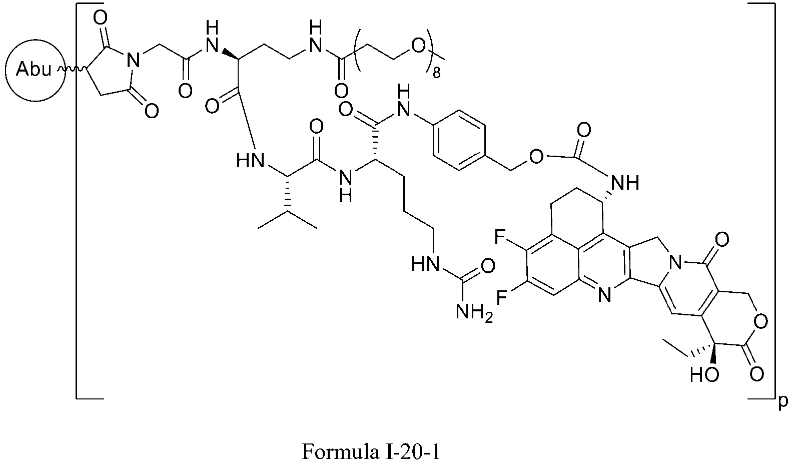 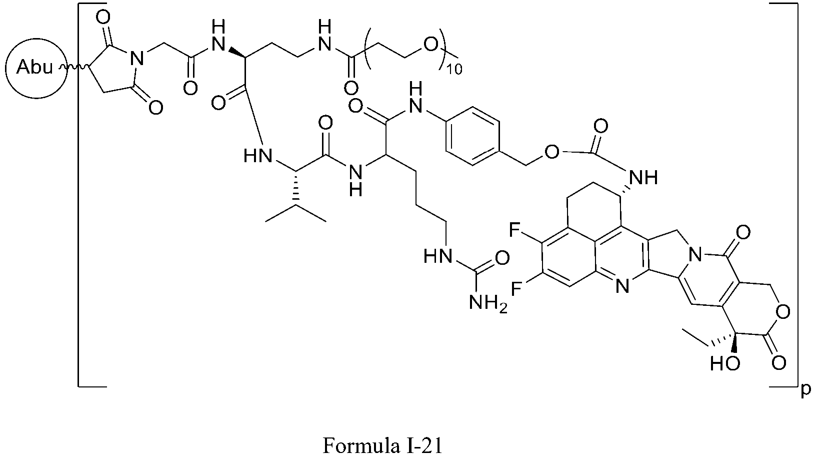 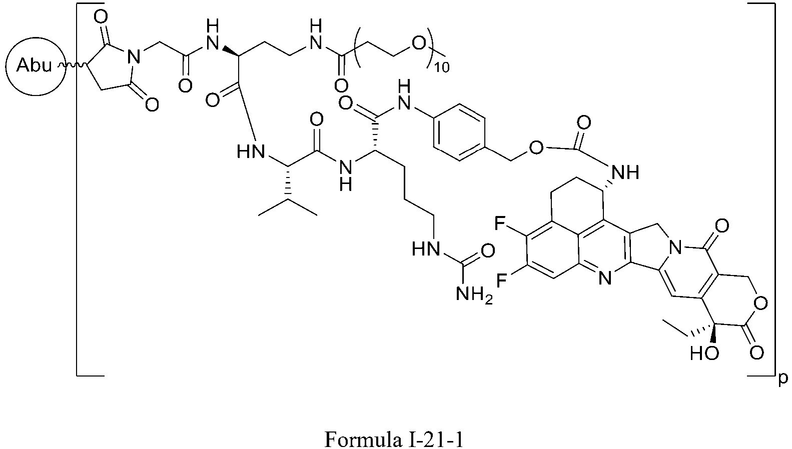 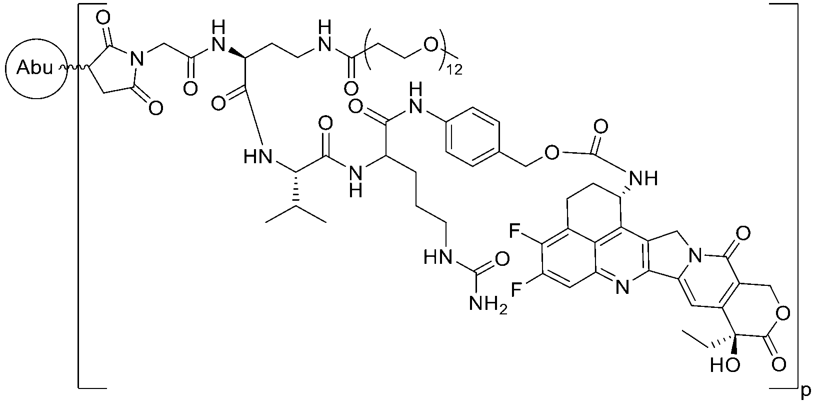 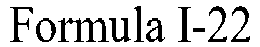 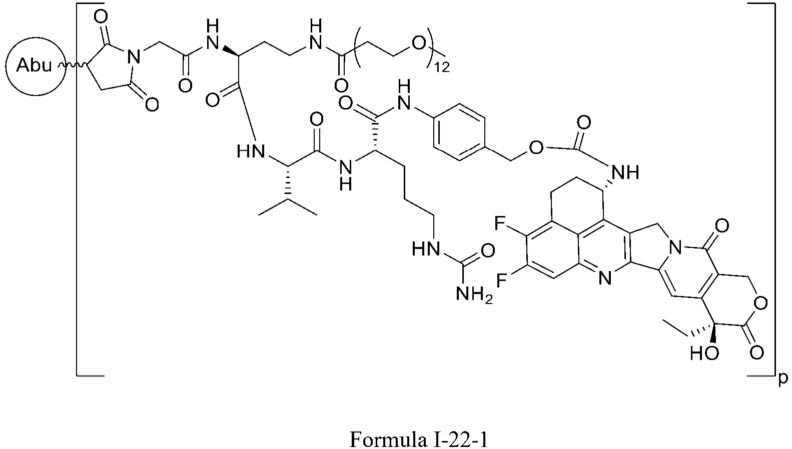 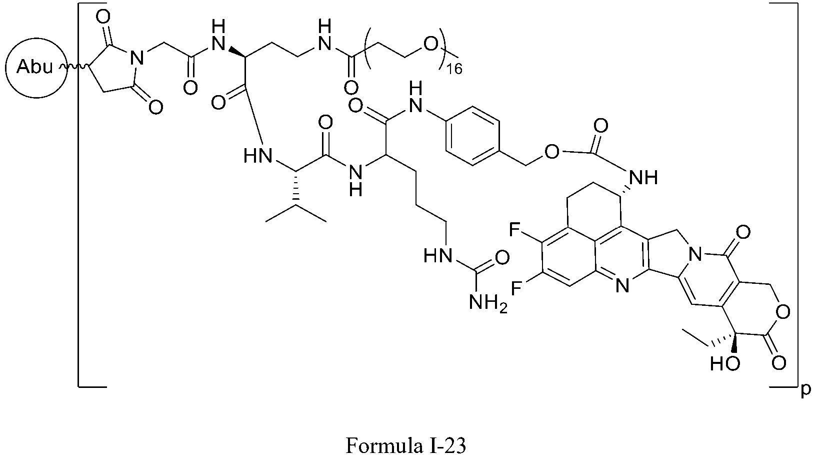 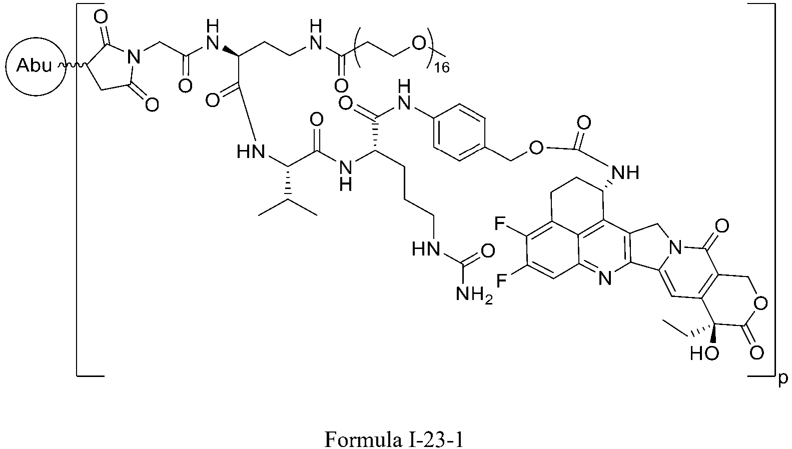 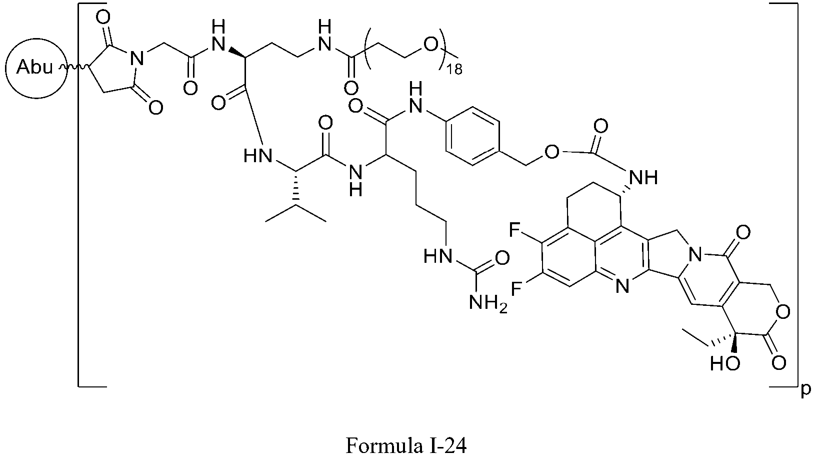 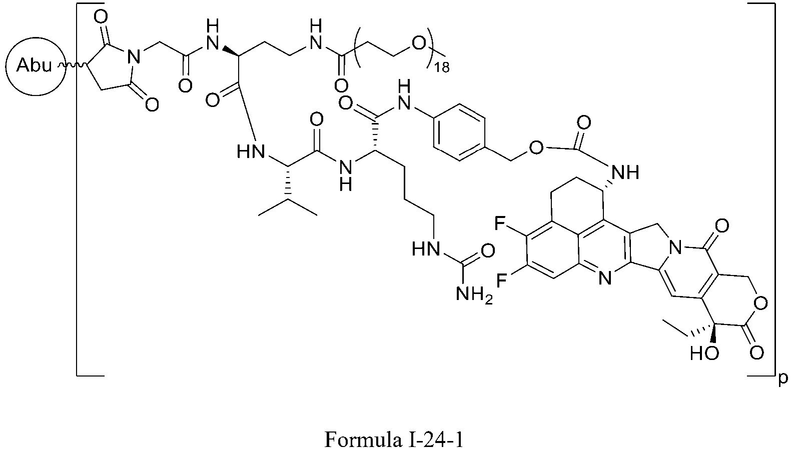 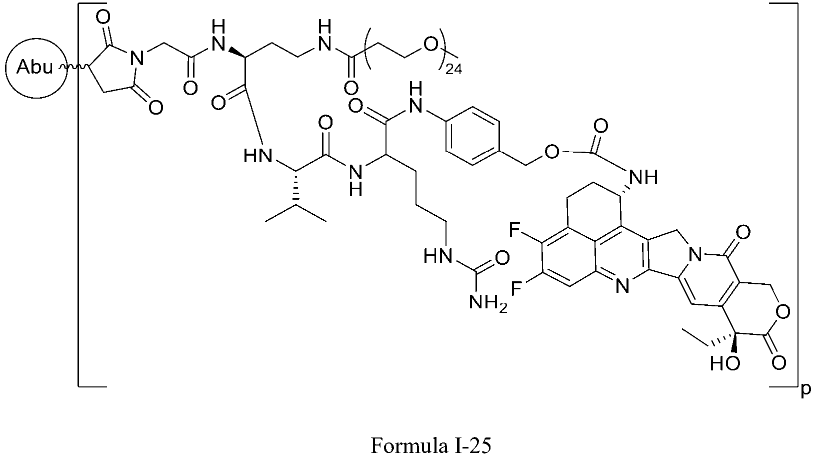 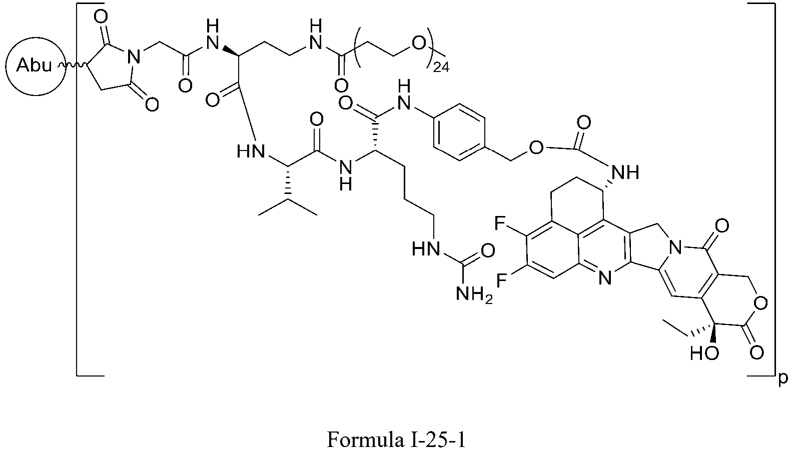 wherein
Abu is an anti-FRα antibody or an antigen-binding unit thereof;
n is an integer from 1 to 24, e.g., 1, 2, 3, 4, 5, 6, 7, 8, 9, 10, 11, 12, 13, 14, 15, 16, 17, 18, 19, 20, 21, 22, 23 or 24;
p is 1-10, e.g., 1, 2, 3, 4, 5, 6, 7, 7.4, 8, 9 or 10.

In one or more embodiments, n is an integer from 4-12.

In one or more embodiments, n is an integer from 4-8.

In one or more embodiments, n is 4.

In one or more embodiments, n is 8.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the anti-FRα antibody or the antigen-binding unit thereof comprises one or more of (a)-(f):
(a) a VH CDR1 comprising or consisting of an amino acid sequence set forth in SEQ ID NO: 1 or an amino acid sequence having substitutions, deletions or insertions at one or more sites compared with the amino acid sequence set forth in SEQ ID NO: 1;
(b) a VH CDR2 comprising or consisting of an amino acid sequence set forth in SEQ ID NO: 2 or an amino acid sequence having substitutions, deletions or insertions at one or more sites compared with the amino acid sequence set forth in SEQ ID NO: 2;
(c) a VH CDR3 comprising or consisting of an amino acid sequence set forth in SEQ ID NO: 3 or an amino acid sequence having substitutions, deletions or insertions at one or more sites compared with the amino acid sequence set forth in SEQ ID NO: 3;
(d) a VL CDR1 comprising or consisting of an amino acid sequence set forth in SEQ ID NO: 4 or an amino acid sequence having substitutions, deletions or insertions at one or more sites compared with the amino acid sequence set forth in SEQ ID NO: 4;
(e) a VL CDR2 comprising or consisting of an amino acid sequence set forth in SEQ ID NO: 5 or an amino acid sequence having substitutions, deletions or insertions at one or more sites compared with the amino acid sequence set forth in SEQ ID NO: 5;
(f) a VL CDR3 comprising or consisting of an amino acid sequence set forth in SEQ ID NO: 6 or an amino acid sequence having substitutions, deletions or insertions at one or more sites compared with the amino acid sequence set forth in SEQ ID NO: 6.

In one or more embodiments, the anti-FRα antibody or the antigen-binding unit thereof comprises:
(a) a VH CDR1 comprising or consisting of an amino acid sequence set forth in SEQ ID NO: 1 or an amino acid sequence having substitutions, deletions or insertions at one or more sites compared with the amino acid sequence set forth in SEQ ID NO: 1; and
(b) a VH CDR2 comprising or consisting of an amino acid sequence set forth in SEQ ID NO: 2 or an amino acid sequence having substitutions, deletions or insertions at one or more sites compared with the amino acid sequence set forth in SEQ ID NO: 2; and
(c) a VH CDR3 comprising or consisting of an amino acid sequence set forth in SEQ ID NO: 3 or an amino acid sequence having substitutions, deletions or insertions at one or more sites compared with the amino acid sequence set forth in SEQ ID NO: 3.

In one or more embodiments, the anti-FRα antibody or the antigen-binding unit thereof comprises a VH CDR1 set forth in SEQ ID NO: 1, a VH CDR2 set forth in SEQ ID NO: 2 and a VH CDR3 set forth in SEQ ID NO: 3.

In one or more embodiments, the anti-FRα antibody or the antigen-binding unit thereof comprises:
(d) a VL CDR1 comprising or consisting of an amino acid sequence set forth in SEQ ID NO: 4 or an amino acid sequence having substitutions, deletions or insertions at one or more sites compared with the amino acid sequence set forth in SEQ ID NO: 4; and
(e) a VL CDR2 comprising or consisting of an amino acid sequence set forth in SEQ ID NO: 5 or an amino acid sequence having substitutions, deletions or insertions at one or more sites compared with the amino acid sequence set forth in SEQ ID NO: 5; and
(f) a VL CDR3 comprising or consisting of an amino acid sequence set forth in SEQ ID NO: 6 or an amino acid sequence having substitutions, deletions or insertions at one or more sites compared with the amino acid sequence set forth in SEQ ID NO: 6.

In one or more embodiments, the anti-FRα antibody or the antigen-binding unit thereof comprises a VL CDR1 set forth in SEQ ID NO: 4, a VL CDR2 set forth in SEQ ID NO: 5 and a VL CDR3 set forth in SEQ ID NO: 6.

In one or more embodiments, the anti-FRα antibody or the antigen-binding unit thereof comprises:
(a) a VH CDR1 comprising or consisting of an amino acid sequence set forth in SEQ ID NO: 1 or an amino acid sequence having substitutions, deletions or insertions at one or more sites compared with the amino acid sequence set forth in SEQ ID NO: 1; and
(b) a VH CDR2 comprising or consisting of an amino acid sequence set forth in SEQ ID NO: 2 or an amino acid sequence having substitutions, deletions or insertions at one or more sites compared with the amino acid sequence set forth in SEQ ID NO: 2; and
(c) a VH CDR3 comprising or consisting of an amino acid sequence set forth in SEQ ID NO: 3 or an amino acid sequence having substitutions, deletions or insertions at one or more sites compared with the amino acid sequence set forth in SEQ ID NO: 3; and
(d) a VL CDR1 comprising or consisting of an amino acid sequence set forth in SEQ ID NO: 4 or an amino acid sequence having substitutions, deletions or insertions at one or more sites compared with the amino acid sequence set forth in SEQ ID NO: 4; and
(e) a VL CDR2 comprising or consisting of an amino acid sequence set forth in SEQ ID NO: 5 or an amino acid sequence having substitutions, deletions or insertions at one or more sites compared with the amino acid sequence set forth in SEQ ID NO: 5; and
(f) a VL CDR3 comprising or consisting of an amino acid sequence set forth in SEQ ID NO: 6 or an amino acid sequence having substitutions, deletions or insertions at one or more sites compared with the amino acid sequence set forth in SEQ ID NO: 6.

In one or more embodiments, the substitutions, deletions or insertions at sites are independently one, two or three.

In one or more embodiments, the anti-FRα antibody or the antigen-binding unit thereof comprises a VH CDR1 set forth in SEQ ID NO: 1, a VH CDR2 set forth in SEQ ID NO: 2, a VH CDR3 set forth in SEQ ID NO: 3, a VL CDR1 set forth in SEQ ID NO: 4, a VL CDR2 set forth in SEQ ID NO: 5 and a VL CDR3 set forth in SEQ ID NO: 6.

In one or more embodiments, the anti-FRα antibody or the antigen-binding unit thereof further comprises one or more of (g)-(n):
(g) a VH FR1 comprising an amino acid sequence set forth in SEQ ID NO: 15, or an amino acid sequence having at least 90% sequence identity compared with the amino acid sequence set forth in SEQ ID NO: 15, or an amino acid sequence having substitutions, deletions or insertions at one or more sites compared with the amino acid sequence set forth in SEQ ID NO: 15;
(h) a VH FR2 comprising an amino acid sequence set forth in SEQ ID NO: 16, or an amino acid sequence having at least 90% sequence identity compared with the amino acid sequence set forth in SEQ ID NO: 16, or an amino acid sequence having substitutions, deletions or insertions at one or more sites compared with the amino acid sequence set forth in SEQ ID NO: 16;
(i) a VH FR3 comprising an amino acid sequence set forth in SEQ ID NO: 17, or an amino acid sequence having at least 90% sequence identity compared with the amino acid sequence set forth in SEQ ID NO: 17, or an amino acid sequence having substitutions, deletions or insertions at one or more sites compared with the amino acid sequence set forth in SEQ ID NO: 17;
(j) a VH FR4 comprising an amino acid sequence set forth in SEQ ID NO: 18, or an amino acid sequence having at least 90% sequence identity compared with the amino acid sequence set forth in SEQ ID NO: 18, or an amino acid sequence having substitutions, deletions or insertions at one or more sites compared with the amino acid sequence set forth in SEQ ID NO: 18;
(k) a VL FR1 comprising an amino acid sequence set forth in SEQ ID NO: 19, or an amino acid sequence having at least 90% sequence identity compared with the amino acid sequence set forth in SEQ ID NO: 19, or an amino acid sequence having substitutions, deletions or insertions at one or more sites compared with the amino acid sequence set forth in SEQ ID NO: 19;
(l) a VL FR2 comprising an amino acid sequence set forth in SEQ ID NO: 20, or an amino acid sequence having at least 90% sequence identity compared with the amino acid sequence set forth in SEQ ID NO: 20, or an amino acid sequence having substitutions, deletions or insertions at one or more sites compared with the amino acid sequence set forth in SEQ ID NO: 20;
(m) a VL FR3 comprising an amino acid sequence set forth in SEQ ID NO: 21, or an amino acid sequence having at least 90% sequence identity compared with the amino acid sequence set forth in SEQ ID NO: 21, or an amino acid sequence having substitutions, deletions or insertions at one or more sites compared with the amino acid sequence set forth in SEQ ID NO: 21; and
(n) a VL FR4 comprising an amino acid sequence set forth in SEQ ID NO: 22, or an amino acid sequence having at least 90% sequence identity compared with the amino acid sequence set forth in SEQ ID NO: 22, or an amino acid sequence having substitutions, deletions or insertions at one or more sites compared with the amino acid sequence set forth in SEQ ID NO: 22.

In one or more embodiments, the anti-FRα antibody or the antigen-binding unit thereof comprises:
(g) a VH FR1 comprising an amino acid sequence set forth in SEQ ID NO: 15, or an amino acid sequence having at least 90% sequence identity compared with the amino acid sequence set forth in SEQ ID NO: 15, or an amino acid sequence having substitutions, deletions or insertions at one or more sites compared with the amino acid sequence set forth in SEQ ID NO: 15; and
(h) a VH FR2 comprising an amino acid sequence set forth in SEQ ID NO: 16, or an amino acid sequence having at least 90% sequence identity compared with the amino acid sequence set forth in SEQ ID NO: 16, or an amino acid sequence having substitutions, deletions or insertions at one or more sites compared with the amino acid sequence set forth in SEQ ID NO: 16; and
(i) a VH FR3 comprising an amino acid sequence set forth in SEQ ID NO: 17, or an amino acid sequence having at least 90% sequence identity compared with the amino acid sequence set forth in SEQ ID NO: 17, or an amino acid sequence having substitutions, deletions or insertions at one or more sites compared with the amino acid sequence set forth in SEQ ID NO: 17; and
(j) a VH FR4 comprising an amino acid sequence set forth in SEQ ID NO: 18, or an amino acid sequence having at least 90% sequence identity compared with the amino acid sequence set forth in SEQ ID NO: 18, or an amino acid sequence having substitutions, deletions or insertions at one or more sites compared with the amino acid sequence set forth in SEQ ID NO: 18; and
(k) a VL FR1 comprising an amino acid sequence set forth in SEQ ID NO: 19, or an amino acid sequence having at least 90% sequence identity compared with the amino acid sequence set forth in SEQ ID NO: 19, or an amino acid sequence having substitutions, deletions or insertions at one or more sites compared with the amino acid sequence set forth in SEQ ID NO: 19; and
(l) a VL FR2 comprising an amino acid sequence set forth in SEQ ID NO: 20, or an amino acid sequence having at least 90% sequence identity compared with the amino acid sequence set forth in SEQ ID NO: 20, or an amino acid sequence having substitutions, deletions or insertions at one or more sites compared with the amino acid sequence set forth in SEQ ID NO: 20; and
(m) a VL FR3 comprising an amino acid sequence set forth in SEQ ID NO: 21, or an amino acid sequence having at least 90% sequence identity compared with the amino acid sequence set forth in SEQ ID NO: 21, or an amino acid sequence having substitutions, deletions or insertions at one or more sites compared with the amino acid sequence set forth in SEQ ID NO: 21; and
(n) a VL FR4 comprising an amino acid sequence set forth in SEQ ID NO: 22, or an amino acid sequence having at least 90% sequence identity compared with the amino acid sequence set forth in SEQ ID NO: 22, or an amino acid sequence having substitutions, deletions or insertions at one or more sites compared with the amino acid sequence set forth in SEQ ID NO: 22.

In one or more embodiments, the anti-FRα antibody or the antigen-binding unit thereof comprises a VH FR1 set forth in SEQ ID NO: 15, a VH FR2 set forth in SEQ ID NO: 16, a VH FR3 set forth in SEQ ID NO: 17 and a VH FR4 set forth in SEQ ID NO: 18.

In one or more embodiments, the anti-FRα antibody or the antigen-binding unit thereof comprises a VL FR1 set forth in SEQ ID NO: 19, a VL FR2 set forth in SEQ ID NO: 20, a VL FR3 set forth in SEQ ID NO: 21 and a VL FR4 set forth in SEQ ID NO: 22.

In one or more embodiments, the anti-FRα antibody or the antigen-binding unit thereof comprises a VH FR1 set forth in SEQ ID NO: 15, a VH FR2 set forth in SEQ ID NO: 16, a VH FR3 set forth in SEQ ID NO: 17, a VH FR4 set forth in SEQ ID NO: 18, a VL FR1 set forth in SEQ ID NO: 19, a VL FR2 set forth in SEQ ID NO: 20, a VL FR3 set forth in SEQ ID NO: 21 and a VL FR4 set forth in SEQ ID NO: 22.

In one or more embodiments, the anti-FRα antibody or the antigen-binding unit thereof comprises a heavy chain variable region (VH) and/or a light chain variable region (VL), wherein:
the heavy chain variable region comprises an amino acid sequence set forth in SEQ ID NO: 7, or an amino acid sequence having at least 90% sequence identity compared with the amino acid sequence set forth in SEQ ID NO: 7, or an amino acid sequence having substitutions, deletions or insertions at one or more sites compared with the amino acid sequence set forth in SEQ ID NO: 7; and/or
the light chain variable region comprises an amino acid sequence set forth in SEQ ID NO: 8, or an amino acid sequence having at least 90% sequence identity compared with the amino acid sequence set forth in SEQ ID NO: 8, or an amino acid sequence having substitutions, deletions or insertions at one or more sites compared with the amino acid sequence set forth in SEQ ID NO: 8.

In one or more embodiments, the anti-FRα antibody or the antigen-binding unit thereof comprises a heavy chain variable region set forth in SEQ ID NO: 7 and a light chain variable region set forth in SEQ ID NO: 8.

In one or more embodiments, the anti-FRα antibody or the antigen-binding unit thereof comprises a heavy chain constant region (CH) and/or a light chain constant region (CL), wherein:
the heavy chain constant region comprises an amino acid sequence set forth in SEQ ID NO: 9, or an amino acid sequence having at least 90% sequence identity compared with the amino acid sequence set forth in SEQ ID NO: 9, or an amino acid sequence having substitutions, deletions or insertions at one or more sites compared with the amino acid sequence set forth in SEQ ID NO: 9; and/or
the light chain constant region comprises an amino acid sequence set forth in SEQ ID NO: 10, or an amino acid sequence having at least 90% sequence identity compared with the amino acid sequence set forth in SEQ ID NO: 10, or an amino acid sequence having substitutions, deletions or insertions at one or more sites compared with the amino acid sequence set forth in SEQ ID NO: 10.

In one or more embodiments, the anti-FRα antibody or the antigen-binding unit thereof comprises a heavy chain constant region set forth in SEQ ID NO: 9 and a light chain constant region set forth in SEQ ID NO: 10.

In one or more embodiments, the anti-FRα antibody comprises a heavy chain (H) and a light chain (L).

In one or more embodiments, the heavy chain of the anti-FRα antibody comprises a heavy chain variable region set forth in SEQ ID NO: 7 and a heavy chain constant region set forth in SEQ ID NO: 9; the light chain of the anti-FRα antibody comprises a light chain variable region set forth in SEQ ID NO: 8 and a light chain constant region set forth in SEQ ID NO: 10.

In one or more embodiments, the heavy chain comprises an amino acid sequence set forth in SEQ ID NO: 11, or an amino acid sequence having at least 90% sequence identity compared with the amino acid sequence set forth in SEQ ID NO: 11, or an amino acid sequence having substitutions, deletions or insertions at one or more sites compared with the amino acid sequence set forth in SEQ ID NO: 11; and/or
the light chain comprises an amino acid sequence set forth in SEQ ID NO: 13, or an amino acid sequence having at least 90% sequence identity compared with the amino acid sequence set forth in SEQ ID NO: 13, or an amino acid sequence having substitutions, deletions or insertions at one or more sites compared with the amino acid sequence set forth in SEQ ID NO: 13.

In one or more embodiments, the anti-FRα antibody comprises a heavy chain set forth in SEQ ID NO: 11 and a light chain set forth in SEQ ID NO: 13.

In one or more embodiments, the anti-FRα antibody is Antibody 1.

In one or more embodiments, the substitution is a conservative amino acid substitution.

In one or more embodiments, the antibody or the antigen-binding unit thereof is chimeric, humanized or fully human.

In one or more embodiments, the anti-FRα antibody is a monoclonal antibody or a multispecific antibody or antigen-binding unit thereof (e.g., a bispecific antibody or antigen-binding unit thereof).

In one or more embodiments, the anti-FRα antibody has two heavy chains with the same sequence and two light chains with the same sequence. In one or more embodiments, the Fc regions pair to form disulfide bonds.

In one or more embodiments, the anti-FRα antibody or the antigen-binding unit thereof is an isolated antibody or an antigen-binding unit thereof.

In one or more embodiments, the anti-FRα antibody or the antigen-binding unit thereof is a monoclonal antibody or a fragment thereof.

In one or more embodiments, the patient has an FRα expression of ≥1%. In one or more embodiments, the patient has an FRα TPS of ≥1%. In one or more embodiments, the patient has an FRα expression of >25%. In one or more embodiments, the patient has an FRα TPS of >25%. In one or more embodiments, the patient is determined (e.g., by immunohistochemistry, flow cytometry, nucleic acid hybridization, etc.) to have an FRα TPS of >25%. In one or more embodiments, the patient is determined to have an FRα TPS of >50%. In one or more embodiments, the patient has an FRα expression of <50%. In one or more embodiments, the patient has an FRα TPS of <50%. In one or more embodiments, the patient has an FRα expression of ≥50%. In one or more embodiments, the patient has an FRα TPS of ≥50%. In one or more embodiments, the patient has an FRα expression of ≥75%. In one or more embodiments, the patient has an FRα TPS of ≥75%.

In one or more embodiments, the present invention provides a method for treating cancer, which comprises administering to a patient with an FRα TPS of >25% an effective amount of an antibody-drug conjugate, the antibody-drug conjugate has a structure of Formula I-13-1, or a stereoisomer thereof, or a pharmaceutically acceptable salt or solvate thereof: 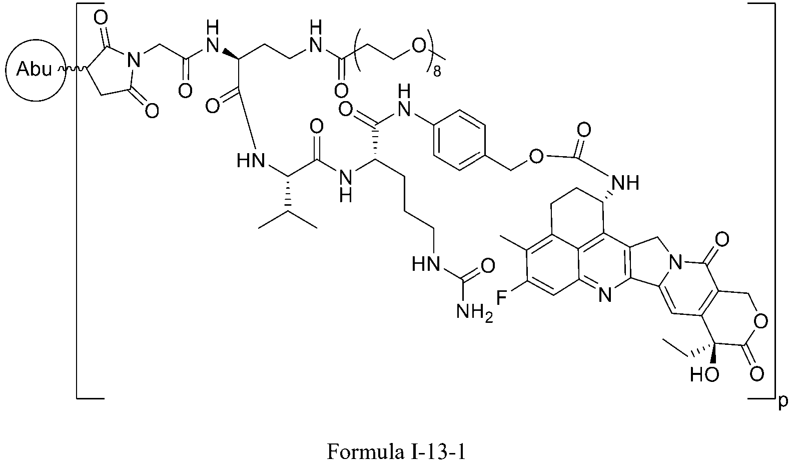 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 7-8.

In one or more embodiments, the antibody-drug conjugate is administered at a dose of 0.5 mg/kg to 5 mg/kg at a frequency of once per day to once every 7 weeks.

In one or more embodiments, the antibody-drug conjugate is administered at a dose of 10 mg to 300 mg at a frequency of once per day to once every 7 weeks.

In one or more embodiments, the antibody-drug conjugate is administered at a dose of 5 mg/m² to 150 mg/m² at a frequency of once per day to once every 7 weeks.

In one or more embodiments, p is 7.4.

In one or more embodiments, p is 7.

In one or more embodiments, p is 8.

In one or more embodiments, the present invention provides a method for treating cancer, which comprises administering to a patient with an FRα TPS of >25% an effective amount of an antibody-drug conjugate, the antibody-drug conjugate is administered at a dose of 0.5 mg/kg to 5 mg/kg at a frequency of once per day to once every 7 weeks.

In one or more embodiments, the present invention provides a method for treating cancer, which comprises administering to a patient with an FRα TPS of >25% an effective amount of an antibody-drug conjugate, the antibody-drug conjugate is administered at a dose of 10 mg to 300 mg at a frequency of once per day to once every 7 weeks.

In one or more embodiments, the present invention provides a method for treating cancer, which comprises administering to a patient with an FRα TPS of >25% an effective amount of an antibody-drug conjugate, the antibody-drug conjugate is administered at a dose of 5 mg/m² to 150 mg/m² at a frequency of once per day to once every 7 weeks.

In one or more embodiments, the patient has an FRα TPS of >50%. In one or more embodiments, the patient has an FRα TPS of <50%. In one or more embodiments, the patient has an FRα TPS of ≥50%. In one or more embodiments, the patient has an FRα TPS of ≥75%.

In one or more embodiments, the antibody-drug conjugate has a structure of Formula I-13-1, or a stereoisomer thereof, or a pharmaceutically acceptable salt or solvate thereof: 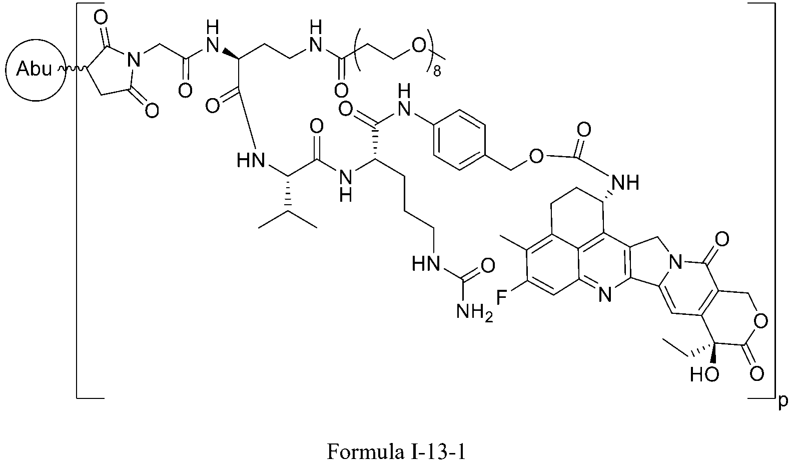 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 1-10.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the present invention provides a method for treating cancer, which comprises administering to a patient with an FRα TPS of >25% an effective amount of an antibody-drug conjugate, wherein the antibody-drug conjugate is administered at a dose of 0.5 mg/kg to 5 mg/kg, or 10 mg to 300 mg, or 5 mg/m² to 150 mg/m², at a frequency of once per day to once every 7 weeks; the antibody-drug conjugate has a structure of Formula I-13-1, or a stereoisomer thereof, or a pharmaceutically acceptable salt or solvate thereof: 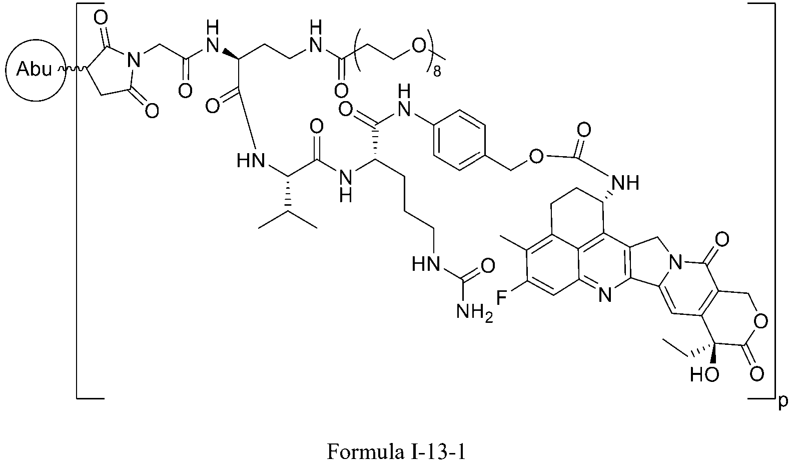 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 1-10.

In one or more embodiments, p is 2-8.

In one or more embodiments, p is 4-8.

In one or more embodiments, p is 6-8.

In one or more embodiments, p is 7-8.

In one or more embodiments, p is 7.4.

In one or more embodiments, p is 7.

In one or more embodiments, p is 8.

In one or more embodiments, the antibody-drug conjugate is ADC1 or a pharmaceutically acceptable salt or solvate thereof, wherein
ADC1 is 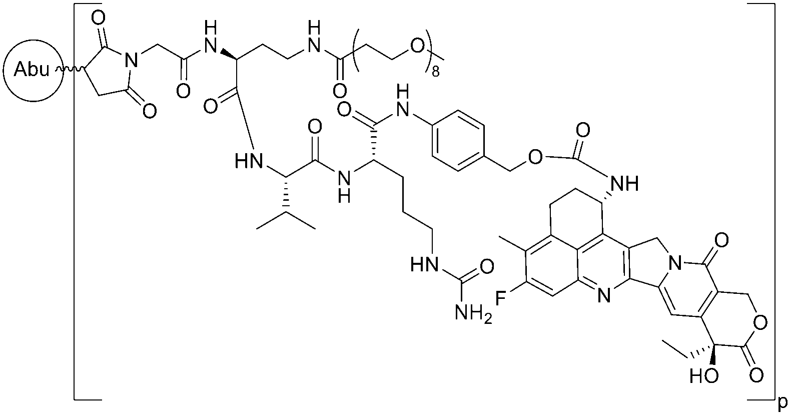 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 7-8.

In one or more embodiments, the antibody-drug conjugate is ADC2 or a pharmaceutically acceptable salt or solvate thereof, wherein
ADC2 is 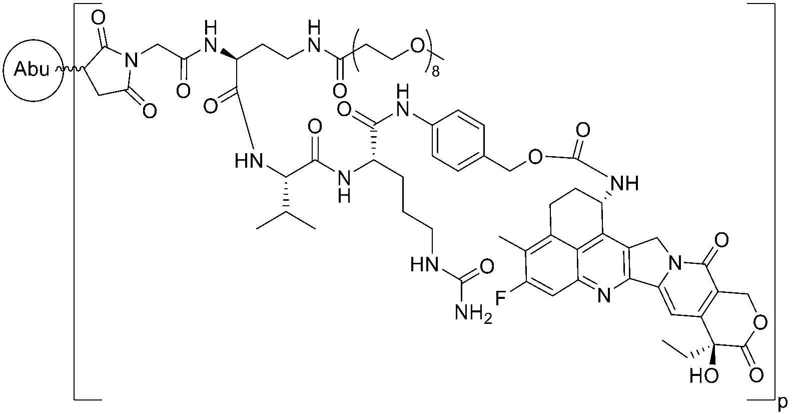 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 3.5-4.5.

In one or more embodiments, administered to the patient is a pharmaceutical composition comprising the antibody-drug conjugate described herein, and a pharmaceutically acceptable carrier, an excipient, and/or an adjuvant.

In one or more embodiments, the disease is a disease associated with the expression of FRα. In one or more embodiments, the disease is a disease associated with the overexpression of FRα. In one or more embodiments, the disease is a tumor. In one or more embodiments, the disease is a tumor expressing FRα. In one or more embodiments, the disease is a tumor overexpressing FRα. In one or more embodiments, the disease is cancer. In one or more embodiments, the disease is cancer expressing FRα. In one or more embodiments, the disease is cancer overexpressing FRα. In one or more embodiments, the cancer is cancer with an FRα expression of ≥1%. In one or more embodiments, the cancer is cancer with an FRα TPS (Tumor cell Proportion Score) of ≥1%. In one or more embodiments, the cancer is cancer with an FRα expression of >25%. In one or more embodiments, the cancer is cancer with an FRα TPS (Tumor cell Proportion Score) of >25%. In one or more embodiments, the cancer is determined (e.g., by immunohistochemistry(IHC), flow cytometry, nucleic acid hybridization, etc.) to have an FRα TPS of >25%. In one or more embodiments, the cancer is cancer with an FRα expression of <50%. In one or more embodiments, the cancer is cancer with an FRα TPS of <50%. In one or more embodiments, the cancer is cancer with an FRα expression of ≥50%. In one or more embodiments, the cancer is cancer with an FRα TPS of ≥50%. In one or more embodiments, the cancer is cancer with an FRα expression of ≥75%. In one or more embodiments, the cancer is cancer with an FRα TPS of ≥75%.

Examples of cancers include, but are not limited to, solid tumors, hematological cancers and metastatic lesions. Specific examples of such cancers include, but are not limited to, colorectal cancer, lung cancer (e.g., non-small cell lung cancer(NSCLC)), ovarian cancer, uterine cancer, endometrial cancer, peritoneal cancer, fallopian tube cancer, pancreatic cancer, head and neck squamous cell carcinoma, nasopharyngeal cancer, laryngeal cancer, lung adenocarcinoma, liver cancer, breast cancer, brain cancer, renal cancer, renal cell carcinoma, colon cancer, testicular cancer, uterine cervical cancer, cervical cancer, tongue cancer, bladder cancer, retinoblastoma, glioblastoma, mesothelioma, oral epithelioid cancer, choriocarcinoma and head and neck cancer. In one or more embodiments, the cancer is advanced or metastatic cancer. In one or more embodiments, the cancer is platinum-refractory cancer. In one or more embodiments, the cancer is platinum-refractory ovarian cancer. In one or more embodiments, the patient has platinum-refractory advanced or metastatic ovarian cancer. In one or more embodiments, the cancer is selected from platinum-refractory epithelial ovarian cancer, fallopian tube cancer, or primary peritoneal cancer. In one or more embodiments, the patient has platinum-refractory advanced or metastatic epithelial ovarian cancer, primary peritoneal cancer, or fallopian tube cancer. In one or more embodiments, the cancer is platinum-resistant cancer. In one or more embodiments, the cancer is platinum-resistant ovarian cancer. In one or more embodiments, the patient has platinum-resistant advanced or metastatic ovarian cancer. In one or more embodiments, the cancer is selected from platinum-resistant epithelial ovarian cancer, fallopian tube cancer, or primary peritoneal cancer. In one or more embodiments, the patient has platinum-resistant advanced or metastatic epithelial ovarian cancer, primary peritoneal cancer, or fallopian tube cancer.

In one or more embodiments, the patient has received prior bevacizumab treatment. In one or more embodiments, the patient has received prior poly ADP-ribose polymerase inhibitor (PARPi) treatment.

In one or more embodiments, the patient has platinum-resistant advanced or metastatic epithelial ovarian cancer, primary peritoneal cancer, or fallopian tube cancer with no more than three prior lines of therapy.

In one or more embodiments, the patient has advanced or metastatic solid tumors that have failed or were intolerant to standard therapy.

In one or more embodiments, the patient has advanced or metastatic endometrial cancer, breast cancer, or non-small cell lung cancer that has failed or were intolerant to standard therapy.

In one or more embodiments, the cancer is ovarian cancer with an FRα TPS of >25%. In one or more embodiments, the ovarian cancer is determined (e.g., by immunohistochemistry) to have an FRα TPS of >25%. In one or more embodiments, the cancer is ovarian cancer with an FRα TPS of >50%. In one or more embodiments, the ovarian cancer is determined (e.g., by immunohistochemistry) to have an FRα TPS of >50%. In one or more embodiments, the cancer is ovarian cancer with an FRα expression of <50%. In one or more embodiments, the cancer is ovarian cancer with an FRα TPS of <50%. In one or more embodiments, the cancer is ovarian cancer with an FRα expression of ≥50%. In one or more embodiments, the cancer is ovarian cancer with an FRα TPS of ≥50%. In one or more embodiments, the cancer is ovarian cancer with an FRα expression of ≥75%. In one or more embodiments, the cancer is ovarian cancer with an FRα TPS of ≥75%.

In one or more embodiments, the present invention provides a method for treating cancer (e.g., ovarian cancer), which comprises administering to a patient having cancer (e.g., ovarian cancer) with an FRα TPS of >25% an effective amount of ADC1. In one or more embodiments, the effective amount refers to the amount of an active compound or drug that results in a biological or drug response of tissues, systems, animals, individuals and humans which is being sought by researchers, vets, doctors or other clinical doctors, including the treatment of a disease. In one or more embodiments, the cancer has been treated with radiotherapy, chemotherapy, targeted therapy (e.g., PARPi therapy), and/or surgical therapy.

In one or more embodiments, the antibody-drug conjugate may be formulated into a pharmaceutical composition and administered to a patient in a form suitable for the chosen route of administration, e.g., parenteral, intravenous (iv), intramuscular, topical or subcutaneous administration.

In one or more embodiments, the antibody-drug conjugate (e.g., ADC1 or ADC2) is administered at a dose of 0.5 mg/kg-5 mg/kg; or 0.5 mg/kg-3 mg/kg; or 1.3 mg/kg-3 mg/kg; or 1.7 mg/kg-2.5 mg/kg. In one or more embodiments, the antibody-drug conjugate (e.g., ADC1 or ADC2) is administered at a dose of about 0.5 mg/kg, about 1 mg/kg, about 1.2 mg/kg, about 1.5 mg/kg, about 1.8 mg/kg, about 2.1 mg/kg, about 2.4 mg/kg, about 3.0 mg/kg, about 3.6 mg/kg, about 4.0 mg/kg, about 4.5 mg/kg, about 5.0 mg/kg, or a range between any two of these values (inclusive) or any value therein.

In one or more embodiments, the antibody-drug conjugate described herein (e.g., ADC1 or ADC2) administered per dose is 0.5 mg/kg to 5 mg/kg; or 0.5 mg/kg-3 mg/kg; or 1.3 mg/kg-3 mg/kg; or 1.7 mg/kg-2.5 mg/kg, or a formulation comprising the antibody-drug conjugate (e.g., ADC1 or ADC2) at said dose. In one or more embodiments, the antibody-drug conjugate described herein (e.g., ADC1 or ADC2) administered per dose is about 0.5 mg/kg, about 1 mg/kg, about 1.2 mg/kg, about 1.5 mg/kg, about 1.8 mg/kg, about 2.1 mg/kg, about 2.4 mg/kg, about 3.0 mg/kg, about 3.6 mg/kg, about 4.0 mg/kg, about 4.5 mg/kg, about 5.0 mg/kg, or a range between any two of these values (inclusive) or any value therein, or a formulation comprising the antibody-drug conjugate (e.g., ADC1 or ADC2) at said dose. In one or more embodiments, the antibody-drug conjugate is given in single administration. In one or more embodiments, the administration is performed once per day to once every 7 weeks. In one or more embodiments, the administration is performed once every 2 days to once every 6 weeks. In one or more embodiments, the administration is performed once every 2-4 weeks. In one or more embodiments, the administration is performed about twice a week or about once a week or about once every 2 weeks, 3 weeks, 4 weeks, 5 weeks, 6 weeks or 7 weeks. In one or more embodiments, the administration is performed once per day, once every 2 days, once every 3 days, once every 4 days, once every 5 days, twice a week, once a week, once every 2 weeks, once every 3 weeks, once every 4 weeks, once every 5 weeks, once every 6 weeks or once every 7 weeks.

In one or more embodiments, the antibody-drug conjugate described herein (e.g., ADC1 or ADC2) is administered at a dose of 0.5 mg/kg to 5 mg/kg; or 0.5 mg/kg-3 mg/kg; or 1.3 mg/kg-3 mg/kg; or 1.7 mg/kg-2.5 mg/kg once every 3 weeks; or a formulation comprising the antibody-drug conjugate (e.g., ADC1 or ADC2) at said dose is administered once every 3 weeks. In one or more embodiments, the antibody-drug conjugate described herein (e.g., ADC1 or ADC2) is administered at a dose of about 0.5 mg/kg, about 1 mg/kg, about 1.2 mg/kg, about 1.5 mg/kg, about 1.8 mg/kg, about 2.1 mg/kg, about 2.4 mg/kg, about 3.0 mg/kg, about 3.6 mg/kg, about 4.0 mg/kg, about 4.5 mg/kg, about 5.0 mg/kg, or a range between any two of these values (inclusive) or any value therein, or a formulation comprising the antibody-drug conjugate (e.g., ADC1 or ADC2) at said dose is administered once every 3 weeks. In one or more embodiments, the antibody-drug conjugate described herein (e.g., ADC1 or ADC2) is administered at about 1.2 mg/kg once every 3 weeks. In one or more embodiments, the antibody-drug conjugate described herein (e.g., ADC1 or ADC2) is administered at about 1.5 mg/kg once every 3 weeks. In one or more embodiments, the antibody-drug conjugate described herein (e.g., ADC1 or ADC2) is administered at about 1.8 mg/kg once every 3 weeks. In one or more embodiments, the antibody-drug conjugate described herein (e.g., ADC1 or ADC2) is administered at about 2.1 mg/kg once every 3 weeks. In one or more embodiments, the antibody-drug conjugate described herein (e.g., ADC1 or ADC2) is administered at about 2.4 mg/kg once every 3 weeks. In one or more embodiments, the antibody-drug conjugate described herein (e.g., ADC1 or ADC2) is administered at about 3.0 mg/kg once every 3 weeks. In one or more embodiments, the antibody-drug conjugate described herein (e.g., ADC1 or ADC2) is administered at about 3.6 mg/kg once every 3 weeks.

In one or more embodiments, the antibody-drug conjugate (e.g., ADC1 or ADC2) is administered at a dose of 5 mg/m²-150 mg/m²; or 5 mg/m²-120 mg/m²; or 10 mg/m²-120 mg/m²; or 30 mg/m²-120 mg/m²; or 50 mg/m²-120 mg/m²; or 60 mg/m²-120 mg/m²; or 70 mg/m²-100 mg/m²; or 75 mg/m²-100 mg/m²; or 80 mg/m²-95 mg/m²; or 76 mg/m²-93 mg/m²; or 76 mg/m²-84 mg/m²; or 84 mg/m²-93 mg/m²; or 87 mg/m²-93 mg/m². In one or more embodiments, the antibody-drug conjugate (e.g., ADC1 or ADC2) is administered at a dose of about 5 mg/m², about 10 mg/m², about 30 mg/m², about 50 mg/m², about 60 mg/m², about 76 mg/m², about 80 mg/m², about 84 mg/m², about 87 mg/m², about 90 mg/m², about 93 mg/m², about 95 mg/m², about 100 mg/m², about 120 mg/m², about 150 mg/m², or a range between any two of these values (inclusive) or any value therein.

In one or more embodiments, the antibody-drug conjugate described herein (e.g., ADC1 or ADC2) administered per dose is 5 mg/m²-150 mg/m²; or 5 mg/m²-120 mg/m²; or 10 mg/m²-120 mg/m²; or 30 mg/m²-120 mg/m²; or 50 mg/m²-120 mg/m²; or 60 mg/m²-120 mg/m²; or 70 mg/m²-100 mg/m²; or 75 mg/m²-100 mg/m²; or 80 mg/m²-95 mg/m²; or 76 mg/m²-93 mg/m²; or 76 mg/m²-84 mg/m²; or 84 mg/m²-93 mg/m²; or 87 mg/m²-93 mg/m². In one or more embodiments, the antibody-drug conjugate described herein (e.g., ADC1 or ADC2) administered per dose is 5 mg/m², about 10 mg/m², about 30 mg/m², about 50 mg/m², about 60 mg/m², about 76 mg/m², about 80 mg/m², about 84 mg/m², about 87 mg/m², about 90 mg/m², about 93 mg/m², about 95 mg/m², about 100 mg/m², about 120 mg/m², about 150 mg/m², or a range between any two of these values (inclusive) or any value therein. In one or more embodiments, the antibody-drug conjugate is given in single administration. In one or more embodiments, the administration is performed once per day to once every 7 weeks. In one or more embodiments, the administration is performed once every 2 days to once every 6 weeks. In one or more embodiments, the administration is performed once every 2-4 weeks. In one or more embodiments, the administration is performed about twice a week or about once a week or about once every 2 weeks, 3 weeks, 4 weeks, 5 weeks, 6 weeks or 7 weeks. In one or more embodiments, the administration is performed once per day, once every 2 days, once every 3 days, once every 4 days, once every 5 days, twice a week, once a week, once every 2 weeks, once every 3 weeks, once every 4 weeks, once every 5 weeks, once every 6 weeks or once every 7 weeks.

In one or more embodiments, the antibody-drug conjugate described herein (e.g., ADC1 or ADC2) is administered at a dose of 5 mg/m²-150 mg/m²; or 5 mg/m²-120 mg/m²; or 10 mg/m²-120 mg/m²; or 30 mg/m²-120 mg/m²; or 50 mg/m²-120 mg/m²; or 60 mg/m²-120 mg/m²; or 70 mg/m²-100 mg/m²; or 75 mg/m²-100 mg/m²; or 80 mg/m²-95 mg/m²; or 76 mg/m²-93 mg/m²; or 76 mg/m²-84 mg/m²; or 84 mg/m²-93 mg/m²; or 87 mg/m²-93 mg/m² once every 3 weeks; or a formulation comprising the antibody-drug conjugate (e.g., ADC1 or ADC2) at said dose is administered once every 3 weeks. In one or more embodiments, the antibody-drug conjugate described herein (e.g., ADC1 or ADC2) is administered at a dose of about 5 mg/m², about 10 mg/m², about 30 mg/m², about 50 mg/m², about 60 mg/m², about 76 mg/m², about 80 mg/m², about 84 mg/m², about 87 mg/m², about 90 mg/m², about 93 mg/m², about 95 mg/m², about 100 mg/m², about 120 mg/m², about 150 mg/m², or a range between any two of these values (inclusive) or any value therein, or a formulation comprising the antibody-drug conjugate (e.g., ADC1 or ADC2) at said dose is administered once every 3 weeks. In one or more embodiments, the antibody-drug conjugate described herein (e.g., ADC1 or ADC2) is administered at about 76 mg/m² once every 3 weeks. In one or more embodiments, the antibody-drug conjugate described herein (e.g., ADC1 or ADC2) is administered at about 84 mg/m² once every 3 weeks. In one or more embodiments, the antibody-drug conjugate described herein (e.g., ADC1 or ADC2) is administered at about 93 mg/m² once every 3 weeks.

In one or more embodiments, the antibody-drug conjugate is administered at a dose of 10 mg-300 mg; or 30 mg-300 mg; or 50 mg-280 mg; or 100 mg-250 mg. In one or more embodiments, the antibody-drug conjugate is administered at a dose of about 10 mg, about 30 mg, about 50 mg, about 55 mg, about 60 mg, about 65 mg, about 70 mg, about 72 mg, about 75 mg, about 80 mg, about 84 mg, about 90 mg, about 95 mg, about 100 mg, about 105 mg, about 108 mg, about 110 mg, about 120 mg, about 126 mg, about 130 mg, about 140 mg, about 144 mg, about 145 mg, about 150 mg, about 155 mg, about 160 mg, about 165 mg, about 168 mg, about 170 mg, about 175 mg, about 180 mg, about 190 mg, about 200 mg, about 210 mg, about 216 mg, about 220 mg, about 230 mg, about 240 mg, about 245 mg, about 250 mg, about 260 mg, about 270 mg, about 280 mg, about 290 mg, about 300 mg, or a range between any two of these values (inclusive) or any value therein.

In one or more embodiments, the antibody-drug conjugate described herein (e.g., ADC1 or ADC2) administered per dose is 10 mg-300 mg; or 30 mg-300 mg; or 50 mg-280 mg; or 100 mg-250 mg, or a formulation comprising the antibody-drug conjugate (e.g., ADC1 or ADC2) at said dose is administered per dose. In one or more embodiments, the antibody-drug conjugate described herein (e.g., ADC1 or ADC2) administered per dose is about 10 mg, about 30 mg, about 50 mg, about 55 mg, about 60 mg, about 65 mg, about 70 mg, about 72 mg, about 75 mg, about 80 mg, about 84 mg, about 90 mg, about 95 mg, about 100 mg, about 105 mg, about 108 mg, about 110 mg, about 120 mg, about 126 mg, about 130 mg, about 140 mg, about 144 mg, about 145 mg, about 150 mg, about 155 mg, about 160 mg, about 165 mg, about 168 mg, about 170 mg, about 175 mg, about 180 mg, about 190 mg, about 200 mg, about 210 mg, about 216 mg, about 220 mg, about 230 mg, about 240 mg, about 245 mg, about 250 mg, about 260 mg, about 270 mg, about 280 mg, about 290 mg, about 300 mg, or a range between any two of these values (inclusive) or any value therein, or a formulation comprising the antibody-drug conjugate (e.g., ADC1 or ADC2) at said dose is administered per dose. In one or more embodiments, the antibody-drug conjugate is given in single administration. In one or more embodiments, the administration is performed once per day to once every 7 weeks. In one or more embodiments, the administration is performed once every 2 days to once every 6 weeks. In one or more embodiments, the administration is performed once every 2-4 weeks. In one or more embodiments, the administration is performed about twice a week or about once a week or about once every 2 weeks, 3 weeks, 4 weeks, 5 weeks, 6 weeks or 7 weeks. In one or more embodiments, the administration is performed once per day, once every 2 days, once every 3 days, once every 4 days, once every 5 days, twice a week, once a week, once every 2 weeks, once every 3 weeks, once every 4 weeks, once every 5 weeks, once every 6 weeks or once every 7 weeks.

In one or more embodiments, the antibody-drug conjugate described herein (e.g., ADC1 or ADC2) administered per treatment cycle is 10 mg to 300 mg; or 30 mg-300 mg; or 50 mg-280 mg; or 100 mg-250 mg. In one or more embodiments, the antibody-drug conjugate described herein (e.g., ADC1 or ADC2) administered per treatment cycle is about 10 mg, about 30 mg, about 50 mg, about 55 mg, about 60 mg, about 65 mg, about 70 mg, about 72 mg, about 75 mg, about 80 mg, about 84 mg, about 90 mg, about 95 mg, about 100 mg, about 105 mg, about 108 mg, about 110 mg, about 120 mg, about 126 mg, about 130 mg, about 140 mg, about 144 mg, about 145 mg, about 150 mg, about 155 mg, about 160 mg, about 165 mg, about 168 mg, about 170 mg, about 175 mg, about 180 mg, about 190 mg, about 200 mg, about 210 mg, about 216 mg, about 220 mg, about 230 mg, about 240 mg, about 245 mg, about 250 mg, about 260 mg, about 270 mg, about 280 mg, about 290 mg, about 300 mg, or a range between any two of these values (inclusive) or any value therein.

In one or more embodiments, the antibody-drug conjugate described herein (e.g., ADC1 or ADC2) is administered at a dose of 10 mg to 300 mg; or 30 mg-300 mg; or 50 mg-280 mg; or 100 mg-250 mg once every 3 weeks; or a formulation comprising the antibody-drug conjugate (e.g., ADC1 or ADC2) at said dose is administered once every 3 weeks. In one or more embodiments, the antibody-drug conjugate described herein (e.g., ADC1 or ADC2) is administered at a dose of about 10 mg, about 30 mg, about 50 mg, about 55 mg, about 60 mg, about 65 mg, about 70 mg, about 72 mg, about 75 mg, about 80 mg, about 84 mg, about 90 mg, about 95 mg, about 100 mg, about 105 mg, about 108 mg, about 110 mg, about 120 mg, about 126 mg, about 130 mg, about 140 mg, about 144 mg, about 145 mg, about 150 mg, about 155 mg, about 160 mg, about 165 mg, about 168 mg, about 170 mg, about 175 mg, about 180 mg, about 190 mg, about 200 mg, about 210 mg, about 216 mg, about 220 mg, about 230 mg, about 240 mg, about 245 mg, about 250 mg, about 260 mg, about 270 mg, about 280 mg, about 290 mg, about 300 mg, or a range between any two of these values (inclusive) or any value therein, or a formulation comprising the antibody-drug conjugate (e.g., ADC1 or ADC2) at said dose is administered once every 3 weeks. In one or more embodiments, the antibody-drug conjugate described herein (e.g., ADC1 or ADC2) is administered at about 72 mg once every 3 weeks. In one or more embodiments, the antibody-drug conjugate described herein (e.g., ADC1 or ADC2) is administered at about 90 mg once every 3 weeks. In one or more embodiments, the antibody-drug conjugate described herein (e.g., ADC1 or ADC2) is administered at about 108 mg once every 3 weeks. In one or more embodiments, the antibody-drug conjugate described herein (e.g., ADC1 or ADC2) is administered at about 126 mg once every 3 weeks. In one or more embodiments, the antibody-drug conjugate described herein (e.g., ADC1 or ADC2) is administered at about 144 mg once every 3 weeks. In one or more embodiments, the antibody-drug conjugate described herein (e.g., ADC1 or ADC2) is administered at about 180 mg once every 3 weeks. In one or more embodiments, the antibody-drug conjugate described herein (e.g., ADC1 or ADC2) is administered at about 216 mg once every 3 weeks.

In one or more embodiments, the method comprises at least 1, at least 2, at least 3, at least 4, at least 5 or at least 6 treatment cycles. In one or more embodiments, one treatment cycle is at least 1 day, at least 2 days, at least 3 days, at least 5 days, at least 1 week, at least 2 weeks, at least 3 weeks, at least 4 weeks, at least 5 weeks, at least 6 weeks or at least 7 weeks. In one or more embodiments, one treatment cycle is about 1 week, about 2 weeks, about 3 weeks, about 4 weeks, about 1 month, about 5 weeks, about 6 weeks, about 2 months, about 6 months or about 1 year, or a range between any two of these values (inclusive) or any value therein. In one or more embodiments, one treatment cycle is about 1 week, about 2 weeks, about 3 weeks, about 4 weeks, about 1 month, about 5 weeks, about 6 weeks, about 2 months or about 6 months. In one or more embodiments, one treatment cycle is 1 week, 2 weeks, 3 weeks, 4 weeks, 5 weeks, 6 weeks, 7 weeks, or a range between any two of these values (inclusive) or any value therein.

In one or more embodiments, the patient is administered a dose once per treatment cycle. In one or more embodiments, the patient is administered multiple doses per treatment cycle, for example, 2, 3, 4, or 5 times. In one or more embodiments, the patient receives treatment for one treatment cycle. In one or more embodiments, the patient receives treatment for multiple (e.g., 2, 3, 4, 5, 6, 7, 8, 9, 10, 11, or 12) treatment cycles. In one or more embodiments, the patient receives treatment until the condition is alleviated and no treatment is required.

In one or more embodiments, the antibody-drug conjugate or the pharmaceutical composition comprising the same is administered by injection. In one or more embodiments, the antibody-drug conjugate or the pharmaceutical composition comprising the same is administered by subcutaneous (s.c.) injection, intraperitoneal (i.p.) injection, parenteral injection, intra-arterial injection, intravenous (i.v.) injection, or the like. In one or more embodiments, the antibody-drug conjugate or the pharmaceutical composition comprising the same is administered by infusion. In one or more embodiments, the antibody-drug conjugate or the pharmaceutical composition comprising the same is administered by bolus injection. In one or more embodiments, the antibody-drug conjugate or the pharmaceutical composition comprising the same is administered by intravenous injection. In one or more embodiments, the antibody-drug conjugate or the pharmaceutical composition comprising the same is administered by subcutaneous injection. In one or more embodiments, the antibody-drug conjugate or the pharmaceutical composition comprising the same is administered by intraperitoneal injection. In one or more embodiments, the antibody-drug conjugate or the pharmaceutical composition comprising the same is administered by intravenous infusion.

In one or more embodiments, the present invention provides a method for treating cancer, which comprises administering to a patient in need an effective amount of an antibody-drug conjugate by injection; wherein the antibody-drug conjugate is administered at a dose of 1.8 mg/kg, 2.1 mg/kg, 2.4 mg/kg, 76-93 mg/m² or 84-93 mg/m² once every 3 weeks; the antibody-drug conjugate has a structure of Formula I-13-1, or a stereoisomer thereof, or a pharmaceutically acceptable salt or solvate thereof: 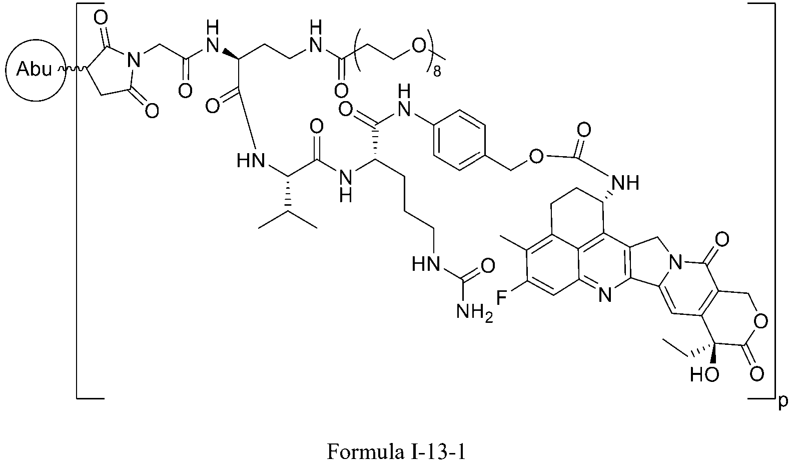 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 3.5-4.5, or 7-8.

In one or more embodiments, the present invention provides a method for treating cancer, which comprises administering to a patient in need an effective amount of an antibody-drug conjugate by injection; wherein the antibody-drug conjugate is administered at a dose of 1.8 mg/kg, 2.1 mg/kg, 2.4 mg/kg, 76-93 mg/m² or 84-93 mg/m² once every 3 weeks; the antibody-drug conjugate has a structure of Formula I-13-1, or a stereoisomer thereof, or a pharmaceutically acceptable salt or solvate thereof: 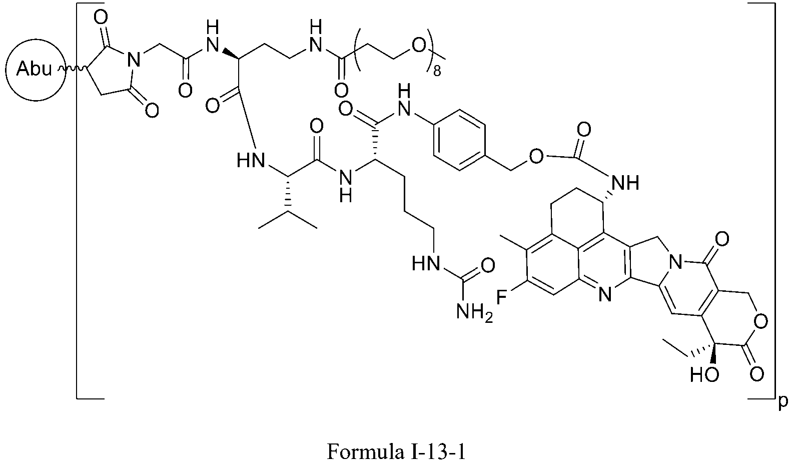 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 3.5-4.5.

In one or more embodiments, the present invention provides a method for treating cancer, which comprises administering to a patient in need an effective amount of an antibody-drug conjugate by injection; wherein the antibody-drug conjugate is administered at a dose of 1.8 mg/kg, 2.1 mg/kg, 2.4 mg/kg, 76-93 mg/m² or 84-93 mg/m² once every 3 weeks; the antibody-drug conjugate has a structure of Formula I-13-1, or a stereoisomer thereof, or a pharmaceutically acceptable salt or solvate thereof: 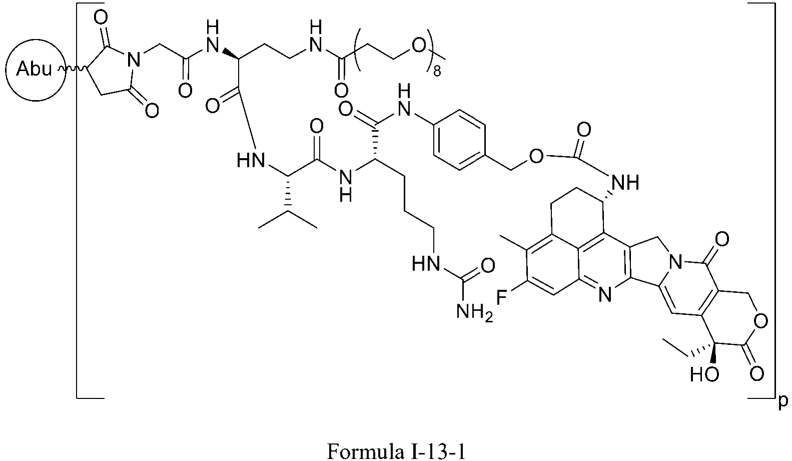 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 7-8.

In one or more embodiments, the present invention provides a method for treating cancer, which comprises administering to a human patient in need thereof a dose of about 1.8 mg/kg of the ADC1 by injection once every 3 weeks. In one or more embodiments, the present invention provides a method for treating cancer, which comprises administering to a human patient in need thereof a dose of about 2.1 mg/kg of the ADC1 by injection once every 3 weeks. In one or more embodiments, the present invention provides a method for treating cancer, which comprises administering to a human patient in need thereof a dose of about 2.4 mg/kg of the ADC1 by injection once every 3 weeks. In one or more embodiments, the present invention provides a method for treating cancer, which comprises administering to a human patient in need thereof a dose of 76-93 mg/m² of the ADC1 by injection once every 3 weeks. In one or more embodiments, the present invention provides a method for treating cancer, which comprises administering to a human patient in need thereof a dose of 84-93 mg/m² of the ADC1 by injection once every 3 weeks. In one or more embodiments, the present invention provides a method for treating cancer, which comprises administering to a human patient in need thereof a dose of about 76 mg/m² of the ADC1 by injection once every 3 weeks. In one or more embodiments, the present invention provides a method for treating cancer, which comprises administering to a human patient in need thereof a dose of about 84 mg/m² of the ADC1 by injection once every 3 weeks. In one or more embodiments, the present invention provides a method for treating cancer, which comprises administering to a human patient in need thereof a dose of about 93 mg/m² of the ADC1 by injection once every 3 weeks.

In one or more embodiments, the antibody-drug conjugate or the pharmaceutical composition comprising the same is administered by intravenous (i.v.) infusion (i.e., intravenous infusion). In one or more embodiments, the duration of the intravenous infusion is about 10 minutes, about 15 minutes, about 20 minutes, about 25 minutes, about 30 minutes, about 40 minutes, about 50 minutes, about 55 minutes, about 60 minutes, about 65 minutes, about 70 minutes, about 75 minutes, about 81 minutes, about 87 minutes, about 90 minutes, about 95 minutes, about 100 minutes, about 110 minutes, about 120 minutes, about 150 minutes, or a range between any two of these values (inclusive), or any value therein. In one or more embodiments, the duration of the intravenous infusion is ≥30 minutes. In one or more embodiments, the duration of the intravenous infusion is ≥ 60 minutes. In one or more embodiments, the duration of the intravenous infusion is ≥90 minutes.

In one or more embodiments, the uses and methods of the present invention employ a pharmaceutical composition comprising the antibody-drug conjugate suitable for injection, such as a pharmaceutical composition for bolus injection or a pharmaceutical composition for infusion (dripping). The pharmaceutical composition suitable for injection includes a sterile aqueous solution or dispersion and sterile powders for the instant preparation of a sterile injectable solution or dispersion.

For intravenous administration, a suitable carrier includes normal saline, bacteriostatic water or phosphate-buffered saline (PBS), ethanol, a solvent or dispersion medium for polyol (e.g., glycerol, propylene glycol and liquid polyethylene glycol), and a suitable mixture thereof. In one or more embodiments, the pharmaceutical composition further comprises a pharmaceutically acceptable carrier. In one or more embodiments, the pharmaceutically acceptable carrier may comprise an antibacterial and/or antifungal agent, e.g., p-hydroxylbenzoate, chlorobutanol, phenol, ascorbic acid and thimerosal. In one or more embodiments, the pharmaceutically acceptable carrier may comprise an isotonic agent, such as sugar, polyol (such as mannitol and sorbitol) and sodium chloride. In one or more embodiments, the pharmaceutical composition comprises at least 0.1% of the antibody-drug conjugate. The amount of the antibody-drug conjugate in such a pharmaceutical composition may be an effective amount for administration.

The methods and uses of the present invention provide substantial single-agent clinical efficacy while maintaining a favorable safety profile. In one or more embodiments, the methods and uses of the present invention may achieve an ORR greater than or equal to 40% and/or a DCR greater than or equal to 80%. In one or more embodiments, the methods and uses of the present invention demonstrate reduced toxicity, such as hematological toxicity or gastrointestinal toxicity

### BRIEF DESCRIPTION OF THE DRAWINGS

FIG. 1 shows the overall efficacy data of subjects across all dose cohorts; in the figure, columns 1, 2, 4, 5, 7, 8, 11, 17, 18, 21, 24, and 27-29 from left to right represent the 2.4 mg/kg cohort; columns 3, 9, 10, 14, 16, 19, 22, 23, and 26 represent the 2.1 mg/kg cohort; columns 6, 12, 13, 20, and 25 represent the 1.8 mg/kg cohort; and column 15 represents the 1.2 mg/kg cohort.
FIG. 2 shows the swimmer plot of subjects; in the figure, only row 18 from top to bottom received 1.2 mg/kg treatment.
FIG. 3 shows the efficacy data of subjects with ovarian cancer and fallopian tube cancer.
FIG. 4 shows the spider plot of subjects with ovarian cancer and fallopian tube cancer (N=17).
FIG. 5 shows the waterfall plot of the maximum target lesion reduction in PROC patients (N=54). In the figure, columns 2, 8, 11, 20, 23, 26, 32, 36, 37, 43, and 46-49 from left to right represent the 2.1 mg/kg cohort; columns 29 and 40 represent the 1.8 mg/kg cohort; and columns 4, 9, 14, 25, 27, 28, 39, 41, and 45 represent the 93 mg/m² cohort.
FIG. 6 shows the spider plot of PROC patients (N=54).
FIG. 7 shows the Kaplan-Meier curve for PFS.
FIG. 8 shows the Kaplan-Meier curve for OS.

### DETAILED DESCRIPTION

Unless otherwise defined, scientific and technical terms used in the present invention have the meanings commonly understood by a person skilled in the art.

### DEFINITION

It should be noted that the term "an" entity refers to one or more of the entities. For example, "an antibody" should be interpreted as one or more antibodies. As such, the terms "a" (or "an"), "one or more" and "at least one" can be used interchangeably herein.

As used herein, the term "contain" or "comprise" means that the antibodies, compositions, methods or the like comprise the recited elements, such as components or steps and do not exclude the others. "Consisting essentially of ..." means that the antibodies, compositions, methods or the like exclude other elements that have a fundamental impact on the characteristics of the combination, but do not exclude elements that do not substantially affect the characteristics of the antibodies, compositions, methods or the like. "Consisting of ..." means that elements not specifically listed are excluded.

"About" refers to a general error range for corresponding values as readily understood by those skilled in the relevant art. In some embodiments, "about" mentioned herein refers to the values described and ranges thereof of ± 10%, ± 5%, or ± 1%.

"Treatment" refers to both therapeutic treatment and prophylactic or preventative measures, the purpose of which is to prevent, slow down, ameliorate, or halt undesirable physiological changes or disorders, such as the progression of a disease, including, but not limited to, alleviation of symptoms, diminishment of the extent of disease, stabilized (i.e., not worsening) state of disease, delay or slowing of progressive disease, amelioration, palliation, alleviation, or elimination (whether partial or total) of state of disease, prolongation of life expectancy as compared with that in the absence of treatment, and the like, as either detectable or undetectable. Patients in need of treatment include patients already with a condition or disorder, patients susceptible to a condition or disorder, or patients in need of prevention of the condition or disorder, or patients who may or are expected to benefit from the administration of the antibody or the pharmaceutical composition disclosed herein for detection, diagnostic procedures, and/or treatment.

The term "cancer" is meant to refer to or describe the physiological condition in mammals that is typically characterized by unregulated cell growth. Examples of cancer include, but are not limited to, carcinoma, lymphoma, blastoma, sarcoma, or leukemia. More particular examples of such cancers include, but are not limited to, colorectal cancer, lung cancer, ovarian cancer, uterine cancer, endometrial cancer, salivary gland cancer, peritoneal cancer, fallopian tube cancer, pancreatic cancer, thyroid cancer, head and neck squamous cell carcinoma, nasopharyngeal cancer, laryngeal cancer, lung adenocarcinoma, lung squamous cell carcinoma, liver cancer, hepatocellular carcinoma, gastrointestinal cancer, glioblastoma, breast cancer, brain cancer, renal cancer, renal cell carcinoma, colon cancer, rectal cancer, prostate cancer, vulvar cancer, testicular cancer, squamous cell carcinoma, small cell lung cancer, uterine cervical cancer, cervical cancer, tongue cancer, bladder cancer, retinoblastoma, glioblastoma, mesothelioma, oral epithelioid cancer, choriocarcinoma and head and neck cancer.

The term "overexpression" or "overexpressed" interchangeably refers to a gene that is transcribed or translated at a detectably greater level, usually in certain cells, e.g., a cancer cell, in comparison to a normal cell. Overexpression may be overexpression of a protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., increased enzyme hydrolysis of substrate. Overexpression can be by 50%, 60%, 70%, 80%, 90% or more in comparison to a normal cell or comparison cell. In certain embodiments, the anti-FRα antibodies or antibody-drug conjugates of the present invention are used to treat solid tumors likely to overexpress folate receptor α.

In one embodiment, folate receptor α expression in a tumor sample above background levels of immune tissue (e.g., as determined by immunohistochemical staining) indicates that the tumor is a folate receptor α expressing tumor. Methods for detecting expression of folate receptor α in a tumor are known in the art, and include immunohistochemical assays.

The term "in need" means that the patient has been identified as in need of a particular method or treatment. In some embodiments, the identification may be made by any diagnostic mode. The patient may be in need of any methods and treatments described herein.

The term "patient" refers to any mammal in need of diagnosis, prognosis, or treatment, including, but not limited to, humans, dogs, cats, guinea pigs, rabbits, rats, mice, horses, cattle, etc. In some embodiments, the patient is a human.

The term "administration" or "dosing" as used herein refers to administering a substance for therapeutic purposes (e.g., treating a tumor). The administration mode may be parenteral, enteral, or topical. Parenteral administration is typically performed by injection, including, but not limited to, intravenous, intramuscular, intra-arterial, intrathecal, intracapsular, intraorbital, intracardiac, intradermal, intraperitoneal, transtracheal, subcutaneous, subcuticular, intra-articular, subcapsular, subarachnoid, intraspinal, and intrasternal injection and infusion.

As used herein, the term "effective amount" or "therapeutically effective amount" refers to the amount of a drug, e.g., an antibody or ADC, that is sufficient to reduce or ameliorate the severity and/or duration of a condition (e.g., cancer) or one or more symptoms thereof, prevent the progression of a condition, cause regression of a condition, prevent the recurrence, development, onset or progression of one or more symptoms associated with a condition, detect a condition, or enhance or improve the prophylactic or therapeutic effect of another therapy (e.g., a prophylactic or therapeutic agent). For example, the effective amount of an antibody may inhibit tumor growth (e.g., inhibit an increase in tumor volume), decrease tumor growth (e.g., decrease tumor volume), reduce the number of cancer cells, and/or relieve to some extent one or more of the symptoms associated with cancer. For example, the effective amount may improve progression-free survival (PFS), improve overall survival (OS), or decrease the likelihood of recurrence.

The term "antibody-drug conjugate" and "ADC" refer to a binding protein (e.g., an antibody or an antigen-binding unit thereof) that is linked to one or more chemical drugs, which may optionally be a therapeutic agent or a cytotoxic agent. In a preferred embodiment, the ADC comprises an antibody, a drug (e.g., a cytotoxic drug), and a linker capable of attaching or conjugating the drug to the antibody. Non-limiting examples of drugs that can be included in the ADC include a mitotic inhibitor, an antitumor antibiotic, an immunomodulator, a vector for gene therapy, an alkylating agent, an anti-angiogenic agent, an anti-metabolite, a boron-containing agent, a chemoprotective agent, a hormone, an anti-hormonal agent, a corticosteroid, a photoactive therapeutic agent, an oligonucleotide, a radionuclide agent, a topoisomerase inhibitor, a kinase inhibitor (e.g., a TEC-family kinase inhibitor and a serine/threonine kinase inhibitor), and a radiosensitizer.

The term "antibody-drug conjugate" and "ADC" are used interchangeably. The term "anti-folate receptor α antibody-drug conjugate", "anti- FRα antibody-drug conjugate", "anti- FRα ADC" and "anti-folate receptor α ADC" are used interchangeably and refer to an ADC comprising an antibody that specifically binds to folate receptor α, wherein the antibody is conjugated with one or more drugs. In one or more embodiments, the anti-FRα ADC comprises an antibody conjugated to Exatecan. In one or more embodiments, the anti-FRα antibody or ADC binds to folate receptor alpha (e.g., human folate receptor alpha).

The term "drug to antibody ratio" or "DAR" refers to the number of drugs (e.g., Exatecan) that are attached to one antibody in the ADC. The DAR of an ADC may be in the range of 1 to 10, but a higher load (e.g., 20) is also possible depending on the number of connection sites on the antibody. The term DAR may be used when referring to the number of drugs loaded onto a single antibody, or alternatively, when referring to an average or mean DAR for a set of ADCs. In some embodiments, the value is selected from 1, 2, 3, 4, 5, 6, 7, 8, 9, or 10. In considering the mean binding number of small molecule drugs, i.e., the mean drug binding number of an antibody, or the mean drug to antibody ratio, the value is selected from about 0 to about 10, or about 2 to about 8. In some embodiments, the drug to antibody ratio is about 3 to about 6. In other embodiments, the drug to antibody ratio is about 6 to about 8, or about 7 to about 8. DAR values may be denoted herein by p. A person skilled in the art understands that the average DAR values (p) may vary slightly between different batches of the same ADC. The DAR value of ADC can be measured by ultraviolet-visible absorption spectroscopy (UV-Vis), high performance liquid chromatography-hydrophobic interaction chromatography (HPLC-HIC), reversed-phase high performance liquid chromatography (RP-HPLC), liquid chromatography-mass spectrometry (LC-MS), etc. These techniques are described in Ouyang, J. Methods Mol Biol, 2013, 1045: p. 275-83.

Various substituents are defined below. In some cases, the number of carbon atoms in a substituent (e.g., alkyl, alkenyl, alkynyl, alkoxy, aminoalkoxy, aminoalkyl, aminoalkylamino, alkylamino, heterocyclyl, heterocyclylamino, and aryl) is indicated by the prefix "Cx-Cy" or "Cx-y", wherein x is the minimum number of carbon atoms and y is the maximum number of carbon atoms. Thus, for example, "C1-C6 alkyl" refers to an alkyl containing 1 to 6 carbon atoms. If a substituent is described as "substituted with ...", a hydrogen atom on a carbon or nitrogen is substituted with a non-hydrogen group. For example, a substituted alkyl substituent is an alkyl substituent in which at least one hydrogen atom on the alkyl is substituted with a non-hydrogen group. For illustration, monofluoroalkyl is an alkyl substituted with one fluoro group, and difluoroalkyl is an alkyl substituted with two fluoro groups. It should be recognized that if there is more than one substitution on a substituent, each substitution may be identical or different (unless otherwise stated). If a substituent is described as "optionally substituted with ...", the substituent may be (1) unsubstituted or (2) substituted. Possible substituents include, but are not limited to, hydroxy, C1-C6 alkyl, C2-C6 alkenyl, C2-C6 alkynyl, C1-C6 alkoxy, C1-C6 aminoalkoxy, halogen, nitro, cyano, thiol, alkylthio, amino, C1-C6 aminoalkyl, C1-C6 aminoalkylamino, C1-C6 alkyl connected to a heterocyclic ring, C1-C6 alkylamino connected to a heterocyclic ring, heterocyclyl, amino-substituted heterocyclyl, heterocyclylamino, carbamoyl, morpholin-1-yl, piperidin-1-yl, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-arylene-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, -(CH₂)_{q}C(O)NRⁿ(CH₂)_{q}-CH₃, -(CH₂CH₂O)_{q}-CH₃, -(CH₂CH₂O)_{q}-CH₂-CH₃, -(CH₂)_{q}C(O)NRⁿ(CH₂CH₂O)_{q}-CH₃, -(CH₂)_{q}C(O)NRⁿ(CH₂CH₂O)_{q}-CH₂-CH₃, -(CH₂CH₂O)_{q}C(O)NRⁿ(CH₂CH₂O)_{q}-CH₃, -(CH₂CH₂O)_{q}C(O)NRⁿ(CH₂CH₂O)_{q}-CH₂-CH₃, or - (CH₂CH₂O)_{q}C(O)NRⁿ(CH₂)_{q}-CH₃; wherein each Rⁿ is independently H, C1-C6 alkyl, C3-C8 carbocyclyl, phenyl, or benzyl, and each q is independently 1, 2, 3, 4, 5, 6, 7, 8, 9, or 10.

The "alkyl" refers to a saturated aliphatic hydrocarbyl group, and this term includes linear and branched hydrocarbyl groups. For example, C1-C20 alkyl, such as C1-C6 alkyl. C1-C20 alkyl refers to an alkyl group containing 1 to 20 carbon atoms, e.g., an alkyl group containing 1 carbon atom, 2 carbon atoms, 3 carbon atoms, 4 carbon atoms, 5 carbon atoms, 6 carbon atoms, 7 carbon atoms, 8 carbon atoms, 9 carbon atoms, 10 carbon atoms, 11 carbon atoms, 12 carbon atoms, 13 carbon atoms, 14 carbon atoms, 15 carbon atoms, 16 carbon atoms, 17 carbon atoms, 18 carbon atoms, 19 carbon atoms, or 20 carbon atoms. Non-limiting examples of alkyl include methyl, ethyl, n-propyl, isopropyl, n-butyl, isobutyl, sec-butyl, tert-butyl, n-pentyl, neopentyl, n-hexyl, etc. The alkyl may be unsubstituted or substituted with one or more substituents including, but not limited to, alkyl, alkoxy, cyano, hydroxy, carbonyl, carboxy, aryl, heteroaryl, amino, halogen, sulfonyl, sulfinyl, phosphono, etc.

The term "alkenyl", by itself or as part of another substituent, refers to an unsaturated branched, linear, or cyclic alkyl having at least one carbon-carbon double bond derived by the removal of one hydrogen atom from a single carbon atom of a parent olefin. Typical alkenyl includes, but is not limited to, ethenyl; propenyl (e.g., prop-1-en-1-yl, prop-1-en-2-yl, prop-2-en-1-yl, prop-2-en-2-yl, or cycloprop-1-en-1-yl); cycloprop-2-en-1-yl; butenyl (e.g., but-1-en-1-yl, but-1-en-2-yl, 2-methyl-prop-1-en-1-yl, but-2-en-1-yl, but-2-en-2-yl, but-1,3-dien-1-yl, but-1,3-dien-2-yl, cyclobut-1-en-1-yl, cyclobut-1-en-3-yl, cyclobut-1,3-dien-1-yl, etc.), and the like.

The term "alkynyl", by itself or as part of another substituent, refers to an unsaturated branched, linear, or cyclic alkyl having at least one carbon-carbon triple bond derived by the removal of one hydrogen atom from a single carbon atom of a parent alkyne. Typical alkynyl includes, but is not limited to, ethynyl; propynyl (e.g., prop-1-yn-1-yl, prop-2-yn-1-yl, etc.); butynyl (e.g., but-1-yn-1-yl, but-1-yn-3-yl, but-3-yn-1-yl, etc.), and the like.

The "carbocyclyl" refers to a stable non-aromatic monocyclic or polycyclic hydrocarbyl radical consisting of carbon and hydrogen atoms only, and may comprise a fused or bridged ring system, contains 3 to 15 carbon atoms, for example, 3 to 10 (e.g., 3, 4, 5, 6, 7, 8, 9 or 10) carbon atoms. It is saturated or unsaturated, and links to the remainder of the molecule through a single bond. Monocyclic radicals include, for example, cyclopropyl, cyclobutyl, cyclopentyl, cyclohexyl, cycloheptyl and cyclooctyl. Polycyclic radicals include, for example, adamantyl, norbornyl, and decahydronaphthyl. When specifically indicated, the carbocyclyl may be optionally substituted with one or more substituents independently selected from: alkyl, halogen, haloalkyl, cyano, nitro, oxo, aryl, aralkyl, carbocyclyl, carbocyclylalkyl, heterocyclyl, heterocyclylalkyl, heteroaryl and heteroarylalkyl.

The "aryl" refers to an all-carbon monocyclic or all-carbon fused ring having a completely conjugated π-electron system, and typically has 5-14 carbon atoms, e.g., 6, 10, 12, or 14 carbon atoms. Aryl may be unsubstituted or substituted with one or more substituents including, but not limited to, alkyl, alkoxy, cyano, hydroxy, carboxy, aryl, aralkyl, amino, halogen, sulfonyl, sulfinyl, phosphono, etc. Examples of unsubstituted aryl include, but are not limited to, phenyl, naphthyl, and anthracenyl.

The "heterocyclyl" refers to a stable 3 to 18 membered aromatic or nonaromatic ring group consisting of 2 to 8 (e.g., 2, 3, 4, 5, 6, 7 or 8) carbon atoms and 1 to 6 (1, 2, 3, 4, 5 or 6) heteroatoms selected from nitrogen, oxygen and sulfur. Unless otherwise specifically stated, heterocyclyl may be a monocyclic, bicyclic, tricyclic or tetracyclic ring system, and may include fused or bridged ring systems; and the nitrogen, carbon or sulfur atoms in heterocyclyl may be optionally oxidized; the nitrogen atoms are optionally quaternized; and heterocyclyl may be partially or fully saturated. Examples of such heterocyclyl include, but are not limited to, dioxolanyl, dioxinyl, thienyl[1,3]dithianyl, decahydroisoquinolinyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidinonyl, pyrrolidinyl, pyrazolidinyl, quinuclidinyl, thiazolidinyl, 1,2,4-thiadiazol-5(4H)-ylidene, tetrahydrofuranyl, trioxacyclohexyl, trithianyl, triazinanyl, tetrahydropyranyl, thiomorpholinyl, thiomorpholinyl, 1-oxo-thiomorpholinyl, and 1,1-dioxo-thiomorpholinyl. When specifically stated in the specification, heterocyclyl may be optionally substituted with one or more substituents selected from: alkyl, alkenyl, halogen, haloalkyl, cyano, oxo, thioxo, nitro, aryl, aralkyl, cycloalkyl, cycloalkylalkyl, optionally substituted heterocyclyl, optionally substituted heterocyclylalkyl, optionally substituted heteroaryl, and optionally substituted heteroarylalkyl.

The "alkoxy" refers to formula -O-(alkyl), wherein alkyl is the alkyl defined herein. Non-limiting examples of alkoxy are methoxy, ethoxy, n-propoxy, 1-methylethoxy (isopropoxy), n-butoxy, isobutoxy, sec-butoxy, and tert-butoxy. Alkoxy may be substituted or unsubstituted.

The "halogen" refers to fluoro (F), chloro (Cl), bromo (Br), or iodo (I).

The "amino" refers to-NH₂.

The "cyano" refers to -CN.

The "nitro" refers to -NO₂.

The "hydroxy" refers to -OH.

The "carboxyl" refers to -COOH.

The "thiol" refers to -SH.

The "carbonyl" refers to C=O.

"Stereoisomers" refer to isomeric compounds having the same linking order of atoms but different spatial arrangements of the atoms. Stereoisomers may have one or more stereocenters and each center may exist as R or S, and stereoisomers may also be cis-trans isomers. Stereoisomers of the compounds provided herein include any one of all diastereomeric, enantiomeric and cis-trans isomeric forms thereof, or suitable mixtures thereof.

The pharmaceutically acceptable salt includes those derived from the compound with a variety of organic and inorganic counterions well known in the art, and salts as examples only include organic or inorganic salts such as lithium salt, sodium salt, potassium salt, calcium salt, magnesium salt, ammonium salt, isopropylamine, trimethylamine, diethylamino, triethylamine, tripropylamine, ethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethyl, piperidine, polyamine resin, and tetraalkylammonium salt when the molecule contains an acidic functional group; and organic or inorganic acid salts such as hydrochloride salt, hydrobromide salt, tartrate salt, mesylate salt, acetate salt, maleate salt, and oxalate salt when the molecule contains a basic functional group. Other non-limiting examples of acids include sulfuric acid, nitric acid, phosphoric acid, propionic acid, glycolic acid, pyruvic acid, malonic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, and the like. These salts can generally be prepared by conventional methods by reacting, for example, an appropriate acid or base with the compound. The solvate includes hydrates.

All the publications, and patents cited herein are incorporated by reference in their entirety for all purposes.

### ANTIBODY-DRUG CONJUGATE

In one or more embodiments, the ADC has a structure of Formula I, or a stereoisomer thereof, or a pharmaceutically acceptable salt or solvate thereof. 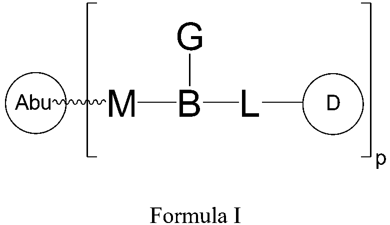 wherein Abu, M, B, G, L, D, and p are as defined herein.

In one or more embodiments, Abu is Antibody 1.

In one or more embodiments, Abu is ADC1 or ADC2 or a pharmaceutically acceptable salt or solvate thereof.

### PREPARATION METHOD

The intermediates and the antibody-drug conjugates of the present invention can be prepared using known formulations and methods.

In one or more embodiments, the preparation method for Intermediate Compound 1-7 is as described below. 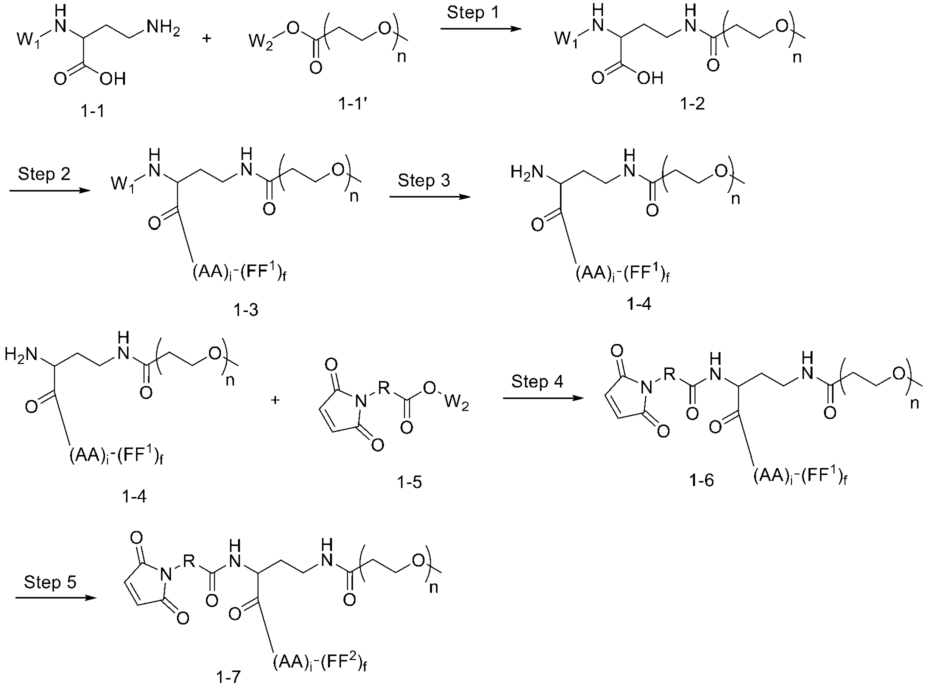
Step 1: reacting a compound of general Formula 1-1 with a compound of general Formula 1-1' under a basic condition to obtain a compound of general Formula 1-2;
Step 2: reacting the compound of general Formula 1-2 with general Formula (AA)ᵢ-(FF¹)_{f} in the presence of a condensing agent under a basic condition to obtain a compound of general Formula 1-3;
Step 3: removing the amino-protecting group W₁ of the compound of general Formula 1-3 to obtain a compound of general Formula 1-4;
Step 4: reacting the compound of general Formula 1-4 with a compound of general Formula 1-5 under a basic condition to obtain a compound of general Formula 1-6; and
Step 5: reacting the compound of general Formula 1-6 with bis(p-nitrophenyl)carbonate under a basic condition to obtain a compound of general Formula 1-7.

### Wherein

W₁ is an amino-protecting group, e.g., 9-fluorenylmethyloxycarbonyl; W₂ is a carboxylic acid active ester, for example, a succinimidyl ester.
wherein n, AA, R, i, f are described herein, each FF¹ is independently 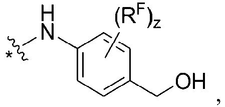 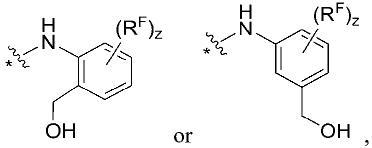 wherein each R^{F} is independently C1-C6 alkyl, C1-C6 alkoxy, -NO₂ or halogen; z is 0, 1, 2, 3 or 4; wherein * links to AA; each FF² is independently 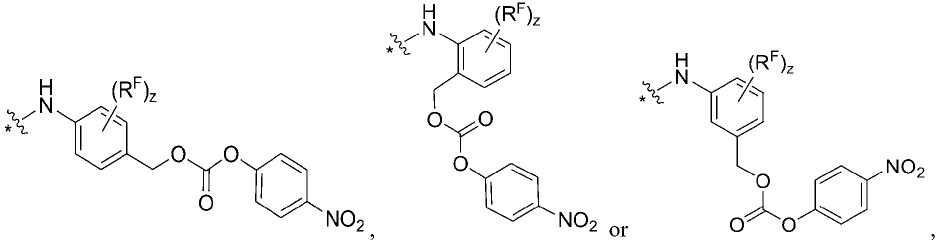
wherein each R^{F} is independently C1-C6 alkyl, C1-C6 alkoxy, -NO₂ or halogen;z is 0, 1, 2, 3 or 4;wherein * links to AA.

In one or more embodiments, R^{F} is F.

In one or more embodiments, z is 0.

In one or more embodiments, z is 1 or 2.

The basic conditions described above can be provided using reagents including organic bases and inorganic bases, wherein the organic bases include, but are not limited to, triethylamine, diethylamine, *N*-methylmorpholine, pyridine, piperidine, *N*,*N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N',N'-tetramethyl-O-(7-azabenzotriazol-1-yl)urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azobenzotriazol, O-benzotriazol-N,N,N',N'-tetramethyluronium hexafluorophosphate, 2-(7-azobenzotriazol)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

In one or more embodiments, the preparation method for Intermediate Compound 1-8 is as described below.

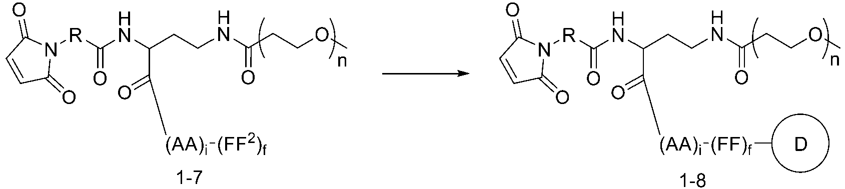

The compound of general Formula 1-7 and D are reacted in the presence of a condensing agent under a basic condition to obtain a compound of general Formula 1-8.

The basic conditions described above can be provided using reagents including organic bases and inorganic bases, wherein the organic bases include, but are not limited to, triethylamine, diethylamine, N-methylmorpholine, pyridine, piperidine, *N*,*N*-diisopropylethylamine, *n*-butyllithium, lithium diisopropylamide, potassium acetate, sodium *tert*-butoxide, or potassium *tert*-butoxide, and the inorganic bases include, but are not limited to, sodium hydride, potassium phosphate, sodium carbonate, potassium carbonate, cesium carbonate, sodium hydroxide, and lithium hydroxide.

The condensing agent described above may be selected from N,N,N',N'-tetramethyl-O-(7-azabenzotriazol-1-yl)urea hexafluorophosphate, 4-(4,6-dimethoxy-1,3,5-triazin-2-yl)-4-methylmorpholinium chloride, 1-hydroxybenzotriazole and 1-(3-dimethylaminopropyl)-3-ethylcarbodiimide hydrochloride, N,N'-dicyclohexylcarbodiimide, N,N'-diisopropylcarbodiimide, O-benzotriazol-N,N,N',N'-tetramethyluronium tetrafluoroborate, 1-hydroxybenzotriazole, 1-hydroxy-7-azobenzotriazol, O-benzotriazol-N,N,N',N'-tetramethyluronium hexafluorophosphate, 2-(7-azobenzotriazol)-N,N,N',N'-tetramethyluronium hexafluorophosphate, benzotriazol-1-yloxytris(dimethylamino)phosphonium hexafluorophosphate, and benzotriazol-1-yl-oxytripyrrolidinophosphonium hexafluorophosphate.

In one or more embodiments, the preparation method for Antibody-Drug Conjugate Compound 1-9 is as described below.

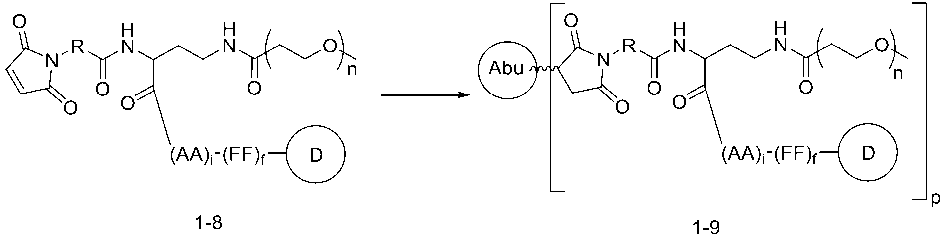

The compound of general Formula 1-8 is conjugated to Abu under a weakly acidic condition to obtain a compound of Formula 1-9.

Wherein n, AA, R, i, f, FF, D, Abu and p are described herein.

Specifically, the interchain disulfide bonds in the anti-FRα antibody provided by the present invention are reduced by a thiol-based reducing agent, generating free thiol groups, which are then conjugated with the compound of general Formula 1-8 to yield the antibody-drug conjugate represented by general Formula 1-9.

The weakly acidic condition described above may be provided using reagents including organic acids and inorganic acids, wherein the organic acids include, but are not limited to, acetic acid, benzoic acid, tartaric acid, oxalic acid, malic acid, citric acid, ascorbic acid, citric acid, salicylic acid, caffeic acid, sorbic acid, quinic acid, oleanolic acid, succinic acid, chlorogenic acid, formic acid, and propionic acid, and the inorganic acids include, but are not limited to, carbonic acid, nitrous acid, acetic acid, hypochlorous acid, hydrofluoric acid, sulfurous acid, hydrosulohuric acid, silicic acid, metasilicic acid, phosphoric acid, metaphosphoric acid, sodium bicarbonate, and sodium bisulfite.

The drug conjugates can be purified by conventional method, e.g., preparative high-performance liquid chromatography (prep-HPLC) or the like.

### TREATMENT METHODS AND USES

The antibody or the antigen-binding unit thereof or the ADC or the pharmaceutical composition comprising the same provided herein can be used for diagnosis, prognosis, monitoring, treatment, alleviation and/or prevention of diseases and disorders related to abnormal expression of FRα in a patient. When a disease and a disorder related to abnormal expression of FRα in a patient are identified by using a standard method, the antibody or the antigen-binding unit thereof or the ADC or the pharmaceutical composition comprising the same disclosed herein may be administered. In one or more embodiments, the extent of FRα expression is detected by methods such as immunohistochemistry (IHC), flow cytometry, or nucleic acid hybridization. WO 2014/036495 and WO 2015/031815 provide exemplary antibodies, detection methods, and kits for detecting FRα, all of which are incorporated herein by reference in their entirety.

In one or more embodiments, the present invention relates to: a method for treating related diseases with FRα as a therapeutic target, thereby ameliorating, slowing, inhibiting, treating, or preventing any disease or disorder associated with abnormal FRα expression (e.g., FRα overexpression); a method for treating tumors (including benign tumors and cancers) in a patient, a method for alleviating symptoms of tumors (including benign tumors and cancers) in a patient, and a method for avoiding recurrence of tumors (including benign tumors and cancers) in a patient, which comprise administering to the patient an effective amount of any of the antibodies or the antigen-binding units thereof or the ADCs described herein.

In one or more embodiments, provided is a method for preventing, treating or ameliorating diseases, which comprises administering to a patient in need an effective amount of the anti- FRα antibody or the antigen-binding unit thereof or the ADC or the pharmaceutical composition comprising the same. In one or more embodiments, provided is use of the antibody or the antigen-binding unit thereof or the ADC in the manufacture of a drug for preventing, treating or ameliorating diseases. In one or more embodiments, provided is use of the antibody or the antigen-binding unit thereof or the ADC in preventing, treating or ameliorating diseases. In one or more embodiments, the disease is a disease associated with the abnormal expression of FRα. In one or more embodiments, the disease is a disease associated with the overexpression of FRα. In one or more embodiments, the disease is a tumor. In one or more embodiments, the disease is a tumor expressing FRα. In one or more embodiments, the disease is a tumor overexpressing FRα. In one or more embodiments, the disease is cancer. In one or more embodiments, the disease is cancer overexpressing FRα. In one or more embodiments, the disease is cancer overexpressing human FRα.

In one or more embodiments, provided is a method for treating tumors (including benign tumors and cancers), which comprises administering to a patient in need an effective amount of the anti- FRα antibody or the antigen-binding unit thereof or the ADC or the pharmaceutical composition comprising the same. In one or more embodiments, provided is use of the antibody or the antigen-binding unit thereof or the ADC in the manufacture of a drug for treating tumors (including benign tumors and cancers). In one or more embodiments, provided is use of the antibody or the antigen-binding unit thereof or the ADC in treating tumors (including benign tumors and cancers).

Examples of cancers include, but are not limited to, solid tumors, hematological cancers and metastatic lesions. Specific examples of such cancers include, but are not limited to, colorectal cancer, lung cancer, ovarian cancer, uterine cancer, endometrial cancer, peritoneal cancer, fallopian tube cancer, pancreatic cancer, head and neck squamous cell carcinoma, nasopharyngeal cancer, laryngeal cancer, lung adenocarcinoma, liver cancer, breast cancer, brain cancer, renal cancer, renal cell carcinoma, colon cancer, testicular cancer, uterine cervical cancer, cervical cancer, tongue cancer, bladder cancer, retinoblastoma, glioblastoma, mesothelioma, oral epithelioid cancer, choriocarcinoma and head and neck cancer. More particular examples of such cancers include, but are not limited to, ovarian cancer, epithelial ovarian cancer, ovarian primary peritoneal cancer or fallopian tube cancer. In one or more embodiments, the cancer is metastatic or advanced cancer. In one or more embodiments, the patient is a patient with increased expression levels of FRα.

### COMBINATION THERAPY

In one or more embodiments, the antibody or the antigen-binding unit thereof or the ADC of the present invention may be used in combination with other therapeutic or prophylactic regimens, including use of one or more antibodies or antigen-binding units thereof or ADCs of the present invention together with or in combination with one or more other therapeutic agents or methods. For combination therapy, the antibody or the antigen-binding unit thereof or the ADC may be administered simultaneously or separately with other therapeutic agents. When administered separately, the antibody or the antigen-binding unit thereof or the ADC of the present invention may be administered before or after the administration of another additional therapeutic agent.

In one or more embodiments, when the antibody or the antigen-binding unit thereof or the ADC of the present invention is administered to a patient, the antibody or the antigen-binding unit thereof or the ADC or the pharmaceutical composition or the immune conjugate disclosed herein and one or more other therapies, such as therapeutic modalities and/or other formulations (e.g., a therapeutic agent), may also be administered in combination to the patient.

In one or more embodiments, the antibody or the antigen-binding unit thereof or the ADC of the present invention can be used with an immune checkpoint inhibitor. In one or more embodiments, the antibody or the antigen-binding unit thereof or the ADC of the present invention is administered in combination with other therapeutic or prophylactic regimens, such as radiotherapy.

Such combination therapies encompass both simultaneous administration (wherein two or more formulations are contained in the same formulation or separate formulations) and separate administration (wherein the antibody or the antigen-binding unit thereof or the ADC of the present invention can be administered prior to, concurrently with, and/or subsequent to the administration of other therapies, e.g., therapeutic modalities or therapeutic agents). The antibody or the antigen-binding unit thereof or the ADC and/or other therapies, e.g., therapeutic agents or therapeutic modalities, can be administered when a disease is active or when the disease is in remission or less active. The antibody or the antigen-binding unit thereof or the ADC can be administered prior to, concurrently with or subsequent to other therapies, or during remission of a disease.

The citation of any publications and patent documents in the present invention does not constitute an acknowledgement that they constitute relevant prior art, nor an acknowledgement as to their contents or dates. Having now been described in the written description, the present invention may be practiced in various embodiments by a person skilled in the art, and the foregoing description and the following examples are intended to illustrate, but not to limit, the claims of the present invention. All publications, patents, and patent applications cited herein are incorporated by reference in their entirety for all purposes.

### Examples

The materials, reagents, etc., used in the following examples can be commercially available or obtained using known methods unless otherwise stated.

### Example 1. Preparation of antibodies

The amino acid sequence and nucleic acid sequence of Antibody 1 are shown in Table 1. The nucleic acid sequences encoding the heavy chain and light chain of the Antibody 1 were cloned into expression vectors, respectively, followed by transient transfection into HEK293 cells. The antibodies were then obtained through culture and purification. Sequencing results confirmed consistency with the expected sequences.

**Table 1. Amino acid sequence and nucleic acid sequence of Antibody 1**

| Type | Amino acid sequence | SEQ ID NO: |
|---|---|---|
| VH CDR1 | GYFMN | 1 |
| VH CDR2 | RIHPYDGDTFYNQNFKD | 2 |
| VH CDR3 | YDGSRAMDY | 3 |
| VL CDR1 | KASQSVSFAGTSLMH | 4 |
| VL CDR2 | RASNLEA | 5 |
| VL CDR3 | QQSREYPYT | 6 |
| VH | QVQLVQSGVEVKKPGASVKVSCKASGYSFTGYFMNWVRQAPG QGLEWIGRIHPYDGDTFYNQNFKDKATLTVDKSTTTAYMELKSL QFDDTAVYYCTRYDGSRAMDYWGQGTTVTVSS | 7 |
| VL | EIVLTQSPATLSLSPGERATLSCKASQSVSFAGTSLMHWYQQKPG QAPRLLIYRASNLEAGVPARFSGSGSKTDFTLTISSLEPEDFAVYY CQQSREYPYTFGGGTKVEIK | 8 |
| Heavy chain constant region | | 9 |
| Light chain constant region | | 10 |
| Heavy chain | QVQLVQSGVEVKKPGASVKVSCKASGYSFTGYFMNWVRQAPG QGLEWIGRIHPYDGDTFYNQNFKDKATLTVDKSTTTAYMELKSL QFDDTAVYYCTRYDGSRAMDYWGQGTTVTVSSASTKGPSVFPL APSSKSTSGGTAALGCLVKDYFPEPVTVSWNSGALTSGVHTFPA VLQSSGLYSLSSVVTVPSSSLGTQTYICNVNHKPSNTKVDKKVEP KSCDKTHTCPPCPAPELLGGPSVFLFPPKPKDTLMISRTPEVTCV VVDVSHEDPEVKFNWYVDGVEVHNAKTKPREEQYNSTYRVVS VLTVLHQDWLNGKEYKCKVSNKALPAPIEKTISKAKGQPREPQV YTLPPSRDELTKNQVSLTCLVKGFYPSDIAVEWESNGQPENNYKT TPPVLDSDGSFFLYSKLTVDKSRWQQGNVFSCSVMHEALHNHY TQKSLSLSPGK | 11 |
| Heavy chain nucleic acid sequence | caggtgcagctggtgcagtccggcgtggaggtgaagaagcccggcgccagcgtgaaggtgagctg caaggcctccggctactccttcaccggctacttcatgaactgggtgaggcaggcccccggccaggg actggaatggatcggcagaatccacccctacgacggcgacaccttttacaatcagaacttcaaggata aggccaccctgaccgtggataagtccaccaccaccgcctacatggagctgaagtccctgcagtttga cgataccgccgtgtactactgtaccagatacgacggctccagagccatggactactggggccaggg caccaccgtgaccgtgtcctccgccagcaccaagggcccatcggtcttccccctggcaccctcctcc aagagcacctctgggggcacagcggccctgggctgcctggtcaaggactacttccccgaaccggtg acggtgtcgtggaactcaggcgccctgaccagcggcgtgcacaccttcccggctgtcctacagtcct caggactctactccctcagcagcgtggtgaccgtgccctccagcagcttgggcacccagacctacat ctgcaacgtgaatcacaagcccagcaacaccaaggtggacaagaaagttgagcccaaatcttgtgac aaaactcacacatgcccaccgtgcccagcacctgaactcctggggggaccgtcagtcttcctcttccc cccaaaacccaaggacaccctcatgatctcccggacccctgaggtcacatgcgtggtggtggacgtg agccacgaagaccctgaggtcaagttcaactggtacgtggacggcgtggaggtgcataatgccaag acaaagccgcgggaggagcagtacaacagcacgtaccgtgtggtcagcgtcctcaccgtcctgcac caggactggctgaatggcaaggagtacaagtgcaaggtctccaacaaagccctcccagcccccatc gagaaaaccatctccaaagccaaagggcagccccgagaaccacaggtgtacaccctgcccccatc ccgggatgagctgaccaagaaccaggtcagcctgacctgcctggtcaaaggcttctatcccagcgac atcgccgtggagtgggagagcaatgggcagccggagaacaactacaagaccacgcctcccgtgct ggactccgacggctccttcttcctctacagcaagctcaccgtggacaagagcaggtggcagcaggg gaacgtcttctcatgctccgtgatgcatgaggctctgcacaaccactacacgcagaagagcctctccct gtctccgggtaaatga | 12 |
| Light chain | EIVLTQSPATLSLSPGERATLSCKASQSVSFAGTSLMHWYQQKPG QAPRLLIYRASNLEAGVPARFSGSGSKTDFTLTISSLEPEDFAVYY CQQSREYPYTFGGGTKVEIKRTVAAPSVFIFPPSDEQLKSGTASV VCLLNNFYPREAKVQWKVDNALQSGNSQESVTEQDSKDSTYSL SSTLTLSKADYEKHKVYACEVTHQGLSSPVTKSFNRGEC | 13 |
| Light chain nucleic acid sequence | gagatcgtgctgacccagtcccccgccaccctgagcctgagcccaggagagagggccaccctgtc ctgcaaggcctcccagagcgtgagctttgccggcaccagcctgatgcactggtatcaacagaagccc ggccaggcccctaggctgctgatctacagagccagcaacctggaggccggcgtgcctgctaggtttt ccggcagcggctccaagaccgatttcaccctgaccatcagcagcctggagcctgaggattttgccgt gtactactgccagcagtccagggagtacccctacaccttcggcggcggcaccaaggtggagatcaa gcgtacggtggctgcaccatctgtcttcatcttcccgccatctgatgagcagttgaaatctggaactgcc tctgttgtgtgcctgctgaataacttctatcccagagaggccaaagtacagtggaaggtggataacgcc ctccaatcgggtaactcccaggagagtgtcacagagcaggacagcaaggacagcacctacagcctc agcagcaccctgacgctgagcaaagcagactacgagaaacacaaagtctacgcctgcgaagtcacc catcagggcctgagctcgcccgtcacaaagagcttcaacaggggagagtgttga | 14 |
| VH FR1 | QVQLVQSGVEVKKPGASVKVSCKASGYSFT | 15 |
| VH FR2 | WVRQAPGQGLEWIG | 16 |
| VH FR3 | KATLTVDKSTTTAYMELKSLQFDDTAVYYCTR | 17 |
| VH FR4 | WGQGTTVTVSS | 18 |
| VL FR1 | EIVLTQSPATLSLSPGERATLSC | 19 |
| VL FR2 | WYQQKPGQAPRLLIY | 20 |
| VL FR3 | GVPARFSGSGSKTDFTLTISSLEPEDFAVYYC | 21 |
| VL FR4 | FGGGTKVEIK | 22 |

### Example 2. Synthesis Procedures for Compound (1S,95)-1-amino-9-ethyl-4,5-difluoro-9-hydroxy-1,2,3,9,12,15-hexahydro-10H,13H-benzo[de]pyrano[3',4':6,7]indolizino[1,2-b]quinolin-10,13-dione hydrochloride (D-1)

### 1. Synthesis of N,N-(3,4-difluoro-8-oxo-5,6,7,8-tetrahydronaphthalene-1,7-diyl)diacetamide

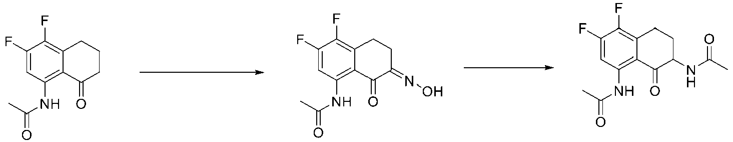

Under a nitrogen atmosphere, a solution of potassium *tert*-butoxide in tetrahydrofuran (42 mL, 1 M) was added to a dry reaction flask, and the mixture was stirred and cooled to 0-5 °C. *N*-(3,4-difluoro-8-oxo-5,6,7,8-tetrahydronaphthalen-1-yl)acetamide (CAS No.: 143655-49-6, 5 g, 21 mmol) dissolved in tetrahydrofuran (25 mL) was slowly added dropwise to the reaction flask, followed by dropwise addition of *tert*-butyl nitrite (4.32 g, 2 eq) (with temperature controlled at 0-5 °C). The resulting mixture was warmed to 15-20 °C and stirred for 2 hours (h). After the completion of the reaction, the mixture was cooled to 0-5 °C. Acetic acid (25 mL) and acetic anhydride (25 mL) were added dropwise (with temperature controlled at no more than 10 °C). After the dropwise addition, the mixture was stirred for 20 minutes (min). Zinc powder (8 eq) was added according to the amount of the *N*-(3,4-difluoro-8-oxo-5,6,7,8-tetrahydronaphthalen-1-yl)acetamide with the temperature maintained at 5-10 °C, and the resulting mixture was stirred at 20-25 °C for 1 h. The mixture was filtered, and the solid was rinsed with ethyl acetate (50 mL). The temperature was reduced to 0-5 °C, and 15% aqueous solution of Na₂CO₃ (50 mL) was added dropwise for three washes. Ethyl acetate (25 mL) was added for extraction, and the organic phases were combined and washed with a saturated aqueous solution of NaCl. Ethyl acetate (10 mL) was added, and the mixture was stirred at 40 °C for 30 min, slowly cooled to 0-5 °C and stirred for 2 h. The mixture was filtered, and the solid was washed with ethyl acetate/petroleum ether (1/2, 10 mL). Drying was performed *in vacuo* to obtain a gray powder (2.1 g, 33.7%). LC-MS: [M+H]⁺ = 297.

### 2. Synthesis of N-(8-amino-5,6-difluoro-1-oxo-1,2,3,4-tetrahydronaphthalen-2-yl)acetamide

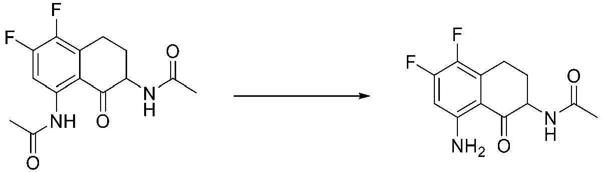

N,N-(3,4-difluoro-8-oxo-5,6,7,8-tetrahydronaphthalene-1,7-diyl)diethylamide (500 mg, 1.68 mmol) was added to a 2 M solution of hydrochloric acid in ethanol (5 mL). The mixture was stirred at 50 °C for 4 h. After the completion of the reaction as detected, water (7.5 mL) was added. The mixture was cooled to 0-5 °C, and triethylamine (1.03 g) was added dropwise. The mixture was stirred for 3 h. The mixture was filtered, and washing was performed successively with 40% cold aqueous solution of ethanol (3 mL) and water (3 mL). Drying was performed *in vacuo* to obtain a gray powder (320 mg, 74.6%). LC-MS: [M+H]⁺ = 255.

### 3. Synthesis of N-((9S)-9-ethyl-4,5-difluoro-9-hydroxy-10,13-dioxo-2,3,9,10,13,15-hexahydro-1H,12H-benzo[de]pyrano[3',4':6,7]indolizino[1,2-b]quinolin-1-yl)acetamide

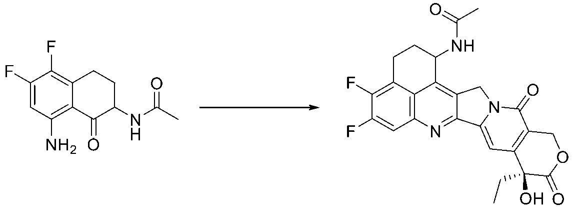

Under a nitrogen atmosphere, *N*-(8-amino-5,6-difluoro-1-oxo-1,2,3,4-tetrahydronaphthalen-2-yl)acetamide (1.1 g, 1 eq), (*S*)-4-ethyl-4-hydroxy-7,8-dihydro-1*H*-pyrano[3,4-*f*]indolizine-3,6,10(4*H*)-trione (1.15 g, 1 eq) and toluene (50 mL) were added to a reaction flask. The mixture was heated to reflux and stirred for 1 h. Then pyridinium 4-toluenesulfonate (100 mg) was added, and the mixture was stirred at reflux for another 20 h. The mixture was cooled to room temperature and stirred for 1 h. The mixture was filtered, and the solid was successively washed with acetone (10 mL) and cold ethanol (5 mL). Drying was performed *in vacuo* to obtain a taupe powder (1.1 g, 53%). LC-MS: [M+H]⁺ = 482.

### 4. Synthesis of (1S,9S)-1-amino-9-ethyl-4,5-difluoro-9-hydroxy-1,2,3,9,12,15-hexahydro-10H,13H-benzo[de]pyrano[3',4':6,7]indolizino[1,2-b]quinolin-10,13-dione hydrochloride (D-1)

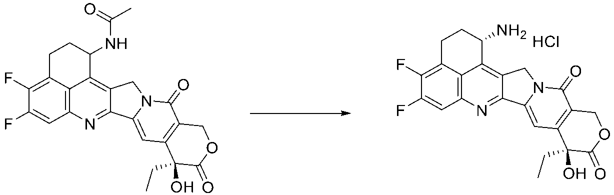

*N*-((9*S*)-9-ethyl-4,5-difluoro-9-hydroxy-10,13-dioxo-2,3,9,10,13,15-hexahydro-1*H*,12*H-*benzo[de]pyrano[3',4':6,7]indolizino[1,2-*b*]quinolin-1-yl)acetamide (1.1 g, 2.28 mmol) and a 6 M aqueous solution of hydrochloric acid (44 mL) were added to a reaction flask. The mixture was stirred at reflux under a nitrogen atmosphere for 4 h. The mixture was concentrated to remove the solvent, and the residue was purified by HPLC to obtain a white powder (200 mg, 18%). LC-MS: [M+H]⁺ = 440.

### Example 3. Synthesis Procedures for 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07)

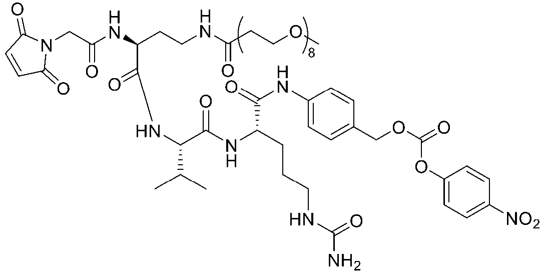

### 1) Synthesis of (S)-30-((((9H-fluoren-9-yl)methoxy)carbonyl)amino)-26-oxo-2,5,8,11,14,17,20,23-octaoxa-27-aza-hentriacontan-31-oic acid (CB01).

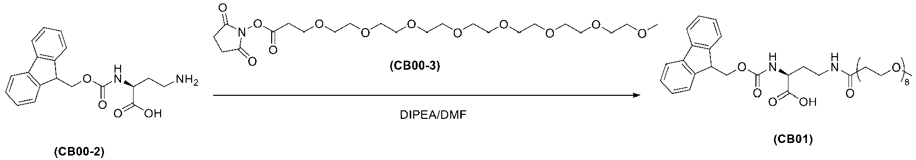

Under a nitrogen atmosphere at 0-5 °C, 8.14 g of *N*2-fluorenylmethoxycarbonyl-L-2,4-diaminobutyric acid (CB00-2) was dissolved in 40 mL of dimethylformamide (DMF), and 10 g of *N*-succinimidyl 4,7,10,13,16,19,22,25-octaoxahexacosanoate (CB00-3) and 10 mL of DMF were added. 6.5 mL of DIPEA was added dropwise with the temperature maintained at 0-5 °C. 1 h after the addition, the mixture was stirred at room temperature for 4 h. After the completion of the reaction, DMF was removed under reduced pressure, and the residue was purified by silica gel column chromatography (with dichloromethane and methanol (volume ratio of 20:1) as elution solvents) to obtain CB01 in the form of a pale yellow oil (14.2 g).

### 2) Synthesis of (9H-fluoren-9-yl)methyl [(S)-1-[[(S)-1-[[4-(hydroxymethyl)phenyl]amino]-1-oxo-5-ureidopentan-2-yl]amino]-3-methyl-1-oxobutan-2-yl]carbamate (CB02).

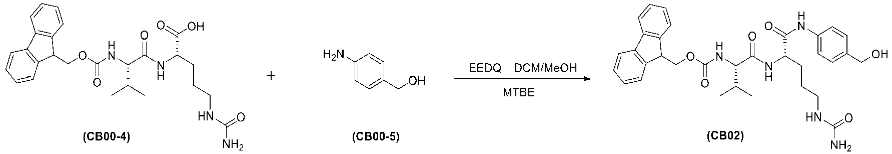

Under a nitrogen atmosphere at room temperature, 11 g of (*S*)-2-((*S*)-2-((((9*H*-fluoren-9-yl)methoxy)carbonyl)amino)-3-methylbutanamido)-5-ureidopentanoic acid (CB00-4) and 5.5 g of (4-aminophenyl)methanol (CB00-5) were dissolved in 400 mL of dichloromethane and 200 mL of methanol, and 17 g of 2-ethoxy-1-ethoxycarbonyl-1,2-dihydroquinoline (EEDQ) were added in portions with mechanical stirring. The resulting mixture was reacted for 15 h in the absence of light. After the completion of the reaction, the solvent was removed under reduced pressure to obtain a paste solid, which was subjected to silica gel column chromatography (with dichloromethane and methanol (volume ratio of 20:1) as elution solvents) to obtain CB02 in the form of an off-white solid (11.9 g).

### 3) Synthesis of (S)-2-((S)-2-amino-3-methylbutanamido)-N-(4-(hydroxymethyl)phenyl)-5-ureidopentanamide (CB03).

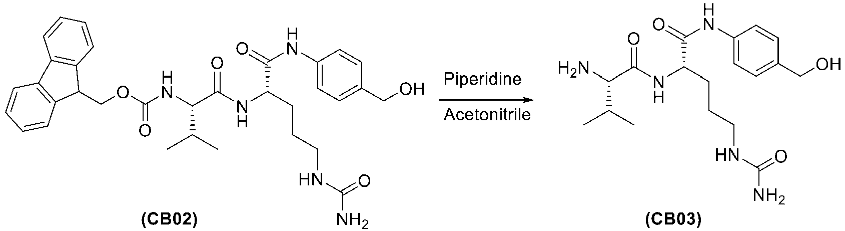

Under a nitrogen atmosphere at room temperature, 300 mL of acetonitrile was added to 11.9 g of (9*H-*fluoren-9-yl)methyl [(*S*)-1-[[(*S*)-1-[[4-(hydroxymethyl)phenyl]

amino]-1-oxo-5-ureidopentan-2-yl]amino]-3-methyl-1-oxobutan-2-yl]carbamate (CB02), and 18 mL of piperidine was added dropwise with stirring. After the dropwise addition, the mixture was reacted at room temperature for 2 h. After the completion of the reaction, the mixture was distilled under reduced pressure to remove the solvent and piperidine, and the residue was subjected to silica gel column chromatography (with dichloromethane and methanol (volume ratio of 20:1) as elution solvents) to obtain CB03 in the form of a white solid (7.5 g).

### 4) Synthesis of (9H-fluoren-9-yl)methyl ((30S,33S,36S)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04).

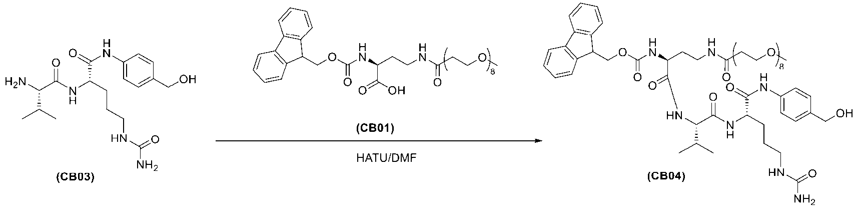

Under a nitrogen atmosphere at 0 °C, 14.2 g of (*S*)-30-((((9*H*-fluoren-9-yl)methoxy)carbonyl)amino)-26-oxo-2,5,8,11,14,17,20,23-octaoxa-27-aza-hentriacontan-31-oic acid (CB01) was dissolved in 100 mL of DMF, and 11 g of *N,N,N',N'-*tetramethyl-*O*-(7-azabenzotriazol-1-yl)uronium hexafluorophosphate (HATU) was added in portions. After 30 min of stirring, 7.5 g of (*S*)-2-((*S*)-2-amino-3-methylbutanamido)-*N*-(4-(hydroxymethyl)phenyl)-5-ureidopentanamide (CB03) was added, and the mixture was reacted for 2.5 h with the temperature maintained at 0 °C. After the completion of the reaction, the mixture was distilled under reduced pressure to remove the solvent, and the residue was subjected to silica gel column chromatography (with dichloromethane and methanol (volume ratio of 10:1) as elution solvents) to obtain CB04 in the form of a solid (9.66 g).

### 5) Synthesis of N-((S)-3-amino-4-(((S)-1-(((S)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05).

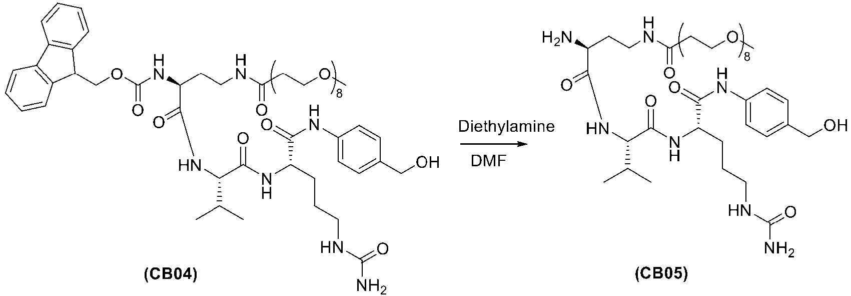

Under a nitrogen atmosphere at room temperature, 9.66 g of (9*H*-fluoren-9-yl)methyl ((30S,33S,36S)-41-amino-36-((4-(hydroxymethyl)phenyl)carbamoyl)-33-isopropyl-26,31,34,41-tetraoxo-2,5,8,11,14,17,20,23-octaoxa-27,32,35,40-tetraaza-30-yl)carbamate (CB04) was dissolved in 50 mL of DMF, and 12 mL of diethylamine was added. The mixture was stirred for 1.5 h. After the completion of the reaction, the mixture was distilled under reduced pressure to remove the solvent, and the residue was subjected to silica gel column chromatography (with dichloromethane and methanol (volume ratio of 7.5:1) as elution solvents) to obtain CB05 in the form of a pale yellow solid (7.7 g).

### 6) Synthesis of N-((S)-3-(2-(2,5-dioxo-2,5-dihydro-1H-pyrrol-1-yl)acetamido)-4-(((S)-1-(((S)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06).

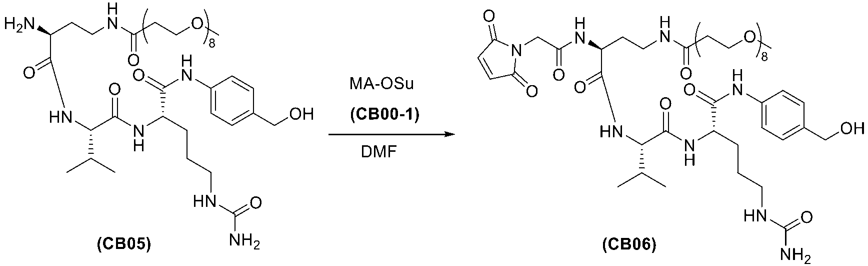

7.7 g of *N*-((*S*)-3-amino-4-(((*S*)-1-(((*S*)-1-((4-(hydroxymethyl)phenyl)amino)-1-oxo-5-ureidopentan-2-yl)amino)-3-methyl-1-oxobutan-2-yl)amino)-4-oxy)-2,5,8,11,14,17,20,23-octaoxahexacosan-26-amide (CB05) was dissolved in 40 mL of DMF, and succinimidyl maleimidoacetate (CB00-1) was added in portions under a nitrogen atmosphere at 0-5 °C. The mixture was reacted for 4 h with the temperature maintained at 0-5 °C. After the completion of the reaction, the mixture was distilled under reduced pressure to remove the solvent, and the residue was subjected to silica gel column chromatography (with dichloromethane and methanol (volume ratio of 10:1) as elution solvents) to obtain CB06 in the form of a pale yellow solid (9.5 g).

### 7) Synthesis of 4-((30S,33S,36S)-30-(2-(2,5-dioxo-2,5-dihydro-1H-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07).

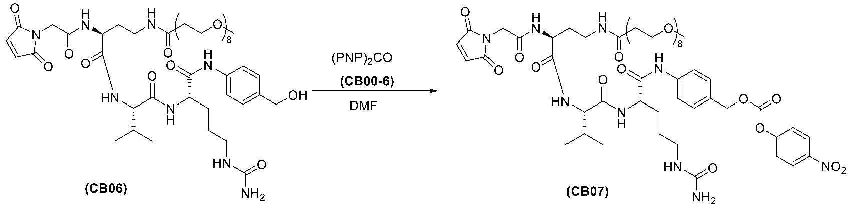

9.5 g of *N*-((*S*)-3-(2-(2,5-dioxo-2,5-dihydro-1*H*-pyrrol-1-yl)acetamido)-4-(((*S*)-1-(((*S*)-1-((4-(hydroxymethyl)phenyl)amino)-1-oxo-5-ureidopentan-2-yl)amino)-3-methyl-1-butanone-2-yl)amino)-4-oxo)-2,5,8,11,14,17,20,23-octaoxahexacosan-26-amide (CB06) was dissolved in 50 mL of DMF, and 14.0 g of bis(*p*-nitrophenyl)carbonate ((PNP)₂CO) was added under a nitrogen atmosphere at 0 °C, followed by addition of 8.2 mL of *N,N-*diisopropylethylamine (DIPEA) after dissolution. The mixture was reacted at 0 °C for 4 h. After the completion of the reaction, the mixture was distilled under reduced pressure to remove the solvent, and the residue was subjected to silica gel column chromatography (with dichloromethane and methanol (volume ratio of 8:1) as elution solvents) to obtain CB07 in the form of a white solid (2.6 g).

### Example 4. Synthesis of 4-((18S,21S,24S)-18-(2-(2,5-dioxo-2,5-dihydro-1H-pyrrol-1-yl)acetamido)-21-isopropyl-14,19,22-trioxo-24-(3-ureidopropyl)-2,5,8,11-tetraoxa-15,20,23-triazapentacosan-25-amido)benzyl (4-nitrophenyl) carbonate (CB14)

### 1. Synthesis of N,N'-(3,4-difluoro-8-oxo-5,6,7,8-tetrahydronaphthalene-1,7-diyl)diacetamide

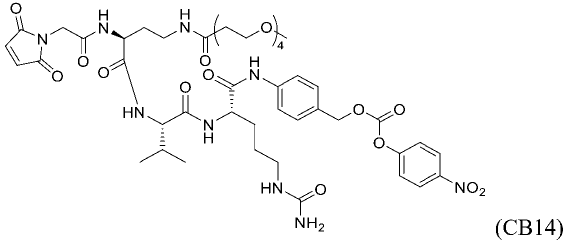

CB14 was prepared by referring to the synthesis of CB07 in Example 3, with N-succinimidyl 4,7,10,13,16,19,22,25-octaoxahexacosanoate replaced by N-succinimidyl 4,7,10,13-tetraoxatetradecanoate. CB14 was eventually obtained in the form of a white solid.

### Example 5. Synthesis of Intermediate (CB07-Exatecan)

Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-5-fluoro-9-hydroxy-4-methyl-10,13-dioxo-2,3,9,10,13,15-hexahydro-1*H*,12*H-*benzo[*de*]pyrano[3',4':6,7]indolizino[1,2-*b*]quinolin-1-yl) carbamate (CB07-Exatecan).

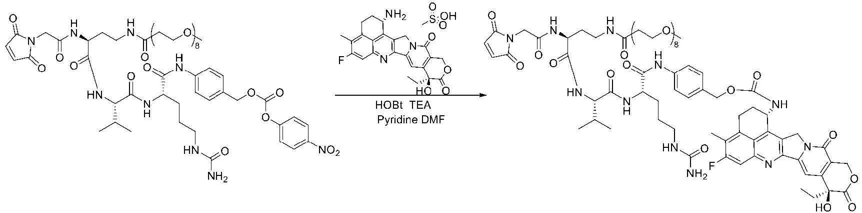

4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (2.6 g, 2.21 mmol) and *N*,*N*-dimethylformamide (23 mL) were added to a reaction flask R1, stirred under a nitrogen atmosphere and cooled to 0-5 °C. At the same time, (1*S*,9*S*)-1-amino-9-ethyl-5-fluoro-9-hydroxy-4-methyl-1,2,3,9,12,15-hexahydro-10*H*,13*H-*benzo[*de*]pyrano[3',4':6,7]indolizino[1,2-*b*]quinolin-10,13-dione methanesulfonate (Exatecan methanesulfonate, 0.98 g, 1.84 mmol, Advanced ChemBlocks) and *N*,*N*-dimethylformamide (5 mL) were added to another reaction flask R2. Triethylamine (230 mg, 2.27 mmol) was added dropwise at 0-5 °C. The mixture was stirred until complete dissolution was achieved. The solution in reaction flask R2 was added dropwise to reaction flask R1. Reaction flask R2 was then washed with *N,N-*dimethylformamide (2 mL), and the washing solution was added to reaction flask R1. 1-Hydroxybenzotriazole (497 mg, 3.68 mmol) and pyridine (1.45 g, 18.4 mmol) were weighed into reaction flask R1. The mixture was stirred at 0-5 °C for 10 min, warmed to room temperature, and stirred for 5.5 h. After the completion of the reaction, the mixture was concentrated under reduced pressure at 35 °C to remove the solvent. The residue was purified by preparative high performance liquid chromatography (prep-HPLC) and lyophilized to obtain a white powder (CB07-Exatecan, 1.6 g, 59%). LC-MS: [1/2M+H]⁺ = 737.

### Example 6. Synthesis of Intermediate (CB07-D-1)

Synthesis of 4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl ((1*S*,9*S*)-9-ethyl-4,5-difluoro-9-hydroxy-10,13-dioxo-2,3,9,10,13,15-hexahydro-1*H*,12*H*-benzo[*de*]pyrano[3',4':6,7]indolizino[1,2-*b*]quinolin-1-yl) carbamate (CB07-D-1).

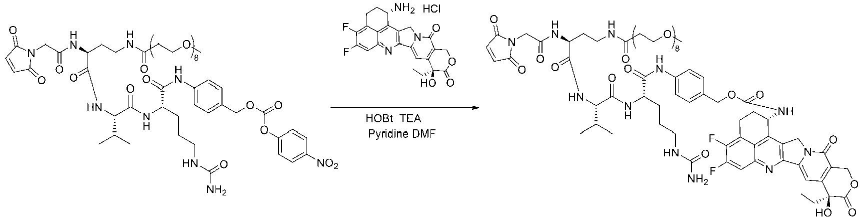

4-((30*S*,33*S*,36*S*)-30-(2-(2,5-dioxo-2,5-dihydro-1*H*-pyrrol-1-yl)acetamido)-33-isopropyl-26,31,34-trioxo-36-(3-ureidopropyl)-2,5,8,11,14,17,20,23-octaoxa-27,32,35-triazaheptatriacontan-37-amido)benzyl (4-nitrophenyl) carbonate (CB07) (220 mg, 0.189 mmol) and *N*,*N*-dimethylformamide (5 mL) were added to a reaction flask R1, stirred under a nitrogen atmosphere and cooled to 0-5 °C. At the same time, (1*S*,9*S*)-1-amino-9-ethyl-4,5-difluoro-9-hydroxy-1,2,3,9,12,15-hexahydro-10*H,*13*H-*benzo[*de*]pyrano[3',4':6,7]indolizino[1,2-b]quinolin-10,13-dione hydrochloride (D-1) (90 mg, 0.189 mmol) and *N*,*N*-dimethylformamide (5 mL) were added to another reaction flask R2. Three drops of triethylamine were added at 0-5 °C. The mixture was stirred until complete dissolution was achieved. The solution in reaction flask R2 was added dropwise to reaction flask R1, and 1-hydroxybenzotriazole (60 mg, 0.44 mmol) and pyridine (0.5 mL) were weighed into reaction flask R1. The mixture was stirred at 0-5 °C for 10 min, warmed to room temperature, and stirred for 3 h. After the completion of the reaction, the mixture was concentrated under reduced pressure to remove the solvent. The residue was purified by preparative high performance liquid chromatography (prep-HPLC) and lyophilized to obtain a white powder (CB07-D-1, 55 mg, 20%). LC-MS: [1/2M+H]⁺ = 739.

### Example 7. Synthesis of Intermediate (CB14-Exatecan)

### Synthesis of 4-((18S,21S,24S)-18-(2-(2,5-dioxo-2,5-dihydro-1H-pyrrol-1-yl)acetamido)-21-isopropyl-14,19,22-trioxo-24-(3-ureidopropyl)-2,5,8,11-tetraoxa-15,20,23-triazapentacosan-25-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl) carbamate (CB14-Exatecan).

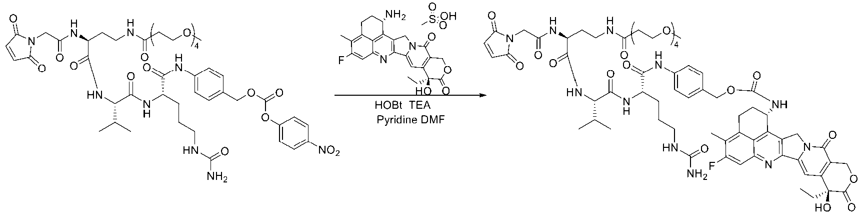

Similarly, 4-((18*S*,21*S*,24*S*)-18-(2-(2,5-dioxo-2,5-dihydro-1*H*-pyrrol-1-yl)acetamido)-21-isopropyl-14,19,22-trioxo-24-(3-ureidopropyl)-2,5,8,11-tetraoxa-15,20,23-triazapentacosan-25-amido)benzyl (4-nitrophenyl) carbonate (190 mg, 0.19 mmol) and *N*,*N*-dimethylformamide (23 mL) were added to a reaction flask R1, stirred under a nitrogen atmosphere and cooled to 0-5 °C. At the same time, (1*S*,9*S*)-1-amino-9-ethyl-5-fluoro-9-hydroxy-4-methyl-1,2,3,9,12,15-hexahydro-10*H*,13*H-*benzo[*de*]pyrano[3',4':6,7]indolizino[1,2-*b*]quinolin-10,13-dione methanesulfonate (Exatecan methanesulfonate, 101 mg, 0.19 mmol, Advanced ChemBlocks) and *N*,*N*-dimethylformamide (5 mL) were added to another reaction flask R2. Triethylamine (3 drops) was added dropwise at 0-5 °C. The mixture was stirred until complete dissolution was achieved. The solution in reaction flask R2 was added dropwise to reaction flask R1. Reaction flask R2 was then washed with *N*,*N*-dimethylformamide (1 mL), and the washing solution was added to reaction flask R1. 1-Hydroxybenzotriazole (60 mg, 0.44 mmol) and pyridine (0.5 mL) were weighed into reaction flask R1. The mixture was stirred at 0-5 °C for 10 min, warmed to room temperature, and stirred for 5.5 h. After the completion of the reaction, the mixture was concentrated under reduced pressure at 35 °C to remove the solvent. The residue was purified by preparative high performance liquid chromatography (prep-HPLC) and lyophilized to obtain CB14-Exatecan in the form of a white powder.

### Example 8. Synthesis of ADCs

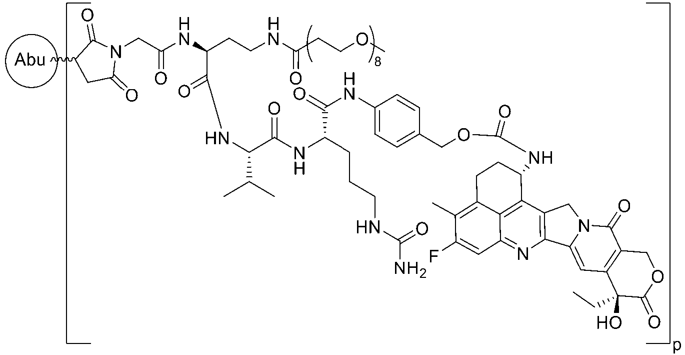

### ADC1 conjugation process:

Reduction reaction: the concentration of Antibody 1 was adjusted to 18 g/L using a conjugation buffer (10 mM aqueous solution of succinic acid); a 0.1 M aqueous solution of EDTA was added until the final concentration of EDTA reached 2 mM; 5.4 molar equivalents of a 0.2 M aqueous solution of TCEP was added according to the amount of the antibody substance; the pH was adjusted to 7; and then the system was stirred and reacted at 25 °C and 180 rpm for 2 h, such that the interchain disulfide bond of the antibody was reduced to free sulfhydryl.

The buffer was exchanged by ultrafiltration for 10 volumes using an ultrafiltration membrane with 30 KD to remove the reducing agent TCEP in the system.

Conjugation reaction: 15-fold molar equivalents of a 100 mM solution of CB07-Exatecan in dimethylacetamide (DMA) was added according to the amount of the antibody substance. The system was stirred and reacted at 25 °C and 180 rpm for 2 h. Finally, a 0.2 M aqueous solution of *N-*acetylcysteine was added until its final concentration reached 2 mM, and the system was reacted for another 15 min to terminate the conjugation reaction. The reaction mixture was subjected to purification and ultrafiltration to obtain ADC1 at a concentration of 23.56 mg/mL, and the DAR (namely p) was 8 as measured by reversed-phase chromatography (batch 1).

### ADC2 conjugation process:

Reduction reaction: the concentration of Antibody 1 was adjusted to about 18 g/L using a conjugation buffer (10 mM aqueous solution of succinic acid); a 0.1 M aqueous solution of EDTA was added until the final concentration of EDTA reached 2 mM; 2.5 molar equivalents of a 10 mM aqueous solution of TCEP was added according to the amount of the antibody substance; the pH was adjusted to 7; and then the system was shaken on a shaker and reacted at 25 °C for 2 h, such that the interchain disulfide bond of the antibody was reduced to free sulfhydryl.

The buffer was exchanged by ultrafiltration for 10 volumes to remove the reducing agent TCEP in the system.

Conjugation reaction: 6-fold molar equivalents of a 100 mM solution of CB07-Exatecan in dimethylacetamide (DMA) was added according to the amount of the antibody substance. The system was shaken on the shaker and reacted at 25 °C for 1 h. Finally, a 0.1 M aqueous solution of *N-*acetylcysteine was added until its final concentration reached 1.5 mM, and the system was reacted for another 15 min to terminate the conjugation reaction. The reaction mixture was subjected to purification to obtain ADC2 at a concentration of 1.34 mg/mL, and the DAR (namely p) was 4 as measured by reversed-phase chromatography.

### Example 9. Clinical Study on ADC1

The study was a multicenter, open-label, dose-escalation and dose-expansion phase I clinical study employing rapid titration and the "3 + 3" dose escalation rule to explore the safety, tolerability and PK characteristics of ADC1 for injection in patients with advanced solid tumors.

### Study objectives

### Primary objectives:

To evaluate the safety and tolerability of ADC1 in patients with advanced solid tumors, explore the maximum tolerated dose (MTD) and provide recommended doses for subsequent clinical trials.

### Secondary objectives:

To characterize the pharmacokinetic (PK) profile and metabolite profile of ADC1 after single and multiple doses in patients with advanced solid tumors;
To evaluate the immunogenicity of ADC1;
To preliminarily evaluate the antitumor efficacy of ADC1;
To investigate the correlation between the antitumor efficacy of ADC1 and folate receptor expression in tumor tissue as well as FRα levels in serum.

### Study endpoints:

### Primary endpoints:

Tolerability and safety endpoints: Vital signs, physical examinations, laboratory examinations, electrocardiogram, cardiac color ultrasound, adverse events, dose-limiting toxicity (DLT) events and their incidence rates, etc.

### Secondary endpoints:

Pharmacokinetic characteristics and metabolite analysis under single and multiple doses;
Immunogenicity;
Clinical efficacy: Objective response rate (ORR), duration of response (DOR), disease control rate (DCR), progression-free survival (PFS), overall survival (OS);
Tumor marker testing;
Biomarkers: Folate receptor expression in tumor tissue, FRα levels in serum.

### Inclusion criteria

1. Aged 18-75 years (inclusive), with no gender restriction;
2. For different cohorts, subjects are required to meet the following requirements:
   2-1) Dose escalation study: patients with histologically or cytologically confirmed advanced or metastatic solid tumors, who have failed standard therapy, or have no effective standard therapy, and who are intolerant to standard therapy or refuse standard therapy; the specific eligibility may be determined by clinicians based on different tumor types;
   2-2) Dose expansion study: cohort 1) ovarian cancer: (1) patients with histologically or cytologically confirmed platinum-resistant advanced or metastatic epithelial ovarian cancer, primary peritoneal cancer, or fallopian tube cancer, and with no more than three prior lines of therapy; (2) platinum resistance defined as platinum-resistant recurrent ovarian cancer according to the criteria of the U.S. Gynecologic Oncology Group (GOG) (radiologically confirmed disease progression or recurrence within 6 months after receiving platinum-containing chemotherapy); (3) positive FRα expression in tumor tissues; cohort 2) other advanced solid tumors: (1) patients with histologically or cytologically confirmed advanced or metastatic solid tumors, who have failed standard therapy or are intolerant to standard therapy, including only endometrial cancer, breast cancer, and non-small cell lung cancer; (2) Tumor tissues available for measurement of the FRα expression level can be provided.
3. At least one measurable tumor lesion is present according to the RECIST1.1 criteria;
4. The U.S. Eastern Cooperative Oncology Group (ECOG) performance status scores 0 or 1;
5. Expected survival ≥12 weeks as assessed by the investigator;
6. Have sufficient organ and bone marrow reserve functions;
7. Female patients with fertility must have a negative serum pregnancy test within 7 days prior to the first administration and be willing to take effective birth control/contraceptive methods to prevent pregnancy during the study until 6 months after the last administration of the study. Male patients must agree to take effective contraceptive methods during the study until 6 months after the last administration of the study; postmenopausal women must have amenorrhea for at least 12 months before they are considered infertile;
8. Dose escalation study: willing to provide previously archived or fresh tumor tissue samples (exemption is allowed if there are no previously archived tumor tissues and the investigator assesses that there is a high risk for patients to re-collect specimens of primary or metastatic tumor tissue); dose expansion study: must be willing to provide previously archived or fresh tumor tissue samples available for measurement of the FRα expression level;
9. Able to understand the study requirements and willing and able to comply with the study and follow-up procedures.

### Exclusion criteria

1. Received investigational drug treatment or participated in clinical studies of medical devices within 4 weeks prior to the first administration of the study drug;
2. Received anti-tumor treatment such as chemotherapy, radiotherapy, small-molecule targeted therapy, biological therapy, endocrine therapy, or immunotherapy within 4 weeks prior to the first administration of the study drug (if the 5-fold half-life of the drug is less than 28 days, the eligibility may be determined based on its pharmacological characteristics after discussion), excluding the following: (1) nitrosourea or mitomycin C is taken within 6 weeks prior to the first administration of the study drug; (2) oral fluorouracils are administered within 2 weeks prior to the first administration of the study drug or within 5 half-lives of the drug (whichever is longer); (3) systemic therapy with traditional Chinese medicines/Chinese patent medicines with definite anti-tumor effects or drugs with immunomodulatory effects (including but not limited to thymosin, interferon, interleukin, etc.) is performed within 2 weeks prior to the first administration of the study drug; (4) palliative radiotherapy is performed within 2 weeks prior to the first administration of the study drug;
3. A history of drug-related or unclear-related grade ≥3 AEs (graded using CTCAE v5.0) after previous treatment with any topoisomerase I inhibitor (e.g., irinotecan);
4. Patients with persistent grade >1 AEs (CTCAE5.0) caused by previous anti-tumor treatment prior to the first administration of the study drug, excluding the following: a. alopecia; b. hyperpigmentation; c. distal toxicity induced by prior chemotherapy or radiotherapy that is judged irreversible; d. hypothyroidism stabilized with hormone replacement therapy;
5. Patients who have received a major surgery or have not recovered after the surgery, or have experienced significant trauma within 4 weeks prior to the first administration of the study drug, or require an elective surgery during the study;
6. Patients with a history of allogeneic cell or solid organ transplantation surgery;
7. Patients with primary central nervous system tumors, symptomatic central nervous system metastasis, meningeal metastasis, or a history of epilepsy. Patients with asymptomatic central nervous system metastasis that is clinically controlled or symptomatic central nervous system metastasis that is judged to be stable by the investigator may be included, provided the following conditions are met: a. clinical symptoms have been stable for ≥4 weeks prior to the first administration; b. no evidence of central nervous system disease progression on enhanced cranial MRI within 4 weeks prior to the first administration is found; c. antiepileptic drugs have been discontinued for ≥2 weeks prior to the first administration, and the prednisone dose is ≤10 mg/day or an equivalent dose of hormones;
8. Other active malignant tumors within 5 years prior to the first administration, except for locally cured tumors (e.g., basal cell carcinoma of the skin, squamous cell carcinoma of the skin, superficial bladder cancer, breast cancer *in situ,* etc.);
9. The following cardiovascular diseases occurred within 6 months prior to the first administration: symptomatic heart failure of the New York Heart Association (NYHA) class II or higher, left ventricular ejection fraction (LVEF) <50%, unstable arrhythmia, unstable angina pectoris, myocardial infarction requiring treatment, pulmonary embolism, or uncontrolled hypertension (defined in this protocol as systolic blood pressure >160 mmHg and/or diastolic blood pressure >100 mmHg after optimal antihypertensive treatment, with clinical significance as assessed by the investigator);
10. Suffering from any other disease or unsuitable for the use of the study drug as judged by the investigator based on physical examination or laboratory test results;
11. Patients who have previously experienced non-infectious pneumonia/lung inflammation that required glucocorticoid therapy, or who currently have interstitial lung disease (ILD), or in whom the possibility of interstitial lung disease/lung inflammation cannot be excluded by imaging during the screening period;
12. Subjects with tuberculosis in need of treatment or under treatment, including but not limited to pulmonary tuberculosis; patients who have undergone standard anti-tuberculosis treatment and have been confirmed by the investigator to be cured may be included;
13. Serious infection within 4 weeks or active infection within 2 weeks prior to the first administration;
14. Patients infected with the following diseases: human immunodeficiency virus (HIV) infection; active hepatitis B virus infection [hepatitis B surface antigen (HBsAg) positive, and hepatitis B virus deoxyribonucleic acid (HBV-DNA) test results are >200 IU/ml or 10³ copies/ml]; hepatitis C virus infection [HCV antibody and viral ribonucleic acid (HCV-RNA) test results are positive]; treponema pallidum antibody positive and RPR positive;
15. Known hypersensitivity or delayed-type hypersensitivity to any ingredient of the study drug;
16. Patients with a known history of grade ≥3 allergic reactions to macromolecular protein formulations/monoclonal antibodies;
17. Patients with uncontrolled pleural effusion, pericardial effusion, or seroperitoneum, or those requiring drainage;
18. Patients with the following thrombosis or bleeding risks:
   a. cerebrovascular accident or transient cerebral ischemic attack within 6 months prior to the first administration;
   b. a history of deep vein thrombosis, pulmonary embolism, or any other severe thromboembolism within 3 months prior to the first administration (thrombosis related to implanted venous infusion ports or catheters, or superficial venous thrombosis is not considered as "severe" thromboembolism);
   c. any life-threatening bleeding event or grade 3 or 4 gastrointestinal/variceal bleeding event requiring transfusion, endoscopy, or surgical treatment within 3 months prior to the first administration;
   d. other diseases with a relatively high bleeding or thrombosis risk in the future, in the opinion of the investigator;
19. Any other severe underlying diseases (e.g., poorly controlled diabetes, active gastric ulcer, poorly controlled convulsions, coagulation disorders with severe symptoms or signs);
20. A known history of mental illness, substance abuse, alcoholism, or drug addiction that may affect the study results;
21. Pregnant or lactating women, or women or men planning to conceive;
22. The patient's compliance to participate in this clinical study estimated to be insufficient, or other factors deemed inappropriate for study participation by the investigator.

The study was divided into two parts:
Part 1: Dose escalation study. Rapid titration and the "3 + 3" dose escalation rule were used to explore the safety, tolerability and pharmacokinetic characteristics of ADC1 for injection (batch 2, with a DAR of 7.4).
Dose cohorts were established at 1.2 mg/kg, 1.8 mg/kg, 2.1 mg/kg, and 2.4 mg/kg. Among these dose cohorts, the 1.2 mg/kg cohort was subjected to dose escalation using a rapid titration method; the 1.8 mg/kg cohort, 2.1 mg/kg cohort and 2.4 mg/kg cohort were subjected to dose escalation following the standard "3 + 3" rule.
Administration regimen: the administration was performed by intravenous infusion once every 3 weeks (Q3W), with an infusion time of ≥ 90 min recommended for the first cycle. If no infusion reaction occurs, the infusion in subsequent cycles may be completed within 30~120 min

### Definition of dose-limiting toxicity (DLT):

AEs were graded according to National Cancer Institute Common Terminology Criteria for Adverse Events (NCI CTCAE), version 5.0. DLT refers to any grade 3 or higher toxicity related to a test drug that occurs within 21 days after a subject is first given the drug and meets the following definitions:
▪ Grade 5 toxicity
▪ Definition of hepatotoxicity DLT:
   ·Grade 4 AST or ALT elevation
   ·AST or ALT > 5-fold upper limit of normal (ULN), with ≥ Grade 2 blood bilirubin elevation ·Subjects with hepatocellular carcinoma or liver metastasis, e.g., AST or ALT ≤ 3-fold ULN at baseline, AST or ALT elevation > 5-fold ULN in a DLT evaluation period and lasting > 5 days
   ·Subjects with hepatocellular carcinoma or liver metastasis, e.g., AST or ALT > 3-fold ULN at baseline, AST or ALT elevation > 8-fold ULN in a DLT evaluation period and lasting > 5 days
   ·Subjects without hepatocellular carcinoma or liver metastases, AST or ALT elevation > 5-fold ULN and lasting > 5 days
▪ Definition of hematological toxicity DLT:
   ·Grade 4 neutropenia lasting > 7 days
   ·≥ Grade 3 neutropenia with fever (single body temperature > 38.3 °C or sustained body temperature ≥ 38 °C for more than 1 hour)
   ·Grade 4 anemia
   ·Grade 4 thrombocytopenia
   ·≥ Grade 3 thrombocytopenia lasting > 7 days
   ·≥ Grade 3 thrombocytopenia with bleeding
▪ Definition of non-hepatotoxicity and non-hematological toxicity DLT:
   ·Other ≥ Grade 3 non-hepatotoxicity and non-hematological toxicity
▪ The following treatment-emergent adverse events (TEAEs) are not considered DLT:
   ·Grade 3 nausea, vomiting, diarrhea, anorexia, fatigue or rash that can be controlled by standard supportive treatment and relieved to ≤ Grade 2 within 3 days
   Abnormalities in laboratory examinations without clinically relevant symptoms or signs, including elevated ALP, elevated uric acid, elevated blood amylase, elevated lipase or decreased lymphocyte to Grade 3 or 4, with Grade 1 hyponatremia at baseline escalating to Grade 3 but lasting < 72 hours
   ·Grade 3 infusion reaction that can be relieved within 6 hours after treatment.

### Evaluation of tolerability and safety:

Tolerability evaluation indexes: dose-limiting toxicity (DLT) events and their incidence rates.

Safety evaluation indexes: vital signs, physical examinations, laboratory examinations (blood routine examination, blood biochemistry, cardiac function, thyroid function, coagulation routine, urinalysis routine, stool routine, and pregnancy test), ECOG score, electrocardiogram, adverse events, etc.

### Pharmacokinetic evaluation:

Subjects in all dose cohorts were required for blood sample collection at specified time points during treatment (first 6 treatment cycles), and the plasma concentrations (C_{trough}) were monitored for 24 h before administration. Concentration levels of ADC1, Exatecan and total anti-FRα antibody in the serum were examined to investigate pharmacokinetic (PK) characteristics of ADC1.

The following PK parameters were calculated from the actual administration doses and actual sampling time by the non-compartmental model:
Single administration: Cₘₐₓ, Tₘₐₓ, T_{1/2}, CL, Vd, Ke, MRT, AUC_{(0-τ)}, and AUC_{(0-∞)};
Multiple administrations: C_{max, ss}, C_{avg, ss}, C_{min, ss}, AUC_{(0-τ)ss}, AUC_{(0-∞)ss}, T_{max, ss}, T_{1/2, ss}, CL, Vₛₛ, Ke, MRT, accumulation index (R_{ac}), and fluctuation index DF.

### Immunogenicity evaluation:

Subjects in all dose cohorts were required for blood sample collection at specified time points during treatment to detect anti-drug and neutralizing antibodies in the serum.

The immunogenicity evaluation indexes are as follows:
Sample positive rate and individual positive rate for anti-ADC1 antibody (ADA);
ADA-positive samples will be further tested for the presence of neutralizing antibodies (Nab);
Number and percentage of subjects positive for ADA and Nab.

### Pharmacodynamic evaluation:

1. Detection of FRα levels in serum prior to administration;
2. Folate receptor expression levels in archive tumor tissue samples or tumor tissue samples freshly collected during the screening phase;
3. Changes in tumor markers.

### Clinical efficacy evaluation:

Throughout the trial, the anti-tumor efficacy of ADC1 for injection was evaluated by using the RECIST 1.1 evaluation standard.

The anti-tumor efficacy indexes include: objective response rate (ORR), duration of response (DOR), disease control rate (DCR), progression-free survival (PFS), and overall survival (OS) as defined by RECIST 1.1.

### Dose Escalation Study Results

### I. Enrollment in the Dose Escalation Phase

In the Phase I dose escalation study of ADC1, a total of 29 subjects with advanced solid tumors received at least one dose of ADC1. Subjects were enrolled into the following dose cohorts: 1.2 mg/kg (N=1), 1.8 mg/kg (N=5), 2.1 mg/kg (N=9), and 2.4 mg/kg (N=14). The enrolled tumor types included ovarian cancer (n=16), breast cancer (n=6), lung cancer (n=2), cervical cancer (n=2), with one case each of fallopian tube cancer, tongue cancer, and endometrial cancer. Baseline characteristics are presented in Table 2.

**Table 2. Baseline characteristics**

| | 1.2 mg/kg (N=1) | 1.8 mg/kg (N=5) | 2.1 mg/kg (N=9) | 2.4 mg/kg (N=14) |
|---|---|---|---|---|
| Age (median, min-max) | 63 | 58(37-67) | 53(43-70) | 58.5(38-70) |
| Male/Female | 0/1 | 1/4 | 0/9 | 2/12 |
| ECOG 0/1 | 0/1 | 0/5 | 0/9 | 1/13 |
| Tumor type | breast cancer (n=1) | breast cancer (n=1) | breast cancer (n=2) | breast cancer (n=2) |
| | | ovarian cancer (n=2) | | ovarian cancer (n=8) |
| | | | ovarian cancer (n=6) | |
| | | endometrial cancer (n=1) | | lung cancer (n=2) |
| | | | cervical cancer (n=1) | |
| | | tongue cancer (n=1) | | fallopian tube cancer (n=1) |
| | | | | cervical cancer (n=1) |
| Prior radiotherapy (Yes/No) | 1/0 | 2/3 | 2/7 | 3/11 |
| Prior PARPi (Yes/No) | 0/1 | 2/3 | 4/5 | 5/9 |
| Prior surgery (Yes/No) | 1/0 | 5/0 | 7/2 | 12/2 |
| Median cycles of study drug (min-max) | 2 | 2(2) | 2 (1-3) | 4 (2-11) |
| Study continuation (Yes/No) | 0/1 | 5/0 | 8/1 | 7/7 |

### II. Preliminary Efficacy Data in the Dose Escalation Phase

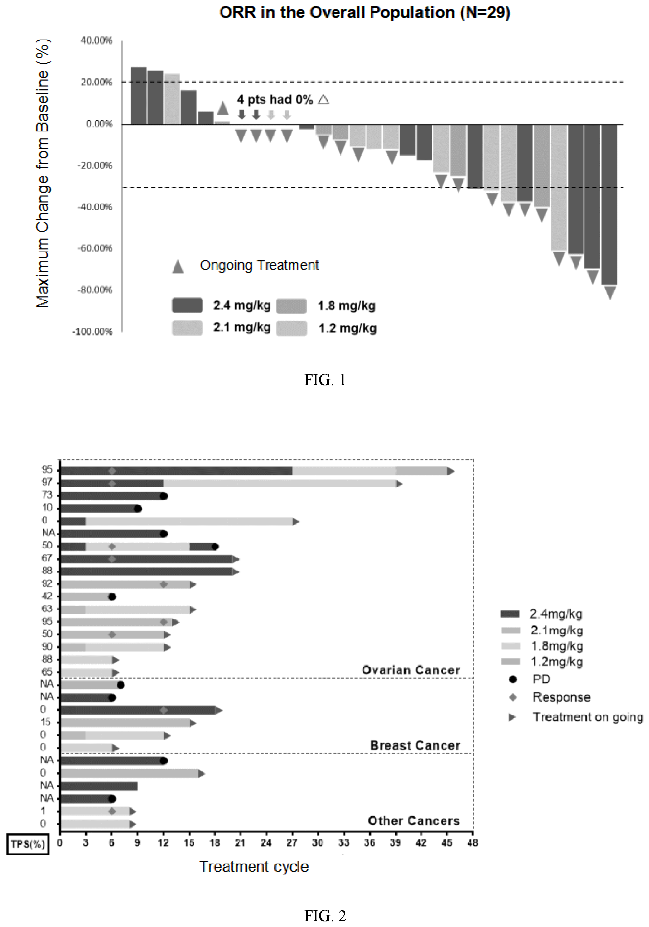

A total of 29 subjects underwent at least 1 efficacy assessment, among whom 9 cases achieved partial response (PR) (7 cases with FRα expression ≥50%), including 6 cases of ovarian cancer, 1 case of fallopian tube cancer, 1 case of breast cancer, and 1 case of endometrial cancer, and 16 cases achieved stable disease (SD). Currently, the objective response rate (ORR) was 31%, and the disease control rate (DCR) was 86.2% (FIG. 1). Most subjects with ovarian cancer had received 3 lines or more of anti-tumor treatment, including bevacizumab, PARPi, and other drugs. All PR cases achieved partial response at the first or second tumor assessment; no subjects discontinued study treatment due to safety issues; some subjects have been treated for more than 1 year (FIG. 2).

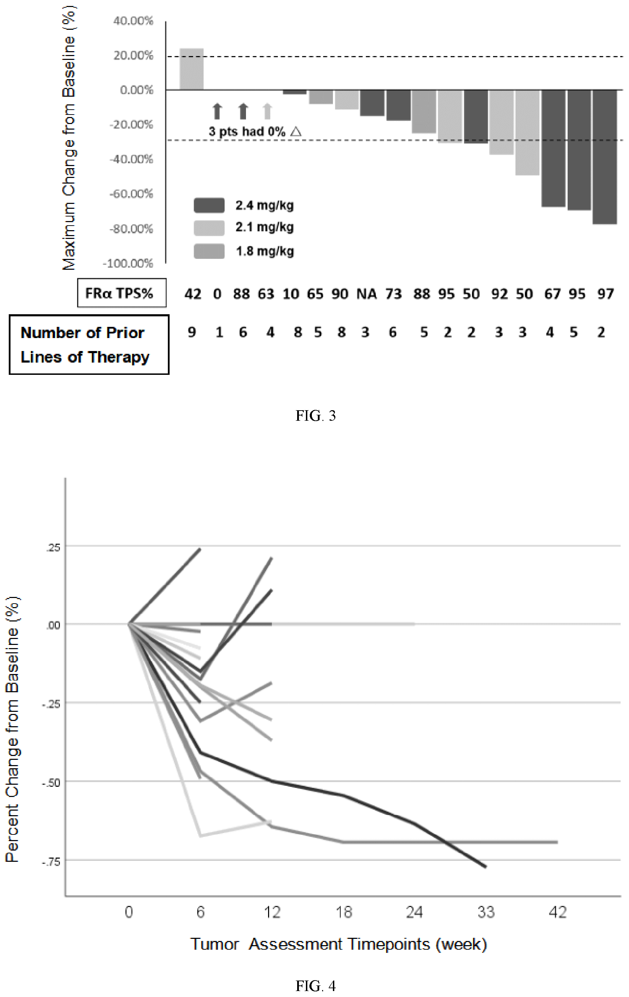

Among subjects with ovarian cancer or fallopian tube cancer, the ORR was 7/17 (41%) and the DCR was 88.2%. Among 14 subjects with ovarian cancer or fallopian tube cancer and with FRα TPS (Tumor cell Proportion Score) >25%, the ORR was 7/14 (50%) and the DCR was 92.8%. Among 15 subjects with ovarian cancer (including fallopian tube cancer) in the 2.1 mg/kg and 2.4 mg/kg dose cohorts, regardless of the FRα expression level, the ORR was 46.7% and the DCR was 86.7%. Among 12 of these subjects with FRα TPS >25%, the ORR was 7/12 (58.3%), 4/6 (66.7%) in the 2.4 mg/kg cohort, and 3/6 (50%) in the 2.1 mg/kg cohort, and the DCR was 91.7% (FIGs. 3 and 4). Disease response was also observed in subjects with breast cancer or endometrial cancer (see Table 3).

**Table 3. Effect of ADC1 on breast cancer or endometrial cancer**

| Tumor type | First dose | FRα TPS | Best change from baseline |
|---|---|---|---|
| breast cancer | 2.4 mg/kg | Undetected | Progression of disease (PD) |
| | 2.4 mg/kg | Negative | PR |
| | 2.1 mg/kg | 15% | SD |
| | 2.1 mg/kg | Negative | SD |
| | 1.8 mg/kg | Negative | SD |
| | 1.2 mg/kg | Undetected | SD |
| endometrial cancer | 1.8 mg/kg | 1% | PR |

The most common adverse events were decreased platelet count, decreased neutrophil count, anemia, leukopenia, nausea, vomiting, and constipation.

Grade ≥3 treatment-related adverse events (TRAEs) were mainly hematological toxicity.

### III. Preliminary Pharmacokinetic Data in the Dose Escalation Phase

A total of 20 subjects treated with ADC1 were included in the PK analysis. After the first dose, the systemic exposure of ADC1 increased with ascending dose levels. The PK parameters for the 1.2, 2.1 and 2.4 mg/kg dose cohorts are presented in Tables 4-6.

**Table 4. PK parameters of ADC1**

| | Cmax (ng/mL) | Tmax* (h) | AUC_{0~21d} (h*ng/mL) | AUCinf (h*ng/mL) | T_{1/2} (h) | CL (L/h) | Vss (L) |
|---|---|---|---|---|---|---|---|
| 1.2 mg/kg (N=1) | 23400 | 2.5 | 1157465 | 1158497 | 49.34 | 0.06 | - |
| 2.1 mg/kg (N=5) | 47940 | 2.22 | 2242726 | 2250106 | 59.88 | 0.07 | - |
| 2.4 mg/kg (N=14) | 59057.14 | 2.79 | 3060417 | 3086562 | 59.50 | 0.06 | 3.04 (N=4) |

**Table 5. PK parameters of total anti-FRα antibody**

| | Cmax (ng/mL) | Tmax* (h) | AUC_{0~21d} (h*ng/mL) | AUCinf (h*ng/mL) | T_{1/2} (h) | CL (L/h) | Vss (L) |
|---|---|---|---|---|---|---|---|
| 1.2 mg/kg (N=1) | 24200 | 3.0 | 992244.9 | 993050.9 | 46.89 | 0.06 | - |
| 2.1 mg/kg (N=5) | 48880 | 2.02 | 2242726 | 2110312 | 63.059 | 0.07 | - |
| 2.4 mg/kg (N=14) | 59114.29 | 2.72 | 2829804 | 2861399 | 65.864 | 0.06 | 3.51 (N=4) |

**Table 6. PK parameters of Exatecan**

| | Cmax (ng/mL) | Tmax* (h) | AUC_{0~21d} (h*ng/mL) | AUCinf (h*ng/mL) | T_{1/2} (h) | CL (L/h) | Vss (L) |
|---|---|---|---|---|---|---|---|
| 1.2 mg/kg (N=1) | 2.63 | 26 | 193.17 | 195.97 | 89.24 | 325.57 | - |
| 2.1 mg/kg (N=5) | 5.56 | 15.92 | 414.46 | 417.08 | 74.30 | 368.25 | - |
| 2.4 mg/kg (N=14) | 5.01 | 25.90 | 371.03 | 374.37 | 79.72 | 448.48 | 27803.38 (N=3) |

Part 2: Dose expansion study. An analysis of the safety and efficacy of body surface area-corrected doses administered to currently enrolled subjects showed that the safety was relatively good and the potential for efficacy benefit was high when the administered dose was about 87-93 mg/m². Two doses, 93 mg/m² and 84 mg/m², were selected for expansion in cohort 1 and cohort 2, respectively. Subjects with ovarian cancer or other tumor types will be randomly assigned in a 1:1 ratio to receive ADC1 at 93 mg/m² Q3W or 84 mg/m² Q3W (see Table 7).

**Table 7. Grouping and Dosing**

| Group | Population | Administration dosage |
|---|---|---|
| Cohort 1a | ovarian cancer | 93 mg/m² |
| Cohort 1b | ovarian cancer | 84 mg/m² |
| Cohort 2a | other solid tumors | 93 mg/m² |
| Cohort 2b | other solid tumors | 84 mg/m² |

Baseline characteristics of patients with ovarian cancer:
As of May 8, 2024, 96 subjects with platinum-refractory or platinum-resistant epithelial ovarian cancer, fallopian tube cancer, or primary peritoneal cancer (PROC) were enrolled and received ADC1 at 1.8 mg/kg (N = 2), 2.1 mg/kg (N = 16), 2.4 mg/kg (N = 15), 84 mg/m² (N = 32), or 93 mg/m² (N = 31). Most subjects (89.6%, 86/96) had not previously received pelvic radiotherapy. All subjects have received bevacizumab treatment, with 53.1% (51/96) of the subjects having received PARPi treatment.

**Table 8. Baseline characteristics of subjects with ovarian cancer (N=96)**

| | 1.8 mg/kg (N=2) | 2.1 mg/kg (N=16) | 2.4 mg/kg (N=15) | 84 mg/m² (N=32) | 93 mg/m² (N=31) |
|---|---|---|---|---|---|
| Age (median, min-max) | 57.5(55-60) | 52.5(43-74) | 58.0(38-70) | 56.0(36-74) | 55.0(32-70) |
| ECOG 0/1 | 0/2 | 1/15 | 2/13 | 4/28 | 9/22 |
| Prior surgery (Yes/No) | 2/0 | 16/0 | 15/0 | 32/0 | 31/0 |
| Prior radiotherapy (Yes/No) | 1/1 | 1/15 | 2/13 | 3/29 | 3/28 |
| Prior PARPi (Yes/No) | 2/0 | 9/7 | 8/7 | 16/16 | 16/15 |
| Prior lines of therapy (median, min-max) | 5(5-5) | 4(1-9) | 3(1-8) | 3(1-5) | 3(1-5) |
| Median cycle of study drug (min-max) | 12(11-13) | 7(2-17) | 6(2-22) | 3(1-10) | 2(1-13) |
| Study continuation (Yes/No) | 2/0 | 10/6 | 6/9 | 24/8 | 28/3 |

Efficacy in patients with ovarian cancer:
As of May 8, 2024, 54 subjects with PROC received at least one tumor assessment following the ADC1 treatment, with 38.9% (21/54) of the subjects having received >3 prior lines of systemic therapy. Regardless of the FRα expression level, the ORR (including unconfirmed PR) was 37.0% (20/54), and the DCR was 77.8% (42/54) in all subjects. Among subjects with FRα TPS <50%, the ORR was 33.3% (7/21), and the DCR was 71.4% (15/21). Among subjects with FRα TPS ≥50%, the ORR was 39.4% (13/33), and the DCR was 81.8% (27/33) (see Table 9).

**Table 9. The ORR in ovarian cancer patients (N=54)**

| | All (N=54) | FRα< 50% (N=21) | FRα≥ 50% (N=33) | FRα≥ 75% (N=15) |
|---|---|---|---|---|
| ORR | 20(37.0%) | 7*(33.3%) | 13^{#}(39.4%) | 7(46.7%) |
| DCR | 42(77.8%) | 15(71.4%) | 27(81.8%) | 14(93.3%) |
| CR | 0 | 0 | 0 | 0 |
| PR | 20(37%) | 7*(33.3%) | 13^{#}(39.4%) | 7(46.7%) |
| SD | 22(40.7%) | 8(38.1%) | 14(42.4%) | 7(46.7%) |
| PD | 12(22.2%) | 6(28.6%) | 7(21.2%) | 1(6.7%) |

| | | | | |
|---|---|---|---|---|
| Note: * including 3 unconfirmed PR, # including 2 unconfirmed PR | | | | |

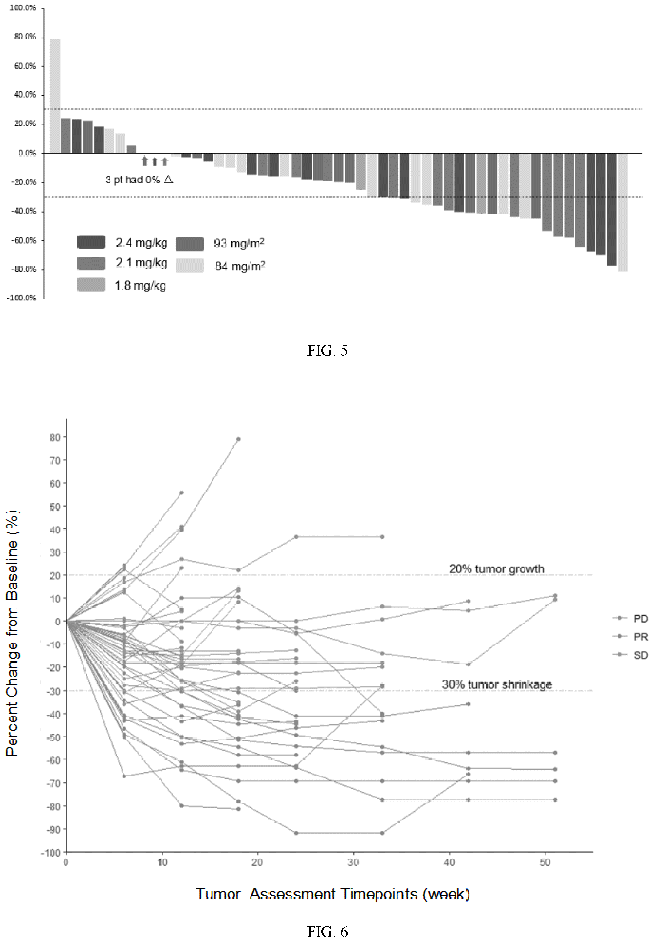

FIGs. 5 and 6 show that most of the subjects receiving the ADC1 treatment had tumor shrinkage, and the study treatment resulted in sustained shrinkage of target tumor lesions in most subjects.

With a median follow-up time of 6.5 months (1.3, 18.0), the median duration of response (mDOR) was 6.3 months (1.8-16.5 months). The majority of PR subjects remain on study treatment.

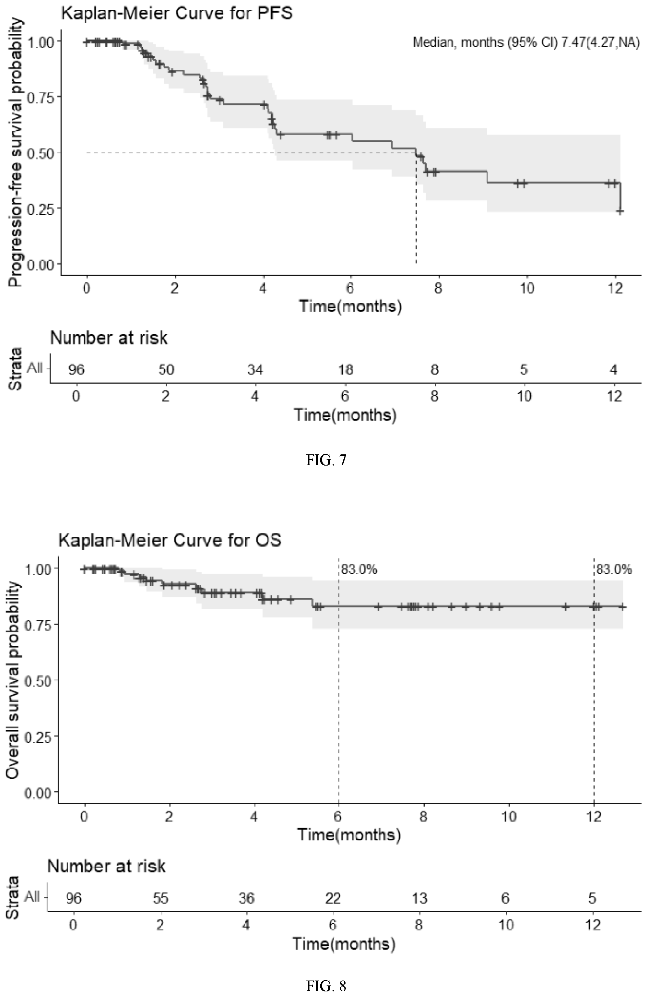

The Kaplan-Meier curve indicated that the mPFS was 7.47 months (4.27~NA), and the 6-month and 1-year OS rates were both 83.0% (see FIGs. 7-8).

The safety of ADC1 is favorable. No ILD or notable ocular toxicity was reported. Regardless of the FRα expression level, ADC1 showed superior preliminary efficacy in all subjects with PROC.

### Example 10. A Multicenter, Open-Label Phase II Clinical Study of ADC1 (DAR 7-8) in Patients with Platinum-Resistant Ovarian Cancer

The patients in the study were subjects with histologically or cytologically confirmed, platinum-resistant, advanced or metastatic epithelial ovarian, primary peritoneal, or fallopian tube cancer, who had received no more than three prior lines of therapy.

Definition of platinum-resistant ovarian cancer:
a. patients who have received only first-line of platinum-based drug treatment must have undergone at least 4 cycles of platinum-containing treatment and achieved a response (CR or PR), with disease progression occurring more than 1 month but within 6 months after the last platinum-containing chemotherapy;
b. disease progression must have occurred within 6 months of the last platinum-containing treatment in patients who have received 2 or 3 platinum-based treatments.

Note: 1) progression should be calculated from the date of the last platinum-containing treatment to the date of radiologically confirmed disease progression; 2) patients who were platinum-refractory during first-line treatment are excluded.

### Study design:

Part I: Dose finding study. Three dose cohorts were set: dose A: 76 mg/m², dose B: 84 mg/m², and dose C: 93 mg/m². Patients were stratified according to FRα expression levels (TPS < 50%, 50% ≤ TPS < 75%, TPS ≥ 75% [FRα non-evaluable subjects will be included in the TPS < 50% cohort]).

Part II: Dose expansion study. The dose administered will be determined based on the data generated in Part I.

Administration regimen: intravenous infusion, once every 3 weeks (Q3W), with an intravenous infusion time of ≥90 min recommended for the first administration cycle. If no infusion reaction occurs, the infusion in subsequent cycles may be completed within 30-60 min.

## Claims

1. A method for treating and/or preventing cancer, comprising administering to a human patient in need an effective amount of an antibody-drug conjugate by injection; wherein, the antibody-drug conjugate is administered at a dose of 1.7 mg/kg to 2.5 mg/kg, or 100 mg to 250 mg, or 70 mg/m² to 100 mg/m² ,or 75 mg/m² to 100 mg/m² once every 2-4 weeks; the antibody-drug conjugate comprises an anti-FRα antibody or an antigen-binding unit thereof conjugated to a drug via a linker.

2. A method for treating and/or preventing cancer, comprising administering to a human patient in need an effective amount of an antibody-drug conjugate by injection; wherein, the antibody-drug conjugate is administered at a dose of 1.7 mg/kg to 2.5 mg/kg, or 100 mg to 250 mg, or 70 mg/m² to 100 mg/m² ,or 75 mg/m² to 100 mg/m² once every 2-4 weeks; the antibody-drug conjugate has a structure of Formula I, or a stereoisomer thereof, or a pharmaceutically acceptable salt or solvate thereof: 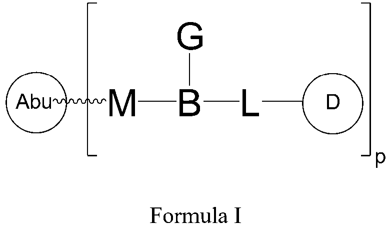 wherein,
Abu is an anti-FRα antibody or an antigen-binding unit thereof;
D is a drug;
M is 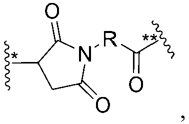 wherein * links to Abu, ** links to B, and R is selected from: -(CH₂)ᵣ-, - (CHR^{m})_{c}, C3-C8 carbocyclyl, -O-(CH₂)ᵣ-, arylene, -(CH₂)ᵣ-arylene-, -arylene-(CH₂)r-, -(CH₂)ᵣ-(C3-C8 carbocyclyl)-, -(C3-C8 carbocyclyl)-(CH₂)ᵣ-, C3-C8 heterocyclyl, -(CH₂)ᵣ-(C3-C8 heterocyclyl)-, -(C3-C8 heterocyclyl)-(CH₂)ᵣ-, -(CH₂)ᵣC(O)NR^{m}(CH₂)ᵣ-, -(CH₂CH₂O)ᵣ-, -(CH₂CH₂O)ᵣ-CH₂-, - (CH₂)ᵣC(O)NR^{m}(CH₂CH₂O)ᵣ-, -(CH₂)ᵣC(O)NR^{m}(CH₂CH₂O)ᵣ₋CH₂-, - (CH₂CH₂O)ᵣC(O)NR^{m}(CH₂CH₂O)ᵣ, -(CH₂CH₂O)ᵣC(O)NR^{m}(CH₂CH₂O)ᵣ-CH₂- and - (CH₂CH₂O)ᵣC(O)NR^{m}(CH₂)ᵣ-; wherein each R^{m} is independently H, C1-C6 alkyl, C3-C8 carbocyclyl, phenyl or benzyl, and each r is independently 1, 2, 3, 4, 5, 6, 7, 8, 9 or 10;
B is 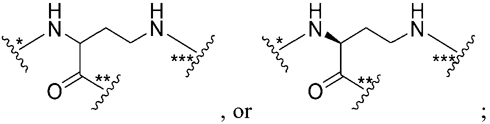 wherein * links to M, ** links to L, and *** links to G;
L is-(AA)ᵢ-(FF)_{f}-, wherein AA is an amino acid or polypeptide, and i is 1, 2, 3, 4, 5, 6, 7, 8, 9, 10, 11, 12, 13, 14, 15, 16, 17, 18, 19 or 20; each FF is independently 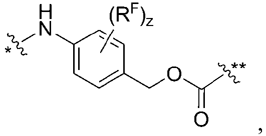 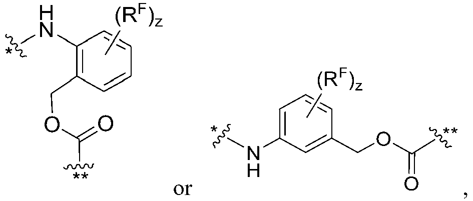 wherein each R^{F} is independently C1-C6 alkyl, C1-C6 alkoxy, -NO₂ or halogen; z is 0, 1, 2, 3 or 4; wherein * links to AA, and ** links to D; f is 1, 2, 3, 4, 5, 6, 7, 8, 9 or 10;
G is 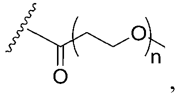 wherein n is an integer from 1-24;
p is 1-10.

3. The method according to claim 2, wherein each AA is independently selected from the following amino acids and peptide sequences: Val-Cit, Val-Lys, Phe-Lys, Lys-Lys, Ala-Lys, Phe-Cit, Leu-Cit, Ile-Cit, Trp, Cit, Phe-Ala, Phe-Phe-Lys, D-Phe-Phe-Lys, Gly-Phe-Lys, Leu-Ala-Leu, Ile-Ala-Leu, Val-Ala-Val, Ala-Leu-Ala-Leu, β-Ala-Leu-Ala-Leu and Gly-Phe-Leu-Gly.

4. The method according to claim 2 or 3, wherein AA is Val-Cit.

5. The method according to any one of claims 2-4, wherein i is 1.

6. The method according to any one of claims 2-5, wherein each FF is independently 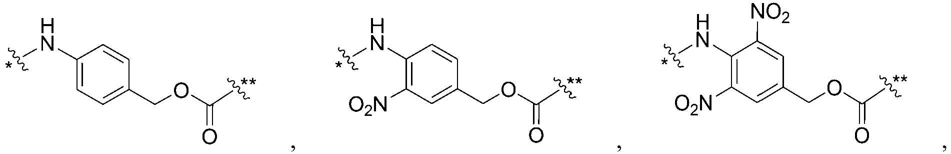 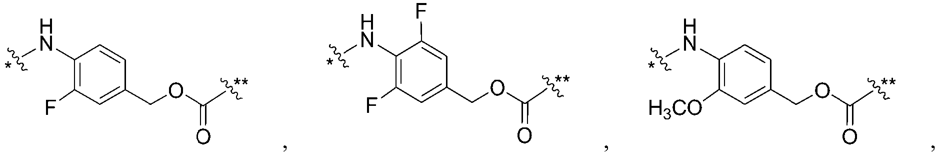 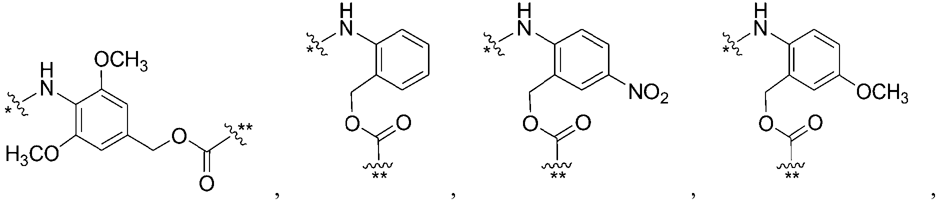 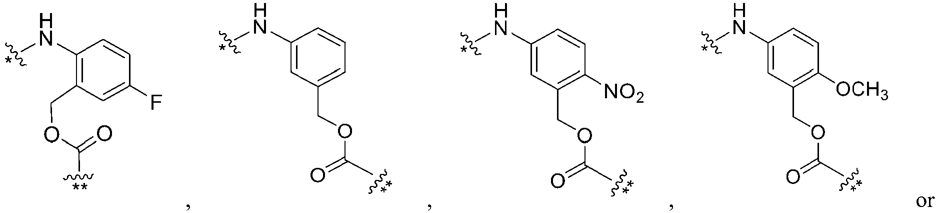 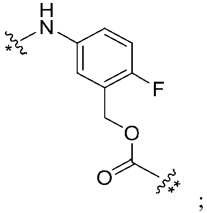 wherein * links to AA, and ** links to D; or FF is 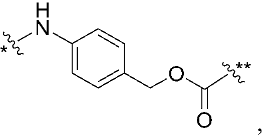 wherein * links to AA, and ** links to D.

7. The method according to any one of claims 2-6, wherein L is 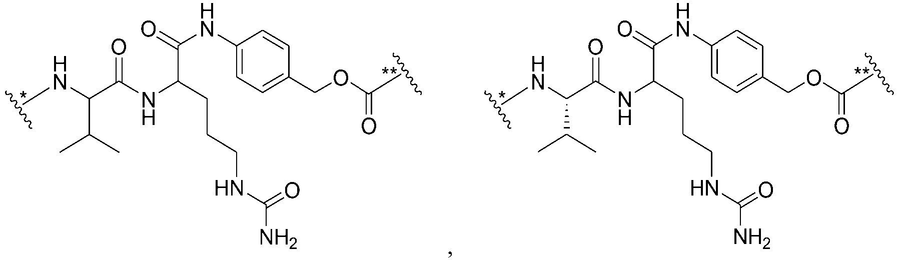 or 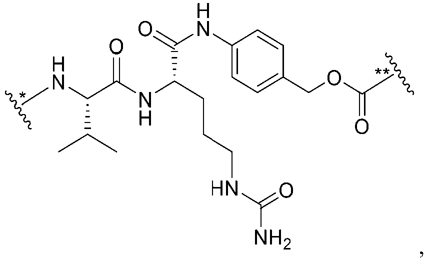 wherein * links to B, and ** links to D.

8. A method for treating and/or preventing cancer, comprising administering to a human patient in need an effective amount of an antibody-drug conjugate by injection; wherein, the antibody-drug conjugate is administered at a dose of 1.7 mg/kg to 2.5 mg/kg, or 100 mg to 250 mg, or 70 mg/m² to 100 mg/m² ,or 75 mg/m² to 100 mg/m² once every 2-4 weeks; the antibody-drug conjugate has a structure of Formula I-1, I-2 or I-2-1, or a stereoisomer thereof, or a pharmaceutically acceptable salt or solvate thereof:
the Formula I-1 is: 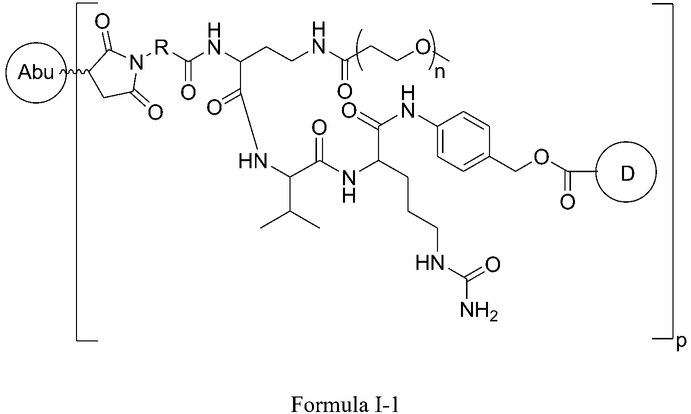
the Formula I-2, I-2-1 is: 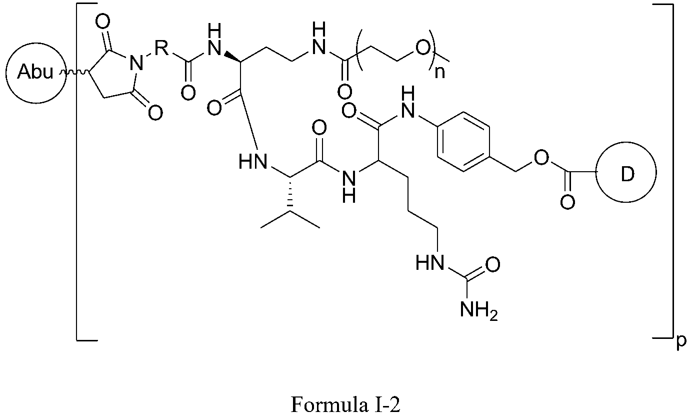 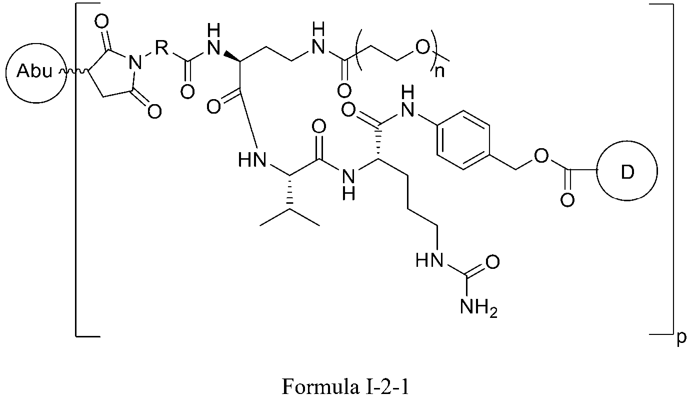 wherein,
Abu is an anti-FRα antibody or an antigen-binding unit thereof;
R is selected from: -(CH₂)ᵣ-, -(CHR^{m})ᵣ-, C3-C8 carbocyclyl, -O-(CH₂)ᵣ-, arylene, -(CH₂)ᵣ-arylene-, - arylene-(CH₂)r-, -(CH₂)ᵣ-(C3-C8 carbocyclyl)-, -(C3-C8 carbocyclyl)-(CH₂)ᵣ-, C3-C8 heterocyclyl, - (CH₂)ᵣ-(C3-C8 heterocyclyl)-, -(C3-C8 heterocyclyl)-(CH₂)ᵣ-, -(CH₂)ᵣC(O)NR^{m}(CH₂)ᵣ-, -(CH₂CH₂O)ᵣ- , -(CH₂CH₂O)ᵣ-CH₂-, -(CH₂)ᵣC(O)NR⁻(CH₂CH₂O),-, -(CH₂)ᵣC(O)NR^{m}(CH₂CH₂O)ᵣ-CH₂-, - (CH₂CH₂O)ᵣC(O)NR^{m}(CH₂CH₂O)ᵣ-, -(CH₂CH₂O)ᵣC(O)NR^{-m}(CH₂CH₂O)ᵣ-CH₂- and - (CH₂CH₂O)ᵣC(O)NR^{m}(CH₂)ᵣ-; wherein each R^{m} is independently H, C1-C6 alkyl, C3-C8 carbocyclyl, phenyl or benzyl, and each r is independently 1, 2, 3, 4, 5, 6, 7, 8, 9 or 10;
D is a drug;
n is an integer from 1 to 24;
p is 1-10.

9. The method according to any one of claims 2-8, wherein R is -(CH₂)ᵣ-.

10. The method according to any one of claims 2-9, wherein r is 1 or 5.

11. The method according to any one of claims 1-10, the antibody-drug conjugate has a structure of Formula I-3, I-4 or I-4-1, or a stereoisomer thereof, or a pharmaceutically acceptable salt or solvate thereof: 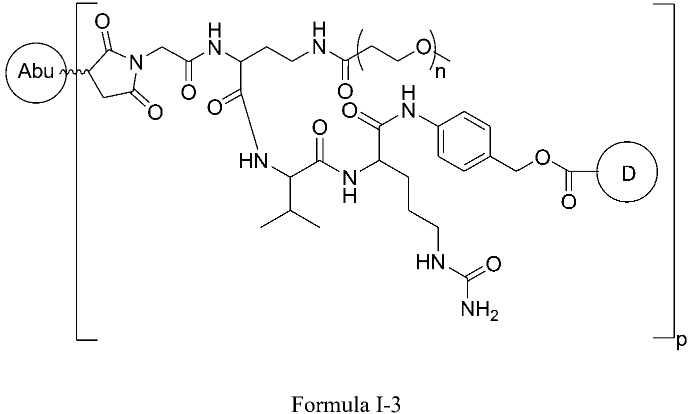 the Formula I-4, I-4-1 is: 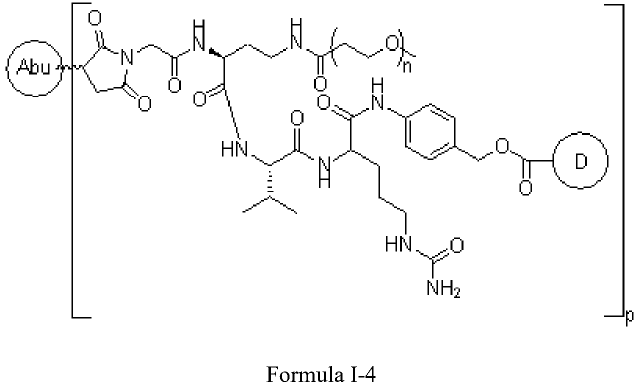 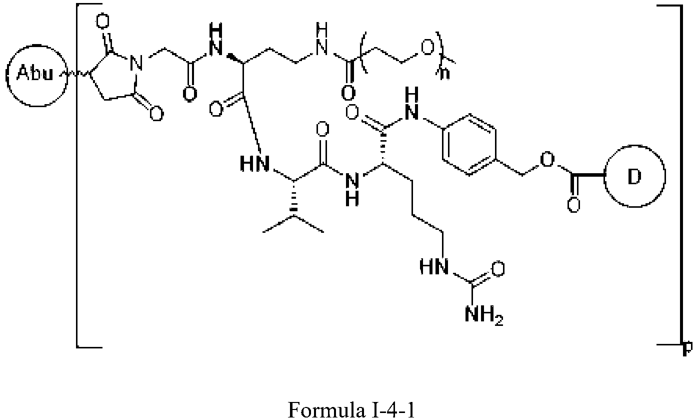 wherein
Abu is an anti-FRα antibody or an antigen-binding unit thereof;
D is a drug;
n is an integer from 1 to 24;
p is 1-10.

12. The method according to any one of claims 2-11, wherein n is an integer from 4-12.

13. The method according to any one of claims 2-12, wherein n is an integer from 4-8.

14. The method according to any one of claims 2-13, wherein n is 4 or 8.

15. The method according to any one of claims 1-14, the antibody-drug conjugate has a structure of Formula I-5, I-5-1, I-6, I-6-1, I-7, I-7-1, I-8, I-8-1, I-9, I-9-1, I-10, I-10-1, I-11 or I-11-1, or a stereoisomer thereof, or a pharmaceutically acceptable salt or solvate thereof:
the Formula I-5, I-5-1 is: 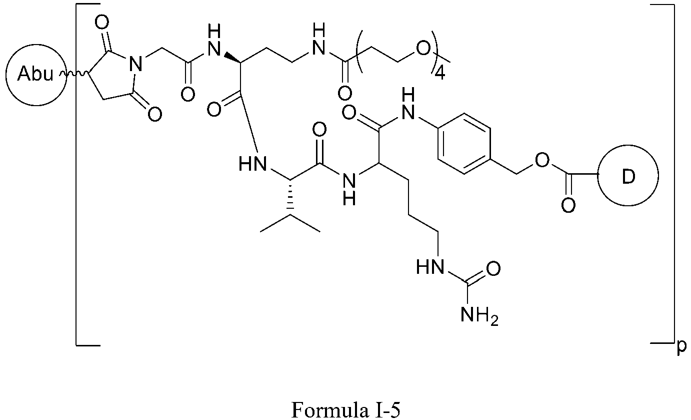 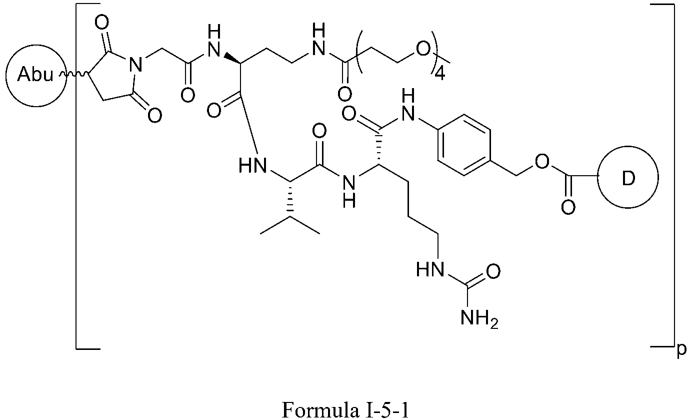
the Formula I-6, I-6-1 is: 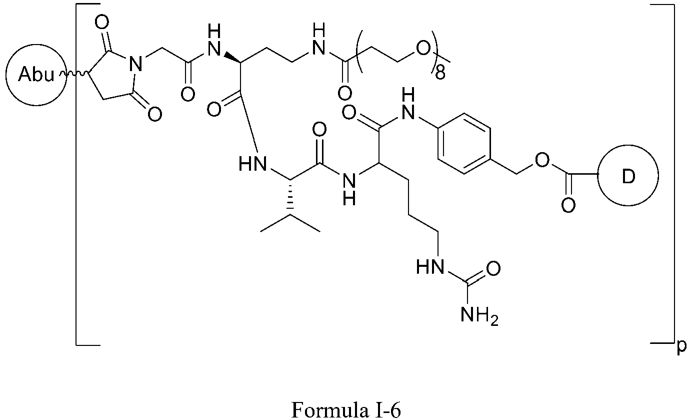 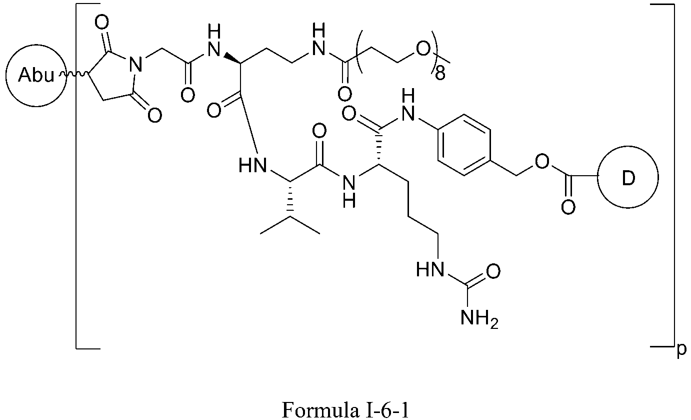
the Formula I-7, I-7-1 is: 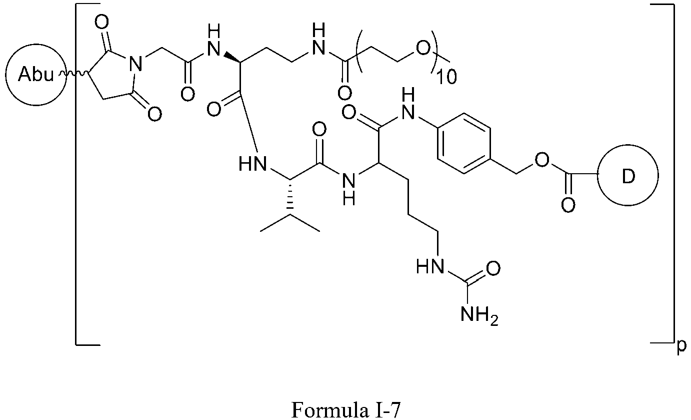 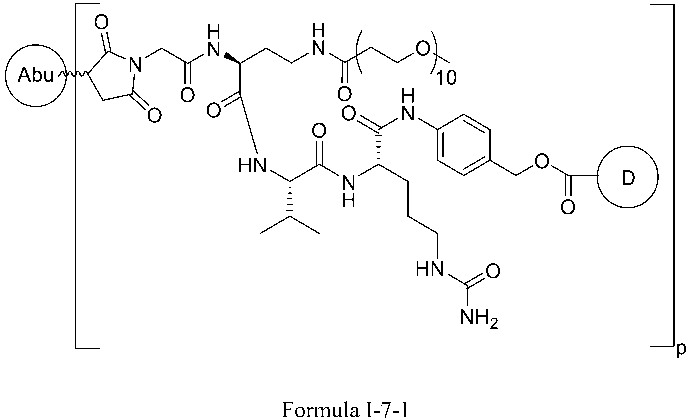
the Formula I-8, I-8-1 is: 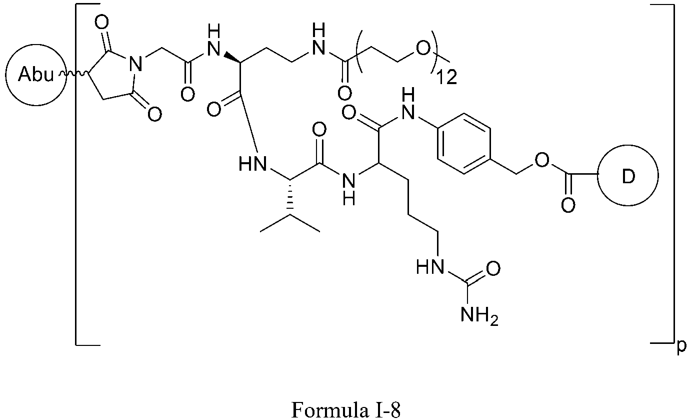 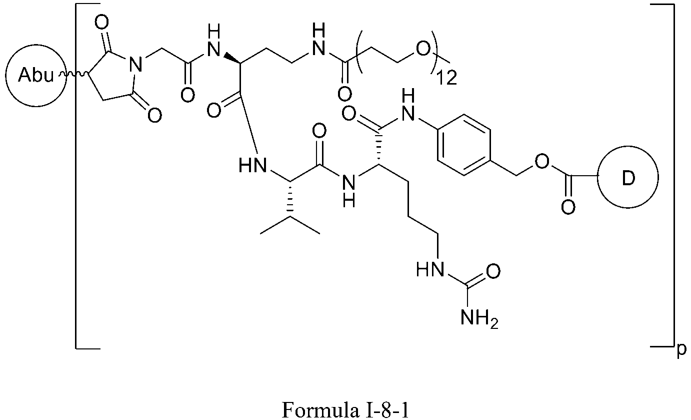
the Formula I-9, I-9-1 is: 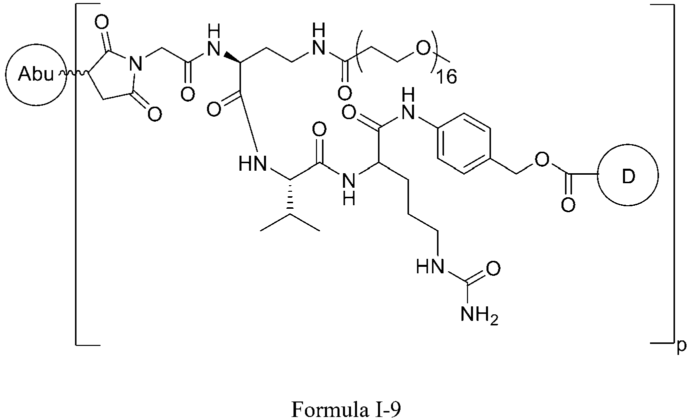 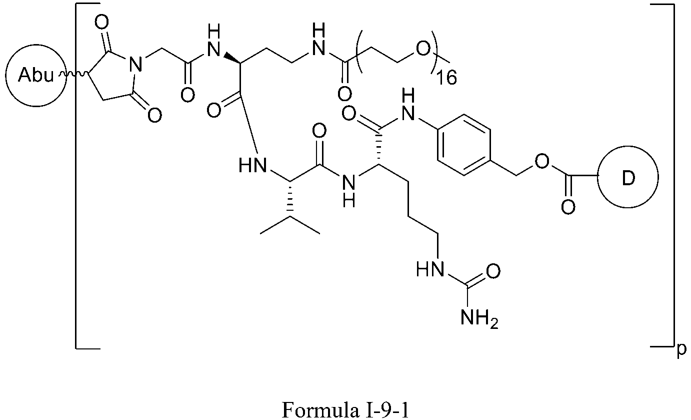
the Formula I-10, I-10-1 is: 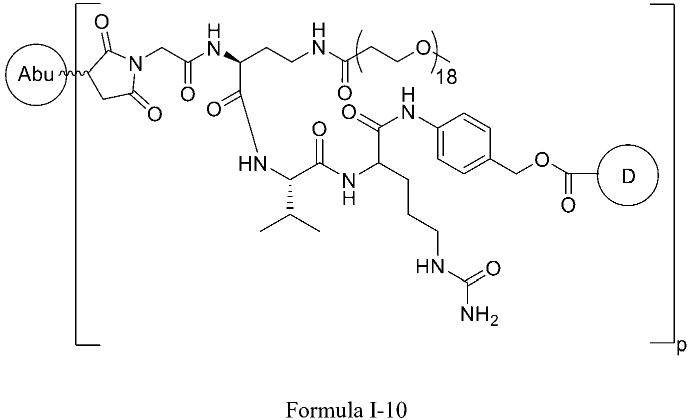 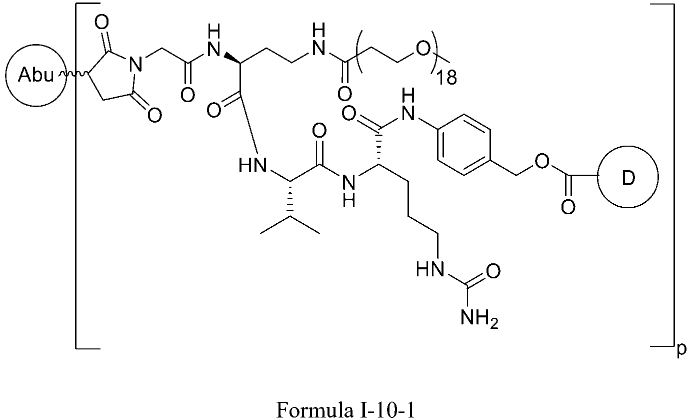
the Formula I-11, I-11-1 is: 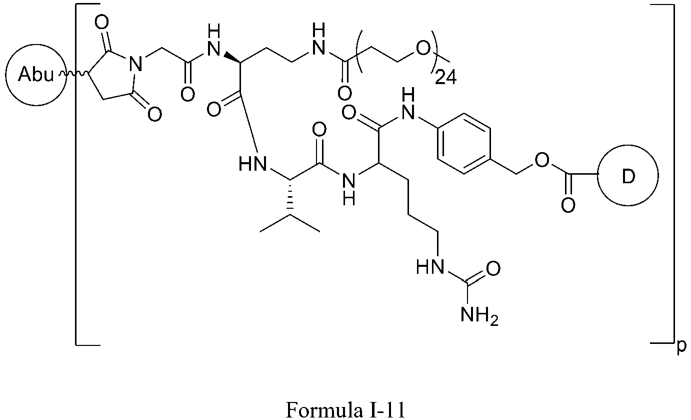 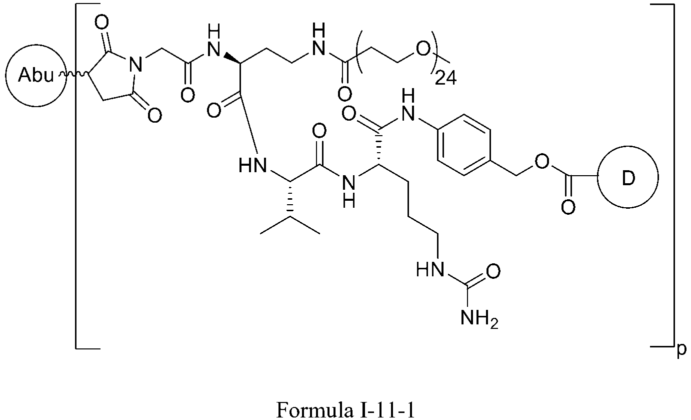 wherein
Abu is an anti-FRα antibody or an antigen-binding unit thereof;
D is a drug;
p is 1-10.

16. The method according to any one of claims 1-15, the drug is an anti-cancer drug, a cytotoxic drug, a cell differentiation factor, a stem cell trophic factor, a steroid drug, a drug for treating autoimmune diseases, an anti-inflammatory drug or a drug for treating infectious diseases; or the drug is an anti-cancer drug; or the drug is a tubulin inhibitor, a DNA damaging agent, or a DNA topoisomerase inhibitor; or the tubulin inhibitor is selected from dolastatin, auristatins and maytansinoids; or the drug is an auristatin, which is selected from MMAE, MMAF or AF; or the drug is a DNA damaging agent, which is selected from a calicheamicin, a duocarmycin, or an anthramycin derivative PBD; or the drug is a DNA topoisomerase inhibitor or a salt thereof, which is selected from irinotecan, irinotecan hydrochloride, an exatecan derivative, camptothecin, 9-aminocamptothecin, 9-nitrocamptothecin, 10-hydroxycamptothecin, 9-chloro-10-hydroxycamptothecin, the camptothecin derivative SN-38, 22-hydroxyacuminatine, topotecan, lurtotecan, belotecan, exatecan, homosilatecan, 6,8-dibromo-2-methyl-3-[2-(D-xylopyranosylamino)phenyl]-4(3*H*)-quinazolinone, 2-cyano-3-(3,4-dihydroxyphenyl)-N-(phenylmethyl)-(2*E*)-2-propenamide, 2-cyano-3-(3,4-dihydroxyphenyl)-*N*-(3-hydroxyphenylpropyl)-(*E*)-2-propenamide, 12-β-D-glucopyranosyl-12,13-dihydro-2,10-dihydroxy-6-[[2-hydroxy-1-(hydroxymethyl)ethyl]amino]-5*H*-indolo[2,3-*a*]pyrrolo[3,4-*c*]carbazole-5,7(6*H*)-dione, *N*-[2-(dimethylamino)ethyl]-4-acridinecarboxamide dihydrochloride, or *N*-[2-(dimethylamino)ethyl]-4-acridinecarboxamide; or the DNA topoisomerase inhibitor is camptothecin, 10-hydroxycamptothecin, topotecan, belotecan, irinotecan, 22-hydroxyacuminatine, or exatecan, or a salt thereof.

17. The method according to any one of claims 1-15, the drug is 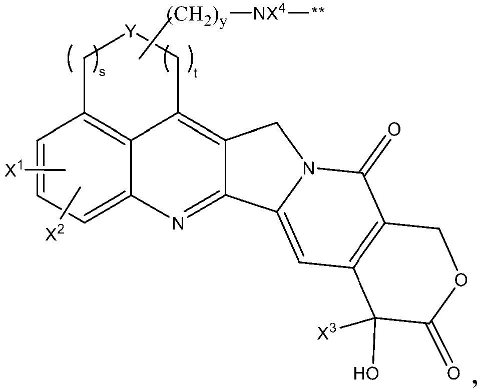 wherein
X¹ and X² are each independently:
H,
hydroxy,
C1-C6 alkyl,
C1-C6 alkyl substituted with one or more hydroxy, halogen, nitro or cyano groups,
C2-C6 alkenyl,
C2-C6 alkynyl,
C1-C6 alkoxy,
C1-C6 aminoalkoxy,
halogen,
nitro,
cyano,
thiol,
alkylthio,
amino, amino substituted with an amino-protecting group, C1-C6 aminoalkyl optionally substituted at the amino moiety with an amino-protecting group or C1-C6 alkyl,
C1-C6 aminoalkylamino optionally substituted at the amino moiety with an amino-protecting group or C1-C6 alkyl,
C1-C6 alkyl linking to a heterocyclic ring, wherein the heterocyclic ring is optionally substituted with one or more C1-C6 alkyl, C1-C6 alkoxy, amino, halogen, nitro or cyano groups,
C1-C6 alkylamino linking to a heterocyclic ring, wherein the heterocyclic ring is optionally substituted with C1-C6 alkyl or C1-C6 alkoxy, and the amino is optionally substituted with an amino-protecting group, halogen, nitro, cyano or protecting group,
amino-substituted heterocyclyl, which is optionally substituted at a nitrogen atom of the heterocyclyl moiety or at the amino moiety with a protecting group or one or more C1-C6 alkyl groups,
heterocyclylamino, which is optionally substituted at a nitrogen atom of the heterocyclic moiety or at the amino moiety with a protecting group or C1-C6 alkyl,
carbamoyl optionally substituted with a carbamoyl-protecting group or C1-C6 alkyl,
morpholin-1-yl, or
piperidin-1-yl;
X³ is C1-C6 alkyl;
X⁴ is H, -(CH₂)_{q}-CH₃, -(CHRⁿ)_{q}-CH₃, C3-C8 carbocyclyl, -O-(CH₂)_{q}-CH₃, arylene-CH₃, -(CH₂)_{q}-aryle ne-CH₃, -arylene-(CH₂)_{q}-CH₃, -(CH₂)_{q}-(C3-C8 carbocyclyl)-CH₃, -(C3-C8 carbocyclyl)-(CH₂)_{q}-CH₃, C3-C8 heterocyclyl, -(CH₂)_{q}-(C3-C8 heterocyclyl)-CH₃, -(C3-C8 heterocyclyl)-(CH₂)_{q}-CH₃, -(CH₂)_{q} C(O)NRⁿ(CH₂)_{q}-CH₃, -(CH₂CH₂O)_{q}-CH₃, -(CH₂CH₂O)_{q}-CH₂-CH₃, -(CHz)_{q}C(O)NRⁿ(CH₂CH₂O)_{q}-CH₃, -(CH₂)_{q}C(O)NRⁿ(CH₂CH₂O)_{q}-CH₂-CH₃, -(CH₂CH₂O)_{q}C(O)NRⁿ(CH₂CH₂O)_{q}-CH₃, -(CH₂CH₂O)_{q}C(O) NRⁿ(CH₂CH₂O)_{q}-CH₂-CH₃ or -(CH₂CH₂O)_{q}C(O)NRⁿ(CH₂)_{q}-CH₃; wherein each Rⁿ is independently H , C1-C6 alkyl, C3-C8 carbocyclyl, phenyl or benzyl, and each q is independently 1, 2, 3, 4, 5, 6, 7, 8, 9 or 10; or X⁴ is H or C1-C6 alkyl;
** is an attachment point;
y is 0, 1 or 2;
Y is O, S or CR¹R², wherein R¹ and R² are each independently H or C1-C6 alkyl;
s and t are each independently 0, 1 or 2, but not both 0.

18. The method according to any one of claims 1-15, 17, the drug is 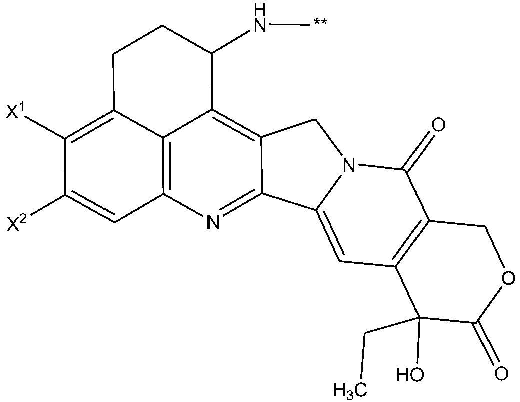 or 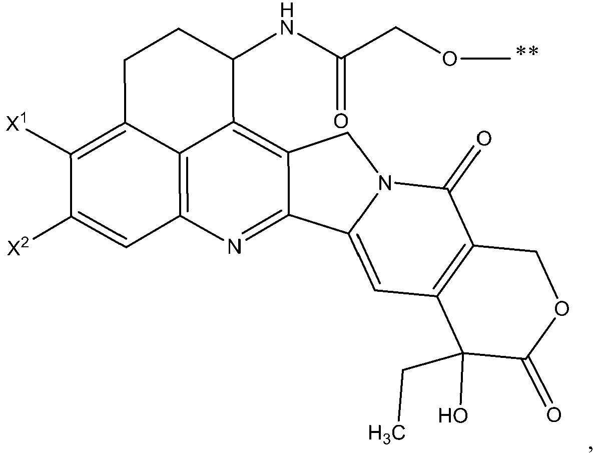 wherein X¹ and X² are each independently C1-C6 alkyl, halogen, or -OH; or the C1-C6 alkyl is -CH₃, or the halogen is F; ** is an attachment point.

19. The method according to any one of claims 1-15, 17-18, the drug is 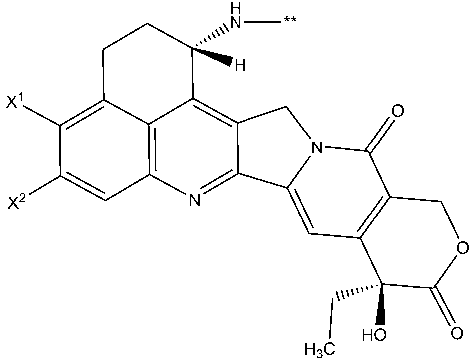 or 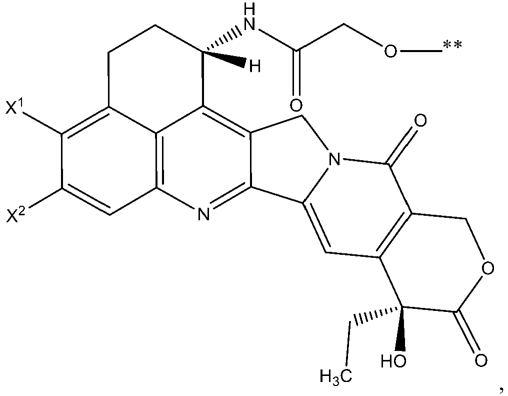 wherein X¹ and X² are each independently C1-C6 alkyl, halogen, or -OH; or the C1-C6 alkyl is -CH₃, or the halogen is F; ** is an attachment point.

20. A method for treating and/or preventing cancer, comprising administering to a human patient in need an effective amount of an antibody-drug conjugate by injection; wherein, the antibody-drug conjugate is administered at a dose of 1.7 mg/kg to 2.5 mg/kg, or 100 mg to 250 mg, or 70 mg/m² to 100 mg/m² ,or 75 mg/m² to 100 mg/m² once every 2-4 weeks; the antibody-drug conjugate has a structure of Formula I-26 or I-27, or a stereoisomer thereof, or a pharmaceutically acceptable salt or solvate thereof: 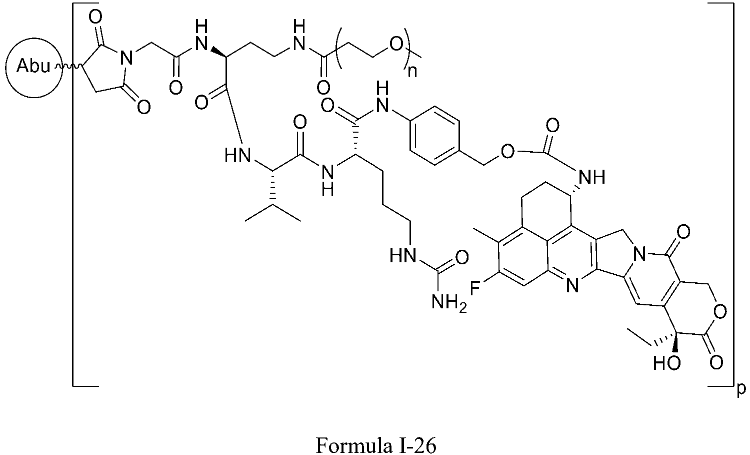 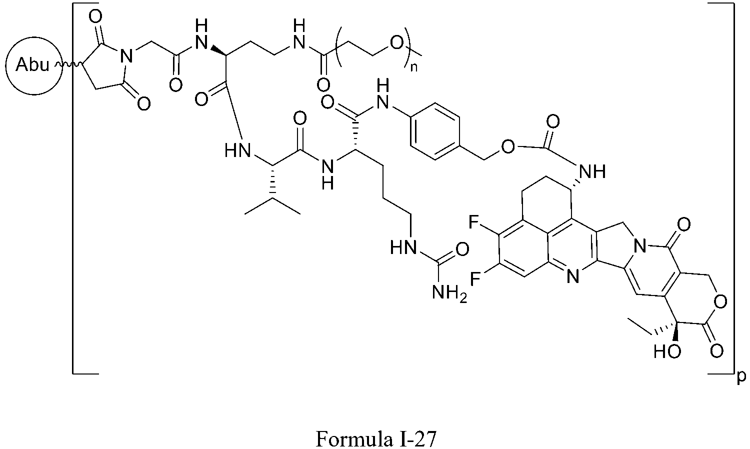 wherein
Abu is an anti-FRα antibody or an antigen-binding unit thereof;
n is an integer from 1 to 24;
p is 1-10.

21. The method according to claim 20, wherein n is an integer from 4-12.

22. The method according to claim 20 or 21, wherein n is an integer from 4-8.

23. The method according to any one of claims 20-22, wherein n is 4 or 8.

24. A method for treating and/or preventing cancer, comprising administering to a human patient in need an effective amount of an antibody-drug conjugate by injection; wherein, the antibody-drug conjugate is administered at a dose of 1.7 mg/kg to 2.5 mg/kg, or 100 mg to 250 mg, or 70 mg/m² to 100 mg/m² ,or 75 mg/m² to 100 mg/m² once every 2-4 weeks; the antibody-drug conjugate has a structure of I-12, I-12-1, I-13, I-13-1, I-14, I-14-1, 1-15, I-15-1, I-16, I-16-1, I-17, I-17-1, I-18, I-18-1, I-19, I-19-1, I-20, I-20-1, I-21, I-21-1, I-22, I-22-1, I-23, I-23-1, I-24, I-24-1, I-25 or I-25-1, or a stereoisomer thereof, or a pharmaceutically acceptable salt or solvate thereof, wherein the Formula I-12, I-12-1, I-13, I-13-1, I-14, I-14-1, I-15, I-15-1, I-16, I-16-1, I-17, I-17-1, I-18, 1-18-1, I-19, I-19-1, I-20, I-20-1, I-21, I-21-1, I-22, I-22-1, I-23, I-23-1, I-24, I-24-1, I-25 or I-25-1 is: 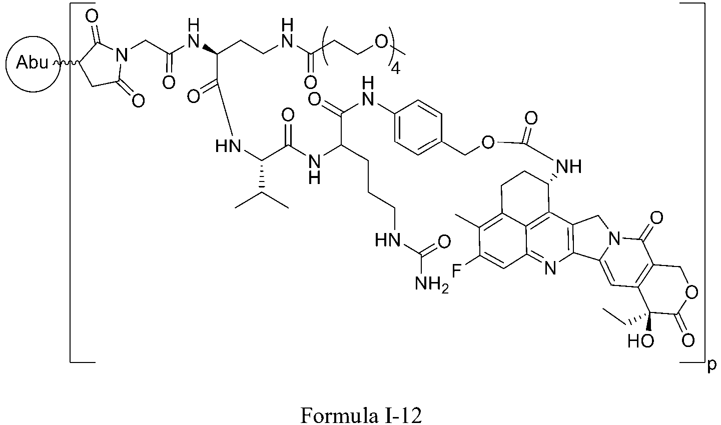 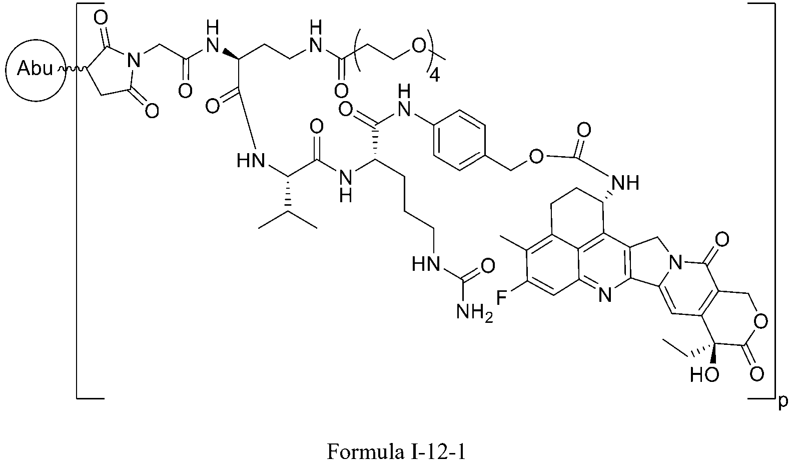 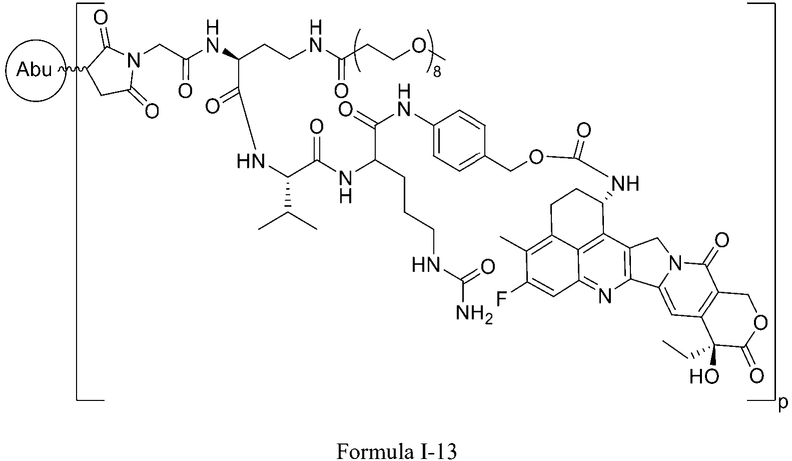 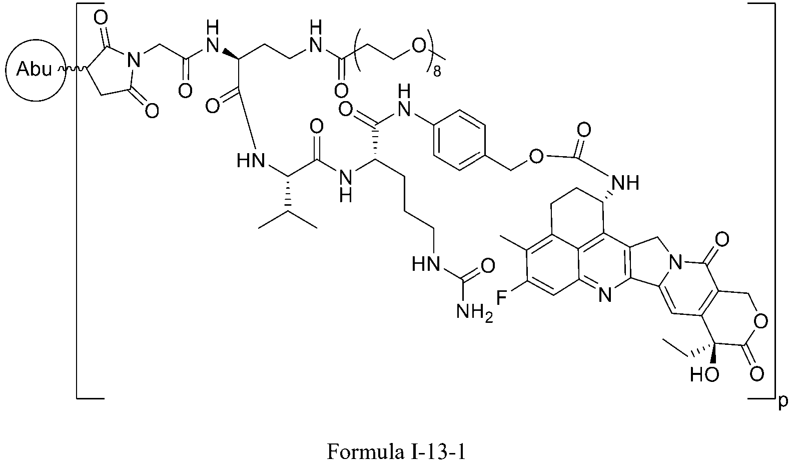 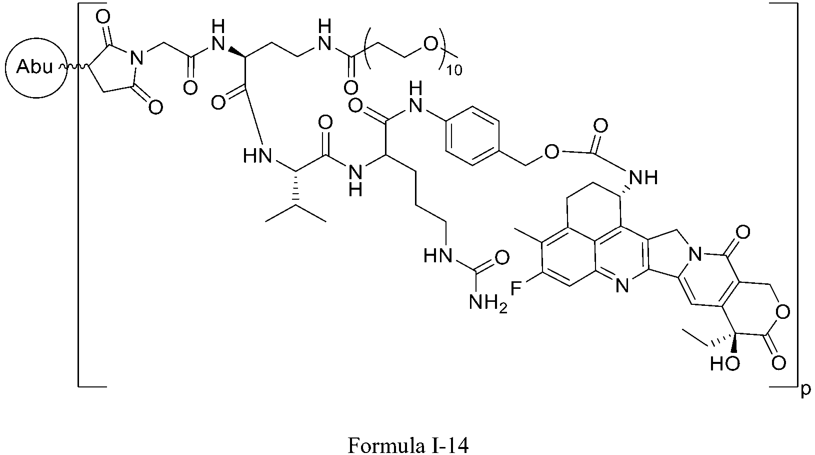 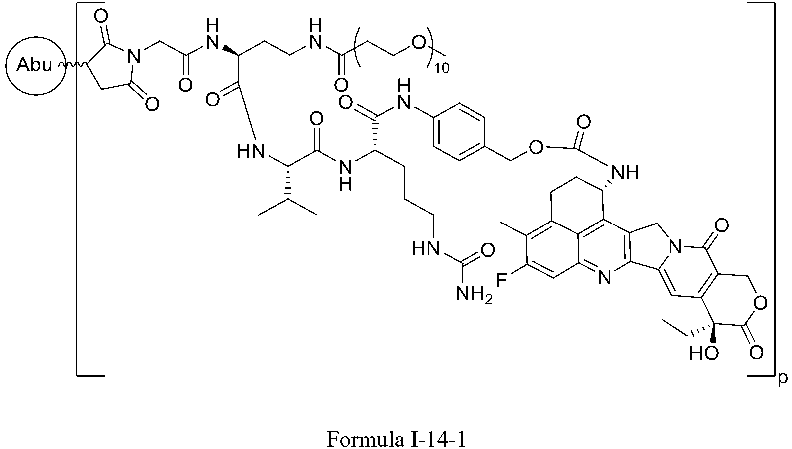 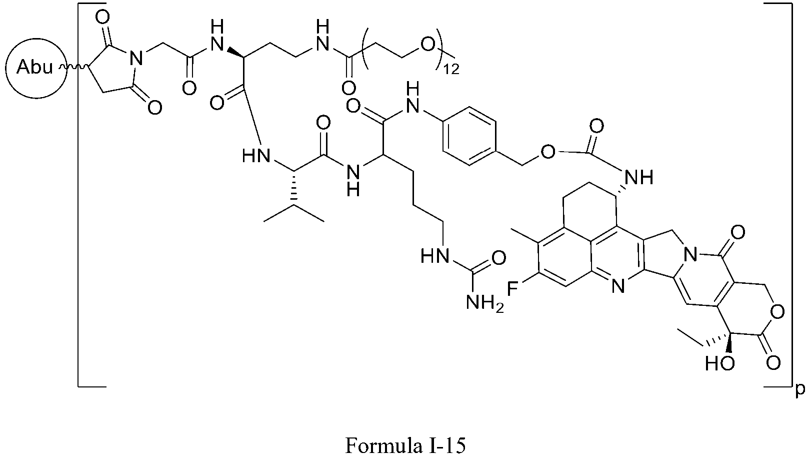 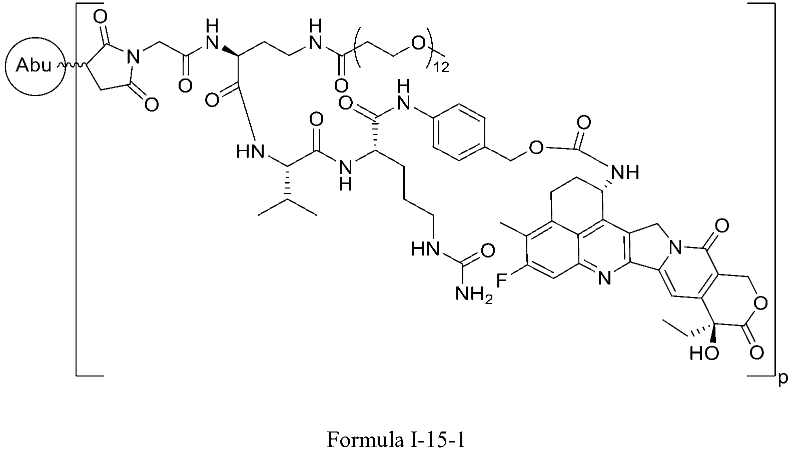 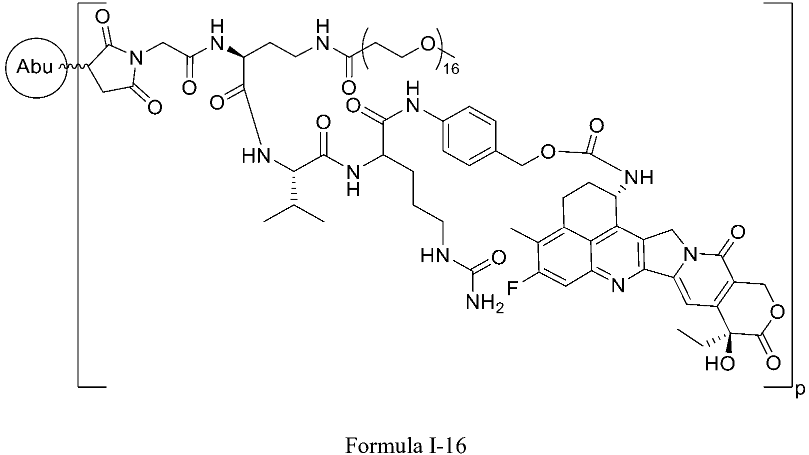 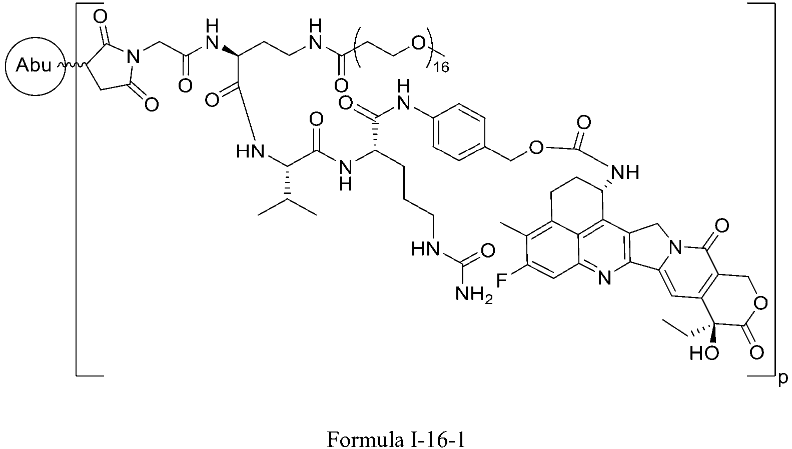 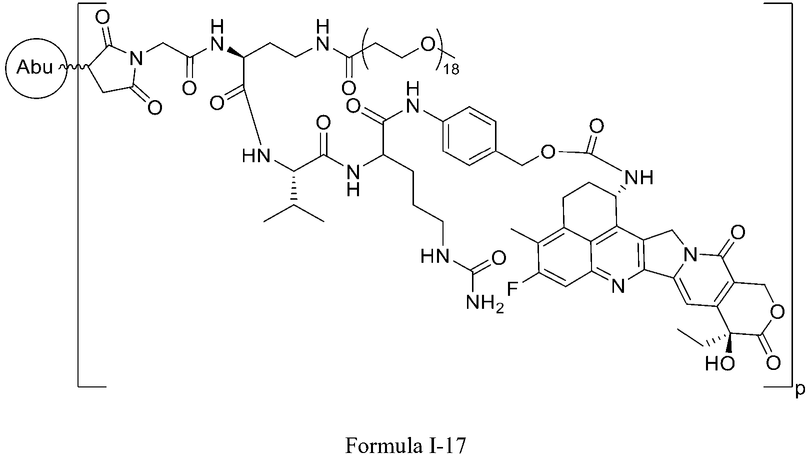 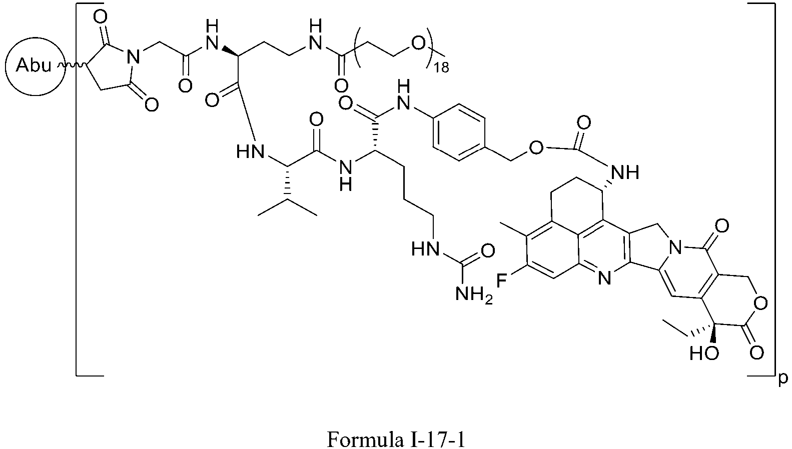 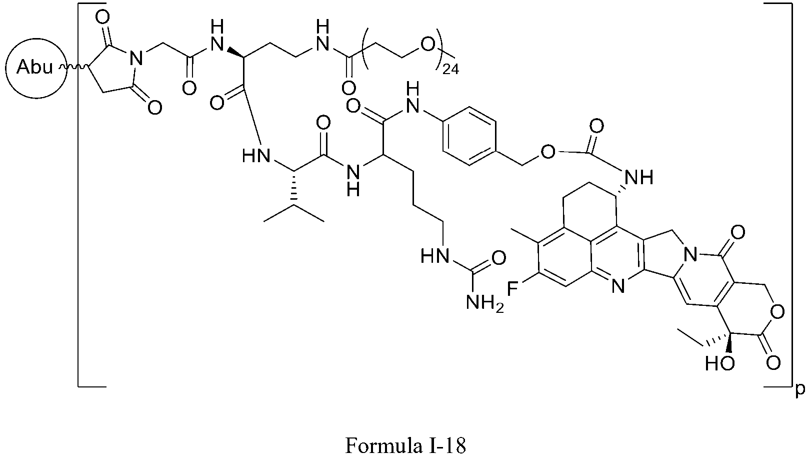 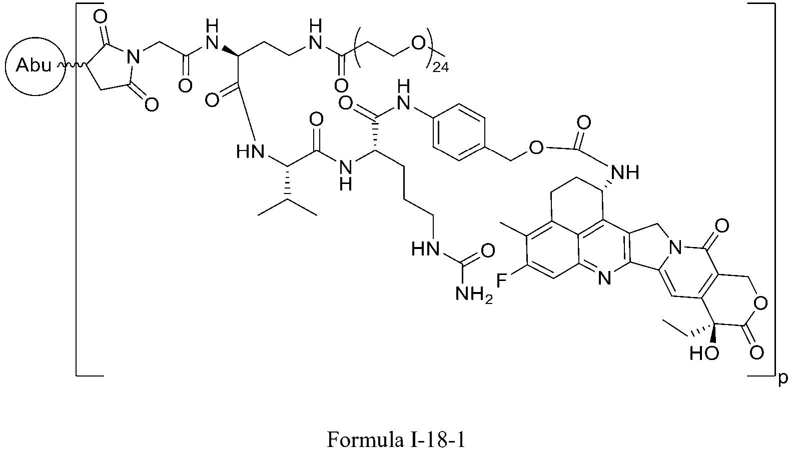 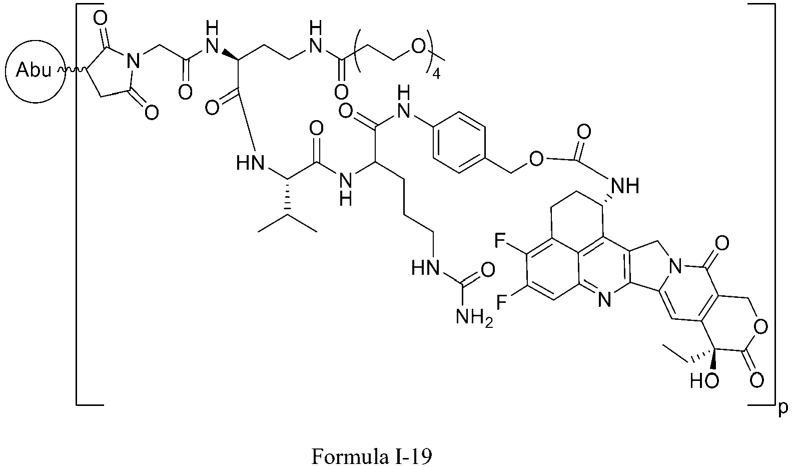 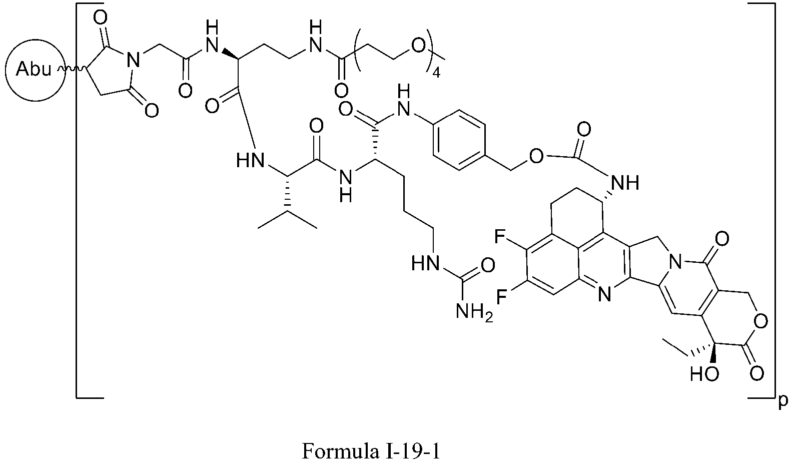 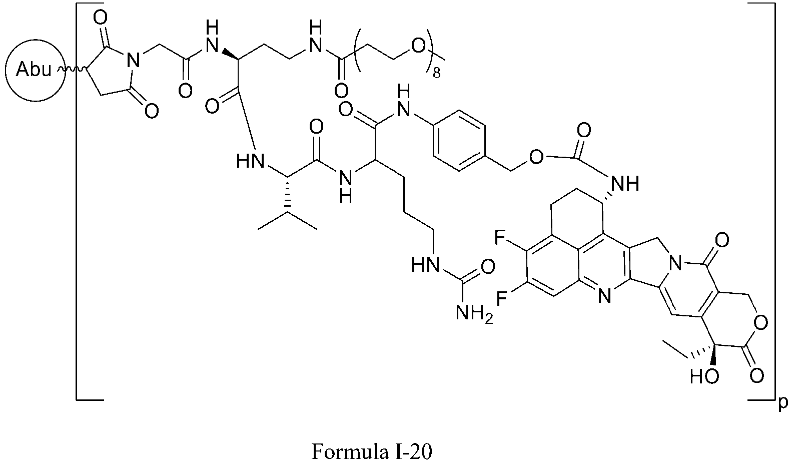 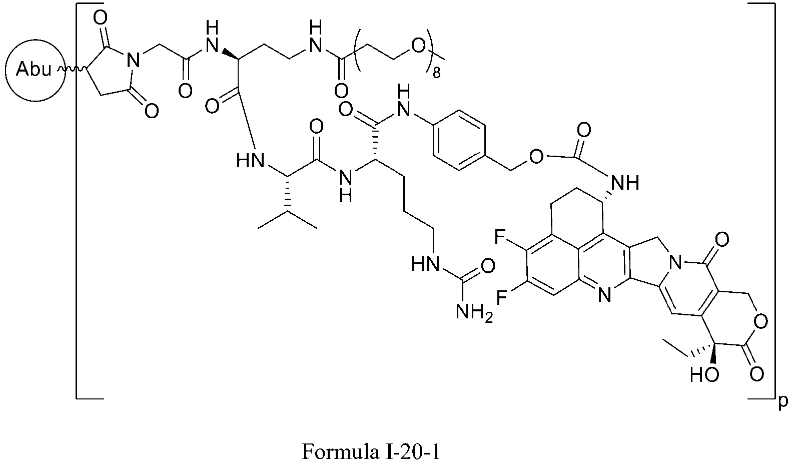 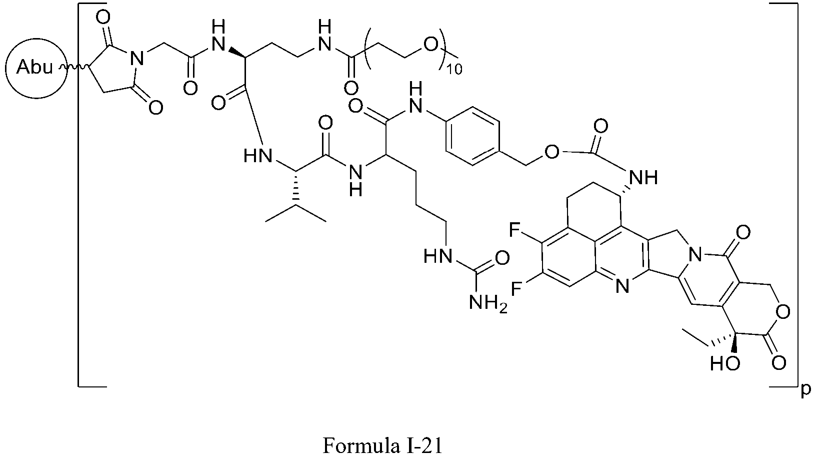 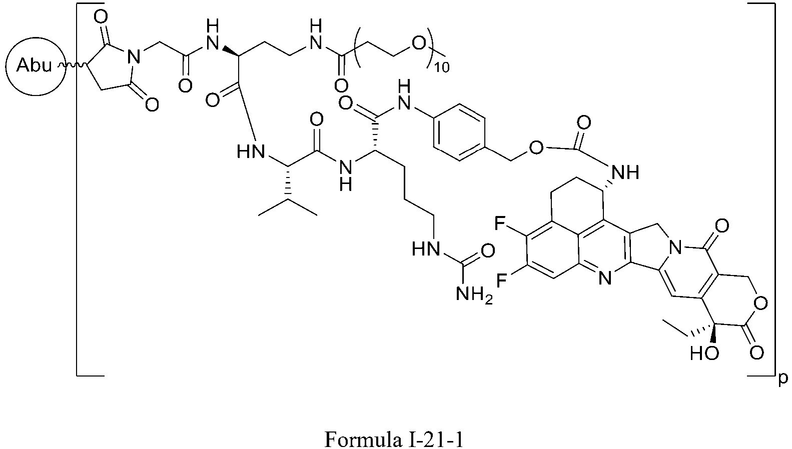 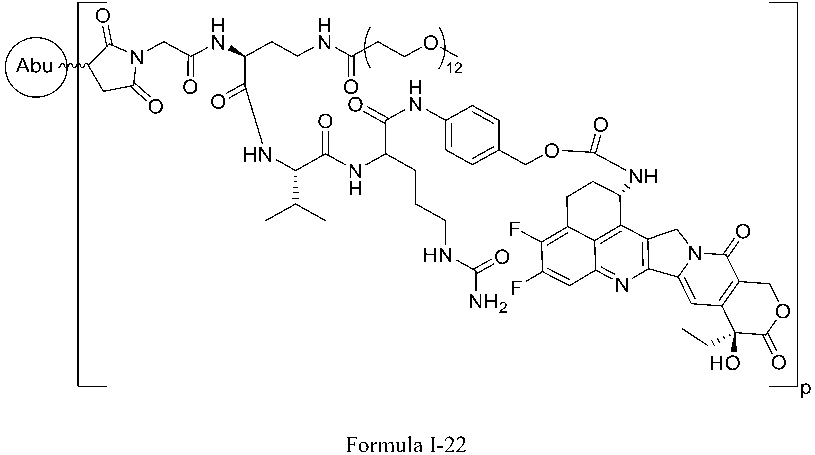 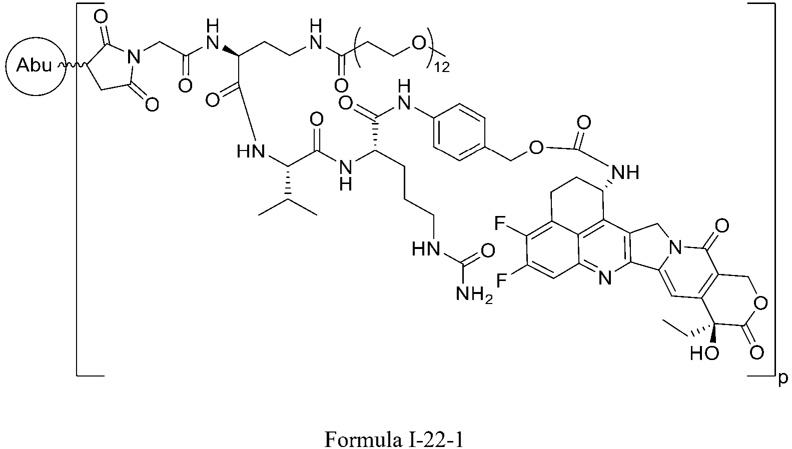 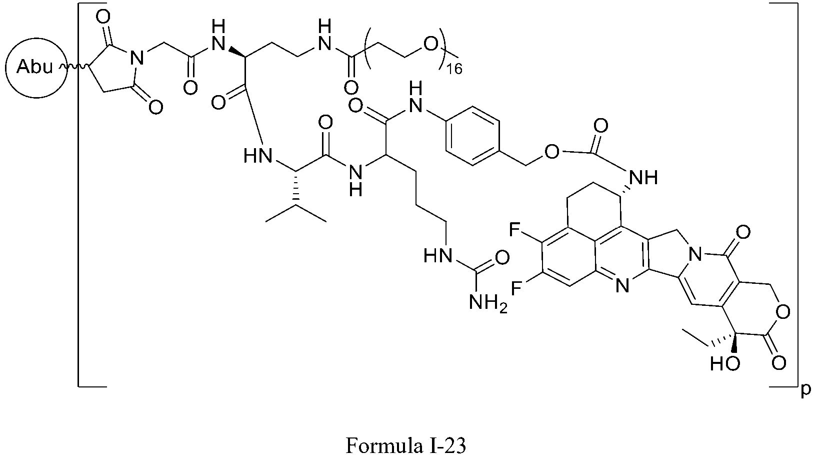 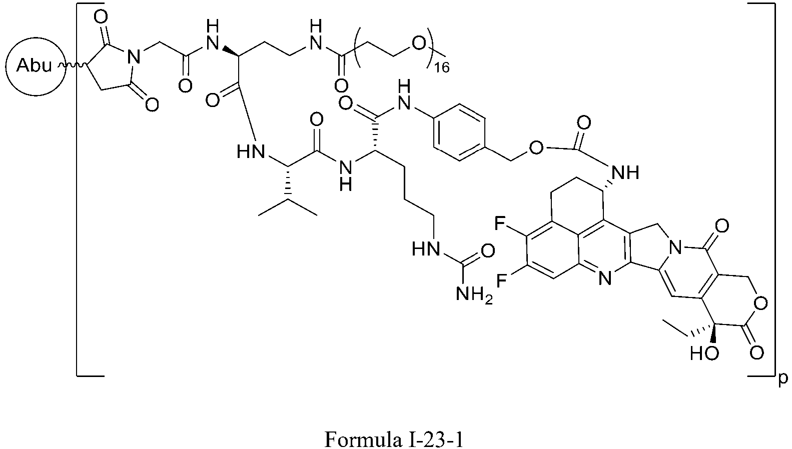 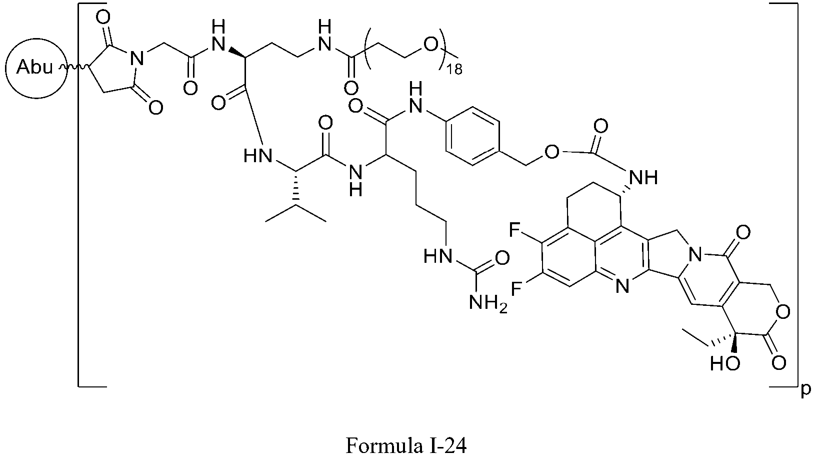 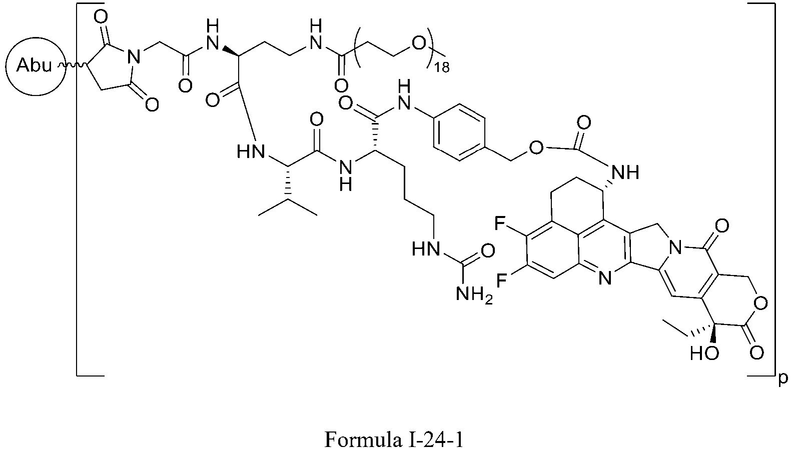 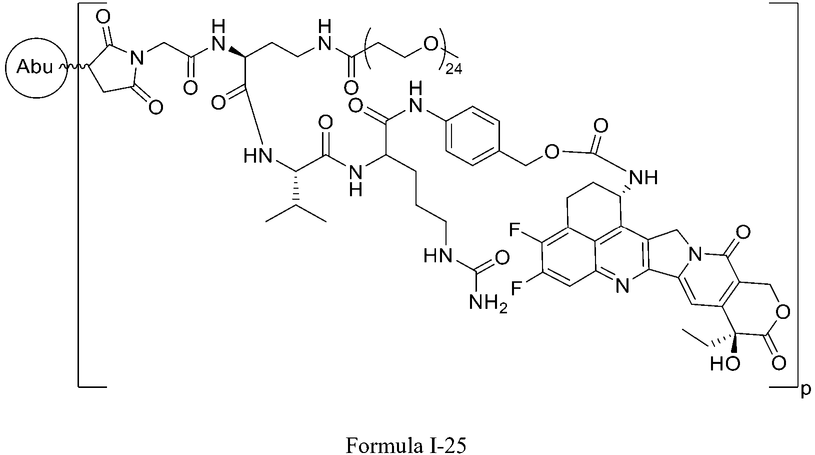 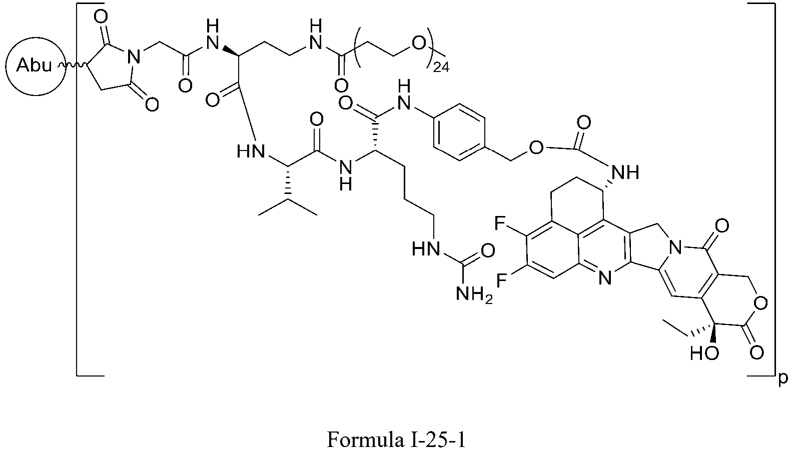 wherein
Abu is an anti-FRα antibody or an antigen-binding unit thereof;
p is 1-10.

25. The method according to any one of claims 2-24, wherein p is 2-8.

26. The method according to any one of claims 2-25, wherein p is 4-8.

27. The method according to any one of claims 2-26, wherein p is 6-8.

28. The method according to any one of claims 2-27, wherein p is 7-8.

29. The method according to any one of claims 2-28, wherein p is about 7.

30. The method according to any one of claims 2-28, wherein p is about 7.4.

31. The method according to any one of claims 2-28, wherein p is about 8.

32. The method according to any one of claims 1-31, the anti-FRα antibody or the antigen-binding unit thereof comprises a VH CDR1 set forth in SEQ ID NO: 1, a VH CDR2 set forth in SEQ ID NO: 2, a VH CDR3 set forth in SEQ ID NO: 3, a VL CDR1 set forth in SEQ ID NO: 4, a VL CDR2 set forth in SEQ ID NO: 5 and a VL CDR3 set forth in SEQ ID NO: 6; optionally,
comprises one or more of (g)-(n):
(g) a VH FR1 comprising an amino acid sequence set forth in SEQ ID NO: 15, or an amino acid sequence having at least 90% sequence identity compared with the amino acid sequence set forth in SEQ ID NO: 15, or an amino acid sequence having substitutions, deletions or insertions at one or more sites compared with the amino acid sequence set forth in SEQ ID NO: 15;
(h) a VH FR2 comprising an amino acid sequence set forth in SEQ ID NO: 16, or an amino acid sequence having at least 90% sequence identity compared with the amino acid sequence set forth in SEQ ID NO: 16, or an amino acid sequence having substitutions, deletions or insertions at one or more sites compared with the amino acid sequence set forth in SEQ ID NO: 16;
(i) a VH FR3 comprising an amino acid sequence set forth in SEQ ID NO: 17, or an amino acid sequence having at least 90% sequence identity compared with the amino acid sequence set forth in SEQ ID NO: 17, or an amino acid sequence having substitutions, deletions or insertions at one or more sites compared with the amino acid sequence set forth in SEQ ID NO: 17;
(j) a VH FR4 comprising an amino acid sequence set forth in SEQ ID NO: 18, or an amino acid sequence having at least 90% sequence identity compared with the amino acid sequence set forth in SEQ ID NO: 18, or an amino acid sequence having substitutions, deletions or insertions at one or more sites compared with the amino acid sequence set forth in SEQ ID NO: 18;
(k) a VL FR1 comprising an amino acid sequence set forth in SEQ ID NO: 19, or an amino acid sequence having at least 90% sequence identity compared with the amino acid sequence set forth in SEQ ID NO: 19, or an amino acid sequence having substitutions, deletions or insertions at one or more sites compared with the amino acid sequence set forth in SEQ ID NO: 19;
(l) a VL FR2 comprising an amino acid sequence set forth in SEQ ID NO: 20, or an amino acid sequence having at least 90% sequence identity compared with the amino acid sequence set forth in SEQ ID NO: 20, or an amino acid sequence having substitutions, deletions or insertions at one or more sites compared with the amino acid sequence set forth in SEQ ID NO: 20;
(m) a VL FR3 comprising an amino acid sequence set forth in SEQ ID NO: 21, or an amino acid sequence having at least 90% sequence identity compared with the amino acid sequence set forth in SEQ ID NO: 21, or an amino acid sequence having substitutions, deletions or insertions at one or more sites compared with the amino acid sequence set forth in SEQ ID NO: 21; and
(n) a VL FR4 comprising an amino acid sequence set forth in SEQ ID NO: 22, or an amino acid sequence having at least 90% sequence identity compared with the amino acid sequence set forth in SEQ ID NO: 22, or an amino acid sequence having substitutions, deletions or insertions at one or more sites compared with the amino acid sequence set forth in SEQ ID NO: 22.

33. The method according to any one of claims 1-32, the anti-FRα antibody or the antigen-binding unit thereof comprises a heavy chain variable region and a light chain variable region, wherein:
the heavy chain variable region comprises an amino acid sequence set forth in SEQ ID NO: 7, or an amino acid sequence having at least 90% sequence identity compared with the amino acid sequence set forth in SEQ ID NO: 7, or an amino acid sequence having substitutions, deletions or insertions at one or more sites compared with the amino acid sequence set forth in SEQ ID NO: 7; and/or
the light chain variable region comprises an amino acid sequence set forth in SEQ ID NO: 8, or an amino acid sequence having at least 90% sequence identity compared with the amino acid sequence set forth in SEQ ID NO: 8, or an amino acid sequence having substitutions, deletions or insertions at one or more sites compared with the amino acid sequence set forth in SEQ ID NO: 8.

34. The method according to any one of claims 1-33, the anti-FRα antibody or the antigen-binding unit thereof comprises a heavy chain and a light chain, wherein:
the heavy chain comprises an amino acid sequence set forth in SEQ ID NO: 11, or an amino acid sequence having at least 90% sequence identity compared with the amino acid sequence set forth in SEQ ID NO: 11, or an amino acid sequence having substitutions, deletions or insertions at one or more sites compared with the amino acid sequence set forth in SEQ ID NO: 11; and/or
the light chain comprises an amino acid sequence set forth in SEQ ID NO: 13, or an amino acid sequence having at least 90% sequence identity compared with the amino acid sequence set forth in SEQ ID NO: 13, or an amino acid sequence having substitutions, deletions or insertions at one or more sites compared with the amino acid sequence set forth in SEQ ID NO: 13.

35. The method according to any one of claims 1-34, the patient has an FRα expression of ≥1%; or the patient has an FRα TPS of ≥1%; or the patient has an FRα expression of >25%; or the patient has an FRα TPS of >25%; or the patient has an FRα expression of <50%; or the patient has an FRα TPS of <50%; or the patient has an FRα expression of ≥50%; or the patient has an FRα TPS of ≥50%; or the patient has an FRα expression of ≥75%; or the patient has an FRα TPS of ≥75%.

36. The method according to any one of claims 1-35, wherein the antibody-drug conjugate is administered at a dose of 1.7 mg/kg to 2.5 mg/kg.

37. The method according to any one of claims 1-35, wherein the antibody-drug conjugate is administered at a dose of 100 mg to 250 mg.

38. The method according to any one of claims 1-35, wherein the antibody-drug conjugate is administered at a dose of 70 mg/m²-100 mg/m².

39. The method according to any one of claims 1-35, wherein the antibody-drug conjugate is administered at a dose of 75 mg/m²-100 mg/m².

40. The method according to any one of claims 1-39, wherein the antibody-drug conjugate is administered once every 2 weeks.

41. The method according to any one of claims 1-39, wherein the antibody-drug conjugate is administered once every 3 weeks.

42. The method according to any one of claims 1-39, wherein the antibody-drug conjugate is administered once every 4 weeks.

43. A method for treating cancer, which comprises administering to a patient with an FRα TPS of >25% an effective amount of an antibody-drug conjugate, the antibody-drug conjugate has a structure of Formula I-13-1, or a stereoisomer thereof, or a pharmaceutically acceptable salt or solvate thereof: 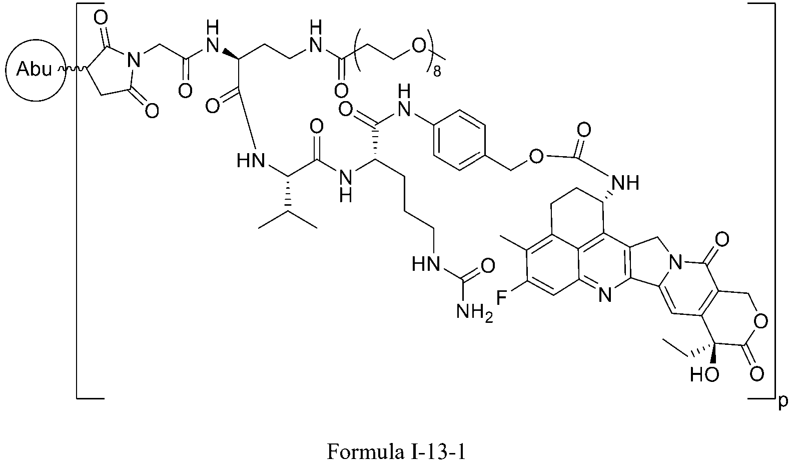 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13;
p is 7-8; or p is 7.4; or p is 7; or p is 8.

44. The method according to claim 43, the antibody-drug conjugate is administered at a dose of 0.5 mg/kg to 5 mg/kg, or 10 mg to 300 mg, or 5 mg/m² to 150 mg/m², at a frequency of once per day to once every 7 weeks.

45. A method for treating cancer, which comprises administering to a patient with an FRα TPS of>25% an effective amount of an antibody-drug conjugate, the antibody-drug conjugate is administered at a dose of 0.5 mg/kg to 5 mg/kg, or 10 mg to 300 mg, or 5 mg/m² to 150 mg/m², at a frequency of once per day to once every 7 weeks.

46. The method according to claim 45, the antibody-drug conjugate has a structure of Formula I-13-1, or a stereoisomer thereof, or a pharmaceutically acceptable salt or solvate thereof: 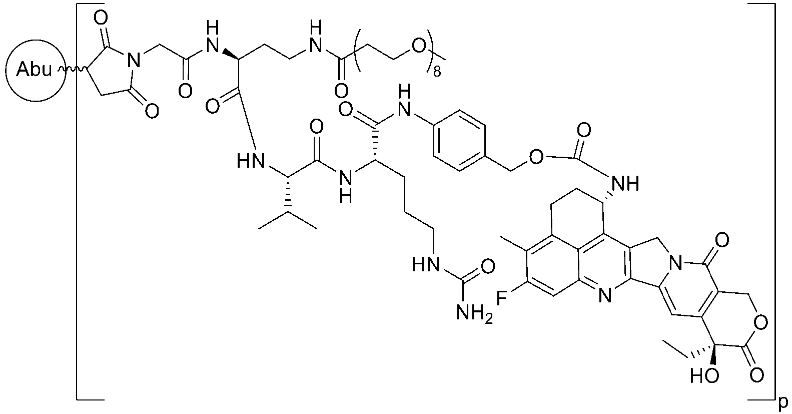 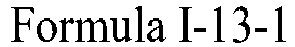 wherein
Abu is an anti-FRα antibody, the heavy chain of the anti-FRα antibody comprises an amino acid sequence set forth in SEQ ID NO: 11, and the light chain of the anti-FRα antibody comprises an amino acid sequence set forth in SEQ ID NO: 13,
p is 1-10; or p is 2-8; or p is 4-8; or p is 6-8; or p is 7-8; or p is 7.4; or p is 7; or p is 8.

47. The method according to any one of claims 1-46, the patient has an FRα TPS of <50%; or the patient has an FRα TPS of ≥50%; or the patient has an FRα TPS of ≥75%.

48. The method according to any one of claims 1-47, the cancer is selected from colorectal cancer, lung cancer (e.g., non-small cell lung cancer), ovarian cancer, uterine cancer, endometrial cancer, peritoneal cancer, fallopian tube cancer, pancreatic cancer, head and neck squamous cell carcinoma, nasopharyngeal cancer, laryngeal cancer, liver cancer, breast cancer, brain cancer, renal cancer, renal cell carcinoma, colon cancer, testicular cancer, cervical cancer, tongue cancer, bladder cancer, retinoblastoma, glioblastoma, mesothelioma, oral epithelioid cancer, choriocarcinoma and head and neck cancer; or the cancer is platinum-refractory cancer; or the cancer is platinum-refractory ovarian cancer; or the patient has platinum-refractory advanced or metastatic ovarian cancer; or the cancer is selected from platinum-refractory epithelial ovarian cancer, fallopian tube cancer, or primary peritoneal cancer; or the patient has platinum-refractory advanced or metastatic epithelial ovarian cancer, primary peritoneal cancer, or fallopian tube cancer; or the cancer is platinum-resistant cancer; or the cancer is platinum-resistant ovarian cancer; or the patient has platinum-resistant advanced or metastatic ovarian cancer; or the cancer is selected from platinum-resistant epithelial ovarian cancer, fallopian tube cancer, or primary peritoneal cancer; or the patient has platinum-resistant advanced or metastatic epithelial ovarian cancer, primary peritoneal cancer, or fallopian tube cancer; or the patient has platinum-resistant advanced or metastatic epithelial ovarian cancer, primary peritoneal cancer, or fallopian tube cancer with no more than three prior lines of therapy; or the patient has advanced or metastatic solid tumors that have failed or were intolerant to standard therapy; or the patient has advanced or metastatic endometrial cancer, breast cancer, or non-small cell lung cancer that has failed or were intolerant to standard therapy.

49. The method according to any one of claims 43-48, the antibody-drug conjugate is administered at a dose of 0.5 mg/kg to 3 mg/kg, or 1.3 mg/kg to 3 mg/kg, or 1.7 mg/kg to 2.5 mg/kg.

50. The method according to any one of claims 43-48, the antibody-drug conjugate is administered at a dose of 30 mg-300 mg; or 50 mg-280 mg; or 100 mg-250 mg.

51. The method according to any one of claims 43-48, the antibody-drug conjugate is administered at a dose of 30 mg/m²-120 mg/m²; or 50 mg/m²-120 mg/m²; or 70 mg/m²-100 mg/m²; or 75 mg/m²-100 mg/m².

52. The method according to any one of claims 1-51, the antibody-drug conjugate is administered once every 2 weeks, once every 3 weeks or once every 4 weeks.

53. The method according to any one of claims 1-51, the antibody-drug conjugate is administered at about 2.4 mg/kg once every 2-4 weeks, e.g., once every 3 weeks; the antibody-drug conjugate is administered at about 2.1 mg/kg once every 2-4 weeks, e.g., once every 3 weeks; or the antibody-drug conjugate is administered at about 1.8 mg/kg once every 2-4 weeks, e.g., once every 3 weeks; or the antibody-drug conjugate is administered at about 1.5 mg/kg once every 2-4 weeks, e.g., once every 3 weeks; or the antibody-drug conjugate is administered at about 93 mg/m² once every 2-4 weeks, e.g., once every 3 weeks; or the antibody-drug conjugate is administered at about 84 mg/m² once every 2-4 weeks, e.g., once every 3 weeks; or the antibody-drug conjugate is administered at about 76 mg/m² once every 2-4 weeks, e.g., once every 3 weeks.

54. The method according to any one of claims 1-53, the antibody-drug conjugate is administered at about 84 mg/m² once every 2-4 weeks, e.g., once every 3 weeks.

55. The method according to any one of claims 1-53, the antibody-drug conjugate is administered at about 93 mg/m² once every 2-4 weeks, e.g., once every 3 weeks.

56. The method according to any one of claims 1-55, the administration mode of the antibody-drug conjugate is injection; or the administration mode of the antibody-drug conjugate is intravenous injection, subcutaneous injection, or intraperitoneal injection; or the administration mode of the antibody-drug conjugate is intravenous infusion.
